(19) Europäisches Patentamt
European Patent Office
Office européen des brevets

(11) Veröffentlichungsnummer: **0 000 500**
**B1**

(12) **EUROPÄISCHE PATENTSCHRIFT**

(45) Veröffentlichungstag der Patentschrift: **28.04.82**

(21) Anmeldenummer: **78100367.8**

(22) Anmeldetag: **11.07.78**

(51) Int. Cl.³: **C 07 D 501/20, A 61 K 31/545, C 07 C 103/46 //C07D307/54, C07D333/24**

(54) **Cephalosporinderivate, Verfahren zu deren Herstellung und deren pharmazeutische Präparate.**

(30) Priorität: **18.07.77 LU 77788**

(43) Veröffentlichungstag der Anmeldung: **07.02.79 Patentblatt 79/3**

(45) Bekanntmachung des Hinweises auf die Patenterteilung: **28.04.82 Patentblatt 82/17**

(84) Benannte Vertragsstaaten: **BE CH DE FR GB LU NL SE**

(56) Entgegenhaltungen:
**FR - A - 2 228 475**
**FR - A - 2 228 476**
**FR - A - 2 235 695**

(73) Patentinhaber: **CIBA-GEIGY AG**
**Patentabteilung Postfach**
**CH-4002 Basel (CH)**

(72) Erfinder: **Kocsis, Karoly, Dr.**
**Maiengasse 19**
**CH-4056 Basel (CH)**
Erfinder: **Schneider, Peter, Dr.**
**Rheinfelderstrasse 21 A/1**
**CH-4058 Basel (CH)**
Erfinder: **Fechtig, Bruno, Dr.**
**Hinterlindenweg 1**
**CH-4153 Reinach (CH)**
Erfinder: **Scartazzini, Riccardo, Dr.**
**Conrad Ferdinand Meyer-Strasse 38**
**CH-4059 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 500 B1

Cephalosporinderivate, Verfahren zu deren Herstellung und deren pharmazeutische Präparate

Die Erfindung betrifft neue Acylamido-3-cephem-4-carbonsäure-Verbindungen und ihre Salze, Verfahren zu ihrer Herstellung, diese Verbindungen enthaltende pharmazeutische Mittel mit antibiotischer Wirksamkeit und ihre therapeutische Verwendung zur Behandlung von Infektionen, sowie neue Zwischenprodukte und deren Herstellung.

Es sind bereits zahlreiche 7β-Acylamido-3-cephem-4-carbonsäure-Verbindungen bekannt geworden, die sich durch die Substituenten in 3-Stellung des 3-Cephemgerüstes, sowie durch die Acylgruppe an der 7β-Aminogruppe unterscheiden. Uebersichten über solche Verbindungen, Verfahren zu ihrer Herstellung, sowie auch über ihre antibiotischen Aktivitäten wurden beispielsweise von Edwin H. Flynn, Cephalosporins and Penicillins, Academic Press, New York und London, 1972, J. Cs. Jászberény und T. E. Gunda, Progr. Med. Chem., Vol. 12, *1975*, S. 395—477, und Peter G. Sammes, Chemical Reviews, *1976*, Vol. 76, No. 1, S. 113—155. veröffentlicht. 7β-Phenylglycinamino-cephalosporinverbindungen, deren Phenylgruppe durch eine Ureido- oder Niederalkylureidogruppe substituiert ist, sind aus den französischen Patentanmeldungen 2.228.475, 2.228.476 und 2.235.695 bekannt geworden. Das vom Cephalosporin C her bekannte Prizip einer endständigen α-Aminocarbonsäuregruppierung wird in diesen Anmeldungen nicht vorgeschlagen. Die vergleichsweise geringe antibiotische Aktivität von Cephalosporin C legt die Einführung einer solchen Gruppierung in eine Acylamidogruppe eines Cephalosporinantibiotikums auch nicht nahe.

Der vorliegenden Erfindung liegt die Aufgabe zugrunde, neue 7β-Acylamino-3-cephem-4-carbonsäure-Verbindungen herzustellen, die neuartige Acylgruppen besitzen, welche durch das Vorhandensein einer endständigen α-Aminocarbonsäuregruppierung charakterisiert sind. Die neuen Verbindungen zeichnen sich durch eine hervorragende Wirkung gegenüber normalen und resistenten Keimen aus und besitzen eine unerwartet lange Verweildauer im Körper.

Die Erfindung betrifft insbesondere Acylamido-3-cephem-4-carbonsäure-Verbindung der Formel

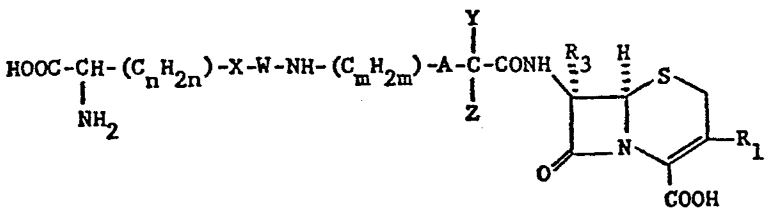

(I)

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2NH$—CO—, oder X—W zusammen eine Gruppe —CO— oder —CO—$NHSO_2$—, A gegebenenfalls durch Niederalkyl, Hydroxy, Niederalkoxy und/oder Halogen substituiertes Phenylen, gegebenenfalls durch Niederalkyl, Niederalkoxy und/oder Halogen substituiertes Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—$R^o$, worin $R^o$ Wasserstoff oder gegebenenfalls durch Niederalkoxy, Halogen, Hydroxy, acyliertes Hydroxy, Sulfo, Carboxy oder durch Niederalkyl verestertes Carboxy substituiertes Niederalkyl darstellt, $R_1$ Wasserstoff, $C_1$—$C_4$-Niederalkyl, $C_1$—$C_4$-Niederalkoxy, Halogen oder eine Gruppe der Formel —$CH_2$—$R_2$, worin $R_2$ eine Hydroxygruppe, eine durch eine niederaliphatische Carbonsäure, die Carbaminsäure oder eine am N-Atom durch Niederalkyl, Halogenniederalkyl oder Niederalkanoylniederalkyl substituierte Carbaminsäure veresterte Hydroxy- oder Mercaptogruppe, Niederalkoxy, Niederalkylthio, oder eine durch einen über ein Ringkohlenstoffatom an die Mercaptogruppe gebundenen heterocyclischen Rest mit 1—4 Ringstickstoffatomen und gegebenenfalls einem weiteren Ringheteroatom der Gruppe Sauerstoff und Schwefel, der durch Niederalkyl, Hydroxyniederalkyl, Niederalkanoylniederalkyl, Halogenniederalkyl, Carboxyniederalkyl, Niederalkoxycarbonylniederalkyl, Sulfoniederalkyl, amidiertes Sulfoniederalkyl, Aminoniederalkyl, Mono- oder Diniederalkylaminoniederalkyl, Acylaminoniederalkyl, Cycloalkyl, Aryl, Arylniederalkyl, Heterocyclyl, Halogen, Amino, Mono- oder Diniederalkylamino, Niederalkanoylamino, Halogenniederalkanoylamino, Carboxyniederalkanoylamino, Nitro, Hydroxy, Niederalkoxy, Carboxy, Niederalkoxycarbonyl, Carbamoyl, N-mono- oder N,N-diniederalkyliertes Carbamoyl, Cyan, Oxo oder Oxido ein- oder mehrfach substituiert sein kann, verätherte Mercaptogruppe oder eine quaternäre Ammoniumgruppe darstellt, wobei unter niederen Resten, sofern nicht ausdrucklich anders definiert, solche mit 1 bis 7 C-Atomen zuverstehen sind, und $R_3$ Wasserstoff oder Methoxy bedeuten, worin die Carboxylgruppen gegebenenfalls in physiologisch spaltbarer Form verestert sind, und ihre Salze Verfahren zur Herstellung dieser Verbindungen, solche Stoffe enthaltende pharmazeutische Mittel und deren therapeutische Verwendung.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden Gruppen, sofern nicht ausdrücklich anders definiert, bis zu. 7, bevorzugt bis zu 4 C-Atome enthalten.

Eine Gruppe —$(C_nH_{2n})$— ist eine verzweigte oder unverzweigte Alkylenkette, und ist insbesondere Methylen, 1,2-Aethylen, 1,3-Propylen oder 1,4-Butylen, ferner beispielsweise 1,1-Aethylen, 1,1-Propylen, 1,2-Propylen, 1,1-Butylen, oder 1,1-Isobutylen.

Eine gegebenenfalls substituierte Phenylengruppe A ist insbesondere p-, kann aber auch o- oder m-Phenylen sein. Substituenten der Phenylengruppe sind beispielsweise Niederalkyl, wie Methyl, Hydroxy, Niederalkoxy, wie Methoxy, und/oder Halogen, wie Fluor, Chlor oder Brom.

Eine gegebenenfalls substituierte Thienylengruppe A ist insbesondere 2,5-Thienylen, ferner 2,4- oder 2,3-Thienylen.

Eine gegebenenfalls substituierte Furylengruppe A ist insbesondere 2,5-Furylen, ferner 2,4- oder 2,3-Furylen.

Substituenten der Thienylen- und Furylengruppe A sind beispielsweise Niederalkyl, wie Methyl, Niederalkoxy, wie Methoxy, und/oder Halogen, wie Fluor, Chlor oder Brom.

Wenn Z Wasserstoff bedeutet, ist Y bevorzugt Wasserstoff oder Hydroxy, oder auch Amino oder Sulfo.

In einer Gruppe =N—O—$R^o$ die bevorzugt in der syn-Form vorliegt, enthält $R^o$ als gegebenenfalls substituiertes Niederalkyl 1—4 Kohlenstoffatome. Substituenten einer solchen Niederalkylgruppe $R^o$ sind beispielsweise Niederalkoxy, wie Methoxy, Halogen, wie Fluor, Chlor oder Brom, Hydroxy oder acyliertes Hydroxy, wie Niederalkanoyloxy, z.B. Acetoxy, Sulfo, und insbesondere freies oder auch verestertes Carboxy.

Repräsentative Gruppen $R^o$ sind beispielsweise Methyl, Aethyl, Propyl, Butyl, Methoxymethyl, Methoxyäthyl, wie 2-Methoxyäthyl, 3-Methoxypropyl, 4-Methoxybutyl, 2-Halogen-, wie 2-Chlor-äthyl, 3-Halogen-, wie 3-Chlorpropyl, oder 4-Halogen-, wie 4-Chlorbutyl, 2-Hydroxyäthyl, 3-Hydroxypropyl oder 4-Hydroxybutyl, worin die Hydroxygruppe beispielsweise durch Niederalkanoyl, wie Acetyl, acyliert sein kann, 2-Sulfoäthyl, 3-Sulfopropyl, 2-Carboxyäthyl, 3-Carboxypropyl oder 4-Carboxybutyl, worin die Carboxygruppe beispielsweise durch Niederalkyl, wie Methyl oder Aethyl verestert sein kann.

Eine Niederalkylgruppe $R_1$ enthält 1—4 C-Atome und ist beispielsweise Aethyl, Propyl, Butyl oder insbesondere Methyl.

Eine Niederalkoxygruppe $R_1$ enthält 1—4 C-Atome und ist beispielsweise Methoxy, Aethoxy, Propoxy oder Butoxy.

$R_1$ als Halogen ist Fluor, Brom, Jod oder bevorzugt Chlor.

Eine veresterte Hydroxy- oder Mercaptogruppe $R_2$ ist durch eine niederaliphatische Carbonsäure oder durch eine gegebenenfalls N-substituierte Carbaminsäure verestert.

Mit niederaliphatischen Carbonsäuren veresterte Hydroxygruppen $R_2$ sind insbesondere Niederalkanoyloxy, insbesondere Acetyloxy, ferner Formyloxy, Propionyloxy, Valeryloxy, Hexanoyloxy, Heptanoyloxy oder Pivaloyloxy.

Mit niederaliphatischen Carbonsäuren veresterte Mercaptogruppen $R_2$ sind Niederalkanoylthio, wie Acetylthio, Formylthio, Propionylthio, Valeroylthio, Hexanoylthio, Heptanoylthio oder Pivaloylthio.

In einer durch eine gegebenenfalls N-substituierte Carbaminsäure veresterten Hydroxy- oder Mercaptogruppe $R_2$ sind N-Substituenten gegebenenfalls durch Halogen, z.B. Chlor, oder durch Niederalkanoyl, z.B. Acetyl oder Propionyl, substituiertes Niederalkyl, z.B. Methyl, Aethyl, 2-Chloräthyl, oder 2-Acetoxyäthyl. In dieser Art veresterte Hydroxygruppen $R_2$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy. Entsprechende veresterte Mercaptogruppen $R_2$ sind z.B. Carbamoylthio, N-Methylcarbamoylthio, N-Aethylcarbamoylthio, N-(2-Chloräthyl)-carbamoylthio oder N-(2-Acetoxyäthyl)carbamoylthio.

Niederalkoxy- und Niederalkylthiogruppen $R_2$ haben insbesondere 1—4 C-Atome, und sind erster Linie Methoxy, sowie Aethoxy, n-Propyloxy oder Isopropyloxy, ferner geradkettiges oder verzweigtes Butyloxy, bzw. Methylthio, sowie Aethylthio, n-Propylthio oder Isopropylthio, ferner geradkettiges oder verzweigtes Butylthio.

Durch einen gegebenenfalls substituierten, über ein Ringkohlenstoffatom an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel verätherte Mercaptogruppen $R_2$ sind in erster Linie gegebenenfalls die unten genannten Substituenten enthaltende, monocyclische, fünfgliedrige diaza-, triaza-, tetraza-, thiaza-, thiadiaza-, thiatriaza-, oxaza- oder oxadiazacyclische Reste aromatischen Charakters.

Substituenten solcher Heterocyclylreste sind Niederalkyl, insbesondere Methyl, sowie Aethyl, n-Propyl, Isopropyl oder geradkettiges oder verzweigtes Butyl, oder durch Hydroxy, Niederalkanoyloxy, Halogen, wie Chlor, Carboxy, Niederalkoxycarbonyl, Sulfo, amidiertes Sulfo, Amino, Mono- oder Diniederalkylamino, Niederalkanoylamino, oder durch substituiertes, wie durch Carboxy oder Halogen substituiertes Niederalkanoylamino substituiertes Niederalkyl, beispeilsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen $R_2$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-(3-Carboxypropionylamino)-1,3,4-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4-oxadiazol-5-ylthio.

Bevorzugte heterocyclisch verätherte Mercaptogruppen $R_2$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxido-pyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio.

Quaternäre Ammoniumgruppen $R_2$ sind von tertiären organischen Basen, vorzugsweise von entsprechenden aliphatischen Aminen oder in erster Linie von entsprechenden heterocyclischen Stickstoffbasen abgeleitete, über das Stickstoffatom mit dem Methylkohlenstoffatom verbundene, quaternäre Ammoniumgruppen.

In einer quaternären Ammoniumgruppe $R_2$, die von einer tertiären organischen Base abgeleitet wird, ist das Stickstoffatom an das Methylkohlenstoffatom gebunden und liegt demgemäss in quaternisierter, positiv geladener Form vor. Quaternäre Ammoniumgruppen sind u.a. Triniederalkylammonium, z.B. Trimethylammonium, Triäthylammonium, Tripropylammonium oder Tributylammonium, insbesondere aber gegebenenfalls substituierte, z.B. Niederalkyl, wie Methyl, Hydroxyniederalkyl, wie Hydroxymethyl, Amino, substituiertes Sulfonamido, wie 4-Aminophenylsulfonamido, Hydroxy, Halogen, wie Fluor, Chlor, Brom oder Jod, Halogenniederalkyl, wie Trifluormethyl, Sulfo, gegebenenfalls funktionell abgewandeltes Carboxy, wie Carboxy, Niederalkoxycarbonyl, z.B. Methoxycarbonyl, Cyan, gegebenenfalls durch Niederalkyl, z.B. Methyl oder Aethyl, oder Hydroxyniederalkyl, z.B. Hydroxymethyl, N-mono- oder N,N-disubstituiertes Carbamoyl, z.B. Carbamoyl, N-Methylcarbamoyl oder N,N-Dimethyl-carbamoyl, gegebenenfalls durch Niederalkyl N-substituiertes Hydrazincarbonyl, z.B. Hydrazinocarbonyl, Carboxyniederalkyl, wie Carboxymethyl, Niederalkanoyl, wie Acetyl, oder 1-Niederalkyl-pyrrolidinyl, wie 1-Methyl-2-pyrrolidinyl, mono- oder polysubstituierte, monocyclische oder bicyclische azacyclische Ammoniumgruppen aromatischen Charakters, mit 1 oder 2 Ringstickstoff- und gegebenenfalls einem Ringschwefelatom, wie Pyrimidinium, Pyridazinium, Thiazolium, Chinolinium und in erster Linie Pyridinium.

Heterocyclische Ammoniumgruppen $R_2$ sind in erster Linie gegebenenfalls Niederalkyl, Hydroxy-

niederalkyl, Amino, substituiertes Sulfonamido, Hydroxy, Halogen, Trifluormethyl, Sulfo, Carboxy, Niederalkoxycarbonyl, Cyan, Niederalkanoyl, 1-Niederalkyl-pyrrolidinyl oder gegebenenfalls durch Niederalkyl oder Hydroxyniederalkyl N-substituiertes Carbamoyl enthaltendes Pyridinium, z.B. Pyridinium, 2-, 3- oder 4-Methyl-pyridinium, 3,5-Dimethyl-pyridinium, 2,4,6-Trimethylpyridinium, 2-, 3- oder 4-Aethyl-pyridinium, 2-, 3- oder 4-Propyl-pyridinium oder insbesondere 4-Hydroxymethylpyridinium, ferner 2-Amino- oder 2-Amino-6-methyl-pyridinium, 2-(4-Aminophenylsulfonylamido)-pyridinium, 3-Hydroxy-pyridinium, 3-Fluor-, 3-Chlor-, 3-Jod- oder insbesondere 3-Brom-pyridinium, 4-Trifluormethyl-pyridinium, 3-Sulfopyridinium, 2-, 3- oder 4-Carboxy- oder 2,3- oder 3,4-Dicarboxy-pyridinium, 4-Methoxycarbonyl-pyridinium, 3- oder 4-Cyan-pyridinium, 3-Carboxymethyl-pyridinium, 3- oder 4-Acetyl-pyridinium, 3-(1-Methyl-2-pyrrolidinyl)-pyridinium, und insbesondere 4-Carbamoyl-, 3-Carbamoyl-, 3,4-Dicarbamoyl-, 3- oder 4-N-Methylcarbamoyl, 4-N,N-Dimethylcarbamoyl-, 4-N-Aethylcarbamoyl-, 3-N,N-Diäthylcarbamoyl, 4-N-Propylcarbamoyl-4-Isopropylcarbamoyl- und 4-Hydroxymethyl-carbamoyl-pyridinium.

Die in Verbindungen der Formel I vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppe sind leicht, das heisst ohne dass unerwünschte Nebenreaktionen stattfinden, beispielsweise solvolytisch reduktiv, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in "The Peptides", Vol. I, Schröder and Lübke, Academic Press, London, New York, 1965.

So sind Carboxylgruppen z.B. üblicherweise in veresterter Form geschützt, wobei solche Estergruppierungen unter schonenden Bedingungen leicht spaltbar sind. Als geeignete geschützte Carboxylgruppen kommen insbesondere Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt substituiertes Benzyloxycarbonyl, z.B. 4-Nitro-benzyloxycarbonyl, oder 4-Methoxybenzyloxycarbonyl, oder z.B. wie oben erwähnt substituiertes Diphenylmethoxycarbonyl, z.B. Benzhydryloxycarbonyl oder Di-(4-methoxyphenyl)-methoxycarbonyl, oder 2-Halogenniederalkoxycarbonyl, z.B. 2,2,2-Trichloräthoxycarbonyl, insbesondere Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl, in Frage. Veresterte Carboxylgruppen sind ebenfalls entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogen-silyl, z.B. Dimethylchlor-silyl, oder entsprechend substituierte Stannylverbindungen, z.B. Tri-n-butylstannyl.

Bevorzugt als geschützte Carboxylgruppe ist insbesondere tert.-Butyloxycarbonyl, gegebenenfalls, z.B. wie erwähnt substituiertes Benzyloxycarbonyl, z.B. 4-Nitrobenzyloxycarbonyl, oder Diphenylmethoxycarbonyl, z.B. Benzhydryloxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethyloxycarbonyl oder Pivaloyloxymethoxycarbonyl, Aminoniederalkanoyloxymethoxycarbonyl, insbesondere $\alpha$-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe kann z.B. in Form einer leicht spaltbaren Acylamino, Triarylmethylamino-, verätherten Mercaptoamino-, 1-Acyl-2-niederalkylidenamino-, Silyl- oder Stannylaminogruppe oder als Azidogruppe vorliegen.

In einer entsprechenden Acylaminogruppe ist Acyl vorzugsweise der Acylrest eines Kohlensäurehalbesters, wie Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt, wie gegebenenfalls, z.B. wie oben erwähnt, substituiertes Benzyloxycarbonyl, z.B. 4-Nitro-benzyloxycarbonyl, oder z.B. wie oben erwähnt substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Bromäthoxycarbonyl oder

2-Iodäthoxycarbonyl, oder Acylmethoxycarbonyl, insbesondere Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl. Acyl in einer Acylaminogruppe kann auch den entsprechenden Rest einer organischen Sulfonsäure darstellen; ein solcher Rest ist insbesondere Arylsulfonyl, worin Aryl einen gegebenenfalls, z.B. durch Niederalkyl, wie Methyl, Halogen, wie Brom, oder Nitro, substituierten Phenylrest bedeutet, z.B. 4-Methylphenylsulfonyl.

In einer Triarylmethylaminogruppe sind die Arylreste insbesonder gegebenenfalls substituierte Phenylreste; eine entsprechende Gruppe ist in erster Linie Trityl.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder eines Kohlensäurehalbesters, wie eines Kohlensäureniederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogen-silyl, z.B. Dimethyl-chlor-silyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäure, wie p-Toluolsulfonsäure, in Frage.

Bevorzugt als Aminoschutzgruppen sind die Acylreste von Kohlensäurehalbestern, insbesondere tert.-Niederalkoxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere einer der im Zusammenhang mit einer geschützten Aminogruppe genannten Acylreste von Kohlensäurehalbestern, insbesondere 2,2,2-Trichloräthoxycarbonyl, oder organischen Silyl- oder Stannylreste, ferner leicht abspaltbare 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. 1-Methoxyäthyl, 1-Aethoxy-äthyl, 1-Methylthio-äthyl oder 1-Aethylthioäthyl, oder 2-Oxa- oder 2-Thiacycloniederalkyl mit 5—7 Ringatomen, z.B. 2-Tetrahydrofuryl oder 2-Tetrahydropyranyl oder entsprechende Thiaanaloge, sowie leicht abspaltbare, gegebenenfalls substituierte $\alpha$-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch-aliphatischen, aromatischen oder araliphatischen Alkohol, wie einem Niederalkanol, oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

Salze sind insbesondere diejenigen von Verbindungen der Formel I mit einer freien Carboxylgruppe, in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatischaliphatische oder araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure, oder Phosphorsäure, oder mit geeigneten organischen Carbon- oder Sulfonsäuren, z.B. Trifluoressigsäure, sowie mit Aminosäuren, wie Arginin und Lysin bilden. Verbindungen der Formel I mit einer freien Carboxylgruppe und freien Aminogruppe können auch in Form von inneren Salzen, d.h. in zwitterionischer Form vorliegen.

Der Acylrest an der 7$\beta$-Aminogruppe enthält ein oder gegebenenfalls zwei Asymmetriezentren. Das gegebenenfalls vorhandene Asymmetriezentrum nächst zur 7-Aminogruppe, nämlich wenn Y Hydroxyl, Amino oder Sulfo und Z Wasserstoff bedeuten, liegt in der R,S— oder bevorzugt in der R-Konfiguration vor. Das Asymmetriezentrum an der endständigen Aminocarbonsäuregruppierung kann die R—, S— oder R,S-Konfiguration besitzen.

Die Verbindungen der Formel I, worin Carboxylgruppen gegebenenfalls in physiologisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie *in vitro* gegen gram-positive und gram-negative Mikroorganismen, wie gegen Kokken, inklusive *Neisseria* Arten, und Anaerobier in Minimalkonzentrationen von etwa 0,02 mcg/ml und gegen Enterobacteriaceae in Minimalkonzentrationen von etwa 0,25 mcg/ml, wirksam. *In vivo,* bei subcutaner Application an der Maus, sind sie beispielsweise gegen grampositive Kokken, wie *Staphylococcus aureus* (in Minimaldosen von etwa 3 mg/kg) und *Streptococcus pneumoniae* (in Minimaldosen von etwa 0,15 mg/kg), gegen Enterobakterien, wie *Escherichia coli* (in Minimaldosen von etwa 8 mg/kg), *Klebsiella pneumoniae* und *Proteus mirabilis* (in Minimaldosen von etwa 0,3 mg/kg), und gegen andere gramnegative Bakterien, wie *Pasteurella multocida* (in Minimaldosen von 0,1 mg/kg), wirksam. Die neuen Verbindungen können deshalb entsprechend, z.B. in Form von antibiotisch wirksamen Präparaten zur Behandlung von durch gram-positive oder gram-negative Bakterien und Kokken, insbesondere durch von Enterobakterien, wie *Escherichia coli, Klebsiella pneumoniae* und Proteus mirabilis, verursachten Infektionen Verwendung finden.

Verbindungen der Formel I, worin die funktionellen Gruppen geschützt sind werden als Ausgangsmaterialien zur Herstellung von Verbindungen der Formel I verwendet.

Die vorliegende Erfindung betrifft in erster Linie diejenigen Verbindungen der Formel I, worin die Gruppe $—(C_nH_{2n})—$ unverzweigt ist und die Indices n und m die angegebene Bedeutung haben, X Sauerstoff oder die Gruppe —NH—, W eine Gruppe —CO— oder $—CO—NHSO_2—$, oder X—W zusammen eine Gruppe —CO— oder $—CO—NHSO_2—$ bedeuten, A p- oder m-Phenylen, 2,5-Thienylen oder 2,5-Furylen darstellt, Y Wasserstoff, Hydroxyl, Amino oder Sulfo und Z Wasserstoff, oder Y und Z Wasserstoff, oder Y und Z zusammen eine Gruppe $=N—O—R^o$ bedeuten, worin $R^o$ Wasserstoff oder Methyl ist, $R_1$ Niederalkyl, Niederalkoxy, Halogen oder eine Gruppe der Formel $—CH_2—R_2$ bedeutet, worin $R_2$ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio oder Pyridinio darstellt, worin die heterocyclischen Ringe gegebenenfalls durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und $R_3$ Wasserstoff oder Methoxy darstellen, pharmazeutisch verwendbare Salze von solchen Verbindungen, sowie die zu ihrer Herstellung verwendbaren Ausgangsstoffe und Zwischenprodukte.

Besonders hervorzuheben sind Verbindungen der Formel I, worin die Gruppe $—(C_nH_{2n})—$ unverzweigt ist und die Indices n und m die angegebene Bedeutung haben, X Sauerstoff oder die Gruppe —NH— bedeutet, W eine Gruppe —CO— oder $—CONHSO_2—$, oder X—W zusammen eine Gruppe —CO— oder $—CONHSO_2—$ bedeuten, A p- oder m-Phenylen, oder, wenn m 1 ist, auch 2,5-Thienylen oder 2,5-Furylen darstellt, Y Wasserstoff, Hydroxyl, Amino oder Sulfo und Z Wasserstoff, oder Y und Z zusammen eine Gruppe $=N—O—R^o$ bedeuten, worin $R^o$ Wasserstoff oder Methyl ist, $R_1$ Methyl, Methoxy, oder eine Gruppe der Formel $—CH_2—R_2$ bedeutet, worin $R_2$ Acetoxy, Carbamoyloxy, Tetrazolylthio, insbesondere 1-Methyl-1H-tetrazol-5-ylthio, 1-Sulfomethyl-1H-tetrazol-5-ylthio, 1-Carboxymethyl-1H-tetrazol-5-ylthio, oder 1-(2-Dimethylaminoäthyl)-1H-tetrazol-5-ylthio, oder Thiadiazolylthio, insbesondere 2-Methyl-1,3,4-thiadiazol-5-ylthio, oder Carbamoylpyridinio, insbesondere 4-Carbamoylpyridinio, darstellt, und $R_3$ Wasserstoff oder Methoxy bedeutet, pharmazeutisch verwendbare Salze von solchen Verbindungen, sowie die zu ihrer Herstellung verwendbaren Ausgangsstoffe und Zwischenprodukte.

Die Erfindung betrifft insbesondere die in den Beispielen beschriebenen Verbindungen der Formel I, deren pharmazeutisch unbedenklichen Salze, sowie die dort beschriebenen Ausgangsstoffe und Zwischenprodukte.

Die Verbindungen der vorliegenden Erfindung werden nach an sich bekannten Verfahren erhalten.

Verbindungen der Formel I, worin die Carboxylgruppe gegebenenfalls in physiologisch spaltbarer Form verestert sind, sowie ihre Salze, werden hergestellt, indem man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung eine Gruppe $R_1$ in eine andere Gruppe $R_1$ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

In den Ausgangsverbindungen der Formel I, worin funktionelle Gruppen geschützt sind, werden diese, z.B. geschützte Carboxyl-, Amino-, Hydroxy-, Mercapto- und/oder Sulfogruppen, in an sich bekannter Weise, wie mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt.

So kann man z.B. eine tert.-Niederalkoxycarbonyl-, Polycycloalkoxycarbonyl- oder Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie Essig-, sowie Ameisensäure, oder eines Alkohols, wobei man vorzugsweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Acylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine Polyhalogenaryloxycarbonylgruppe, wie die Pentachlorphenyloxycarbonylgruppe wird unter milden basischen Bedingungen, wie durch verdünnte Natronlauge oder organische Basen in Gegenwart von Wasser, zur freien Carboxylgruppe verseift. Eine z.B. durch Silylierung oder Stannylierung geschütze Carboxylgruppe kann in üblicher Weise, z.B. durch Behandeln mit Wasser oder einem Alkohol, freigesetzt werden.

Eine geschützte Aminogruppe wird in an sich bekannter und je nach Art der Schutzgruppe in verschiedenartiger Weise, z.B. mittels Solvolyse oder Reduktion, freigesetzt. Eine 2-Halogenniederalkoxycarbonylaminogruppe (gegebenenfalls nach Umwandlung einer 2-Brom-niederalkoxycarbonylgruppe in eine 2-Jod-niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart von wässriger Essigsäure, eine Aroylmethoxycarbonylaminogruppe auch durch Behandeln mit einem nucleophilen, vorzugsweise salzbildenden Reagens, wie Natriumthiophenolat, und eine 4-Nitrobenzyloxycarbonylaminogruppe auch durch Behandeln mit einem Alkali-metall-, z.B. Natriumdithionit, eine Diphenylmethoxycarbonylamino-, tert.-Niederalkoxycarbonylamino- oder Polycycloalkoxycarbonylaminogruppe durch Behandeln z.B. mit Ameisen- oder Trifluoroessigsäure, eine gegebenenfalls substituierte Benzyloxycarbonylaminogruppe, z.B. mittels Hydrogenolyse durch Behandeln mit Wasserstoff in Gegenwart eines Hydrierkatalysators, wie eines Palladiumkatalysators, eine Aryl- oder Arylniederalkylthioaminogruppe z.B. durch Behandeln mit einem nucleophilen Reagens, wie schwefliger Säure, eine Arylsulfonylaminogruppe z.B. mittels elektrolytischer Reduktion, eine 1-Acyl-2-niederalkylidenaminogruppe oder eine Triarylmethylgruppe z.B. durch Behandeln mit wässriger Mineralsäure, und eine mit einer organischen Silyl- oder Stannylgruppe geschützte Aminogruppe z.B. mittels Hydrolyse oder Alkoholyse freigesetzt werden.

Eine in Form einer Azidogruppe geschützte Aminogruppe wird in an sich bekannter Weise durch Reduktion, in die freie Aminogruppe übergeführt, beispielsweise durch katalytische Hydrierung mit Wasserstoff und einem Hydrierkatalysator, wie Platinoxid, Palladium, oder auch Raney-Nickel, oder auch durch Zink und Säure, wie Essigsäure. Die katalytische Hydrierung wird bevorzugt in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°, oder auch bei erniedrigter oder erhöhter Temperatur, durchgeführt.

Eine durch eine Acylgruppe, eine Silyl- oder Stannylgruppe oder durch einen gegebenenfalls substituierten $\alpha$-Phenyl-niederalkylrest geschützte Hydroxygruppe wird wie eine entsprechend geschützte Aminogruppe freigesetzt. Eine durch 2,2-Dichloracetyl geschützte Hydroxygruppe wird durch basische Hydrolyse und eine durch einen 2-oxa- oder 2-thia-aliphatischen oder -cycloaliphatischen Kohlenwasserstoffrest geschützte Hydroxygruppe wird durch Acidolyse freigesetzt.

Eine geschützte Sulfogruppe wird analog einer geschützten Carboxylgruppe freigesetzt.

Bevorzugt werden die Schutzgruppen so gewählt, dass sie alle gleichzeitig abgespalten werden können, beispielsweise acidolytisch, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatomsphäre.

In einer Verbindung der Formel I, worin eine Aminogruppe, wenn notwendig, geschützt ist, und worin die Carboxylgruppe in 4-Stellung des Cephemringes in freier Form vorliegt, kann man in an sich bekannter Weise eine Gruppe $R_1$ durch einen anderen Rest $R_1$ ersetzen oder in einen anderen Rest $R_1$ umwandeln. So ist es z.B. möglich, in einer Verbindung der Formel I, worin $R_1$ eine Gruppe der Formel $-CH_2-R_2$ bedeutet, und $R_2$ z.B. einen, durch nucleophile Substituenten ersetzbaren Rest darstellt, oder in einem Salz davon, durch Behandeln mit einer entsprechenden Mercaptan- oder mit einer Thio-

carbonsäureverbindung einen solchen Rest $R_2$ durch eine verätherte bzw. veresterte Mercaptogruppe $R_2$ zu ersetzen. Ein geeigneter, durch eine verätherte Mercaptogruppe ersetzbarer Rest ist beispielsweise eine durch eine niederaliphatische Carbonsäure veresterte Hydroxygruppe. Solche veresterten Hydroxygruppen sind insbesondere Acetyloxy, ferner Formyloxy.

Die Reaktion einer solchen Verbindung mit einer geeigneten Mercaptanverbindung kann unter neutralen oder schwach basischen Bedingungen in Gegenwart von Wasser und gegebenenfalls einem, mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Kalium- oder Calciumhydroxid, -carbonat oder -hydrogencarbonat, eingestellt werden. Als organische Lösungsmittel können z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden.

Veresterte Hydroxygruppen $R_2$ in einer Verbindung der Formel I, worin $R_1$ die Gruppe $—CH_2—R_2$ bedeutet, wobei $R_2$ für eine, durch den Acylrest eines gegebenenfalls substituierten Halbamids der Kohlensäure veresterte Hydroxygruppe steht, kann man z.B. einführen, indem man eine entsprechende Verbindung der Formel I, worin $R_2$ für freies Hydroxy steht (das man z.B. durch Abspaltung des Acetylrestes aus einer Acetyloxygruppe $R_2$, z.B. durch Hydrolyse in schwach-basischem Medium, wie mit einer wässrigen Natriumhydroxydlösung bei pH 9—10, oder durch Behandeln mit einer geeigneten Esterase, wie einem entsprechenden Enzym aus *Rhizobium tritolii, Rhizobium lupinii, Rhizobium japonicum* oder *Bacillus subtilis,* oder einer geeigneten Citrus-Esterase, z.B. aus Orangenschalen, freisetzen kann), mit einem geeigneten Kohlensäurederivat, insbesondere mit einer Isocyanat- oder Carbaminsäure-verbindung, wie einem Silylisocyanat, z.B. Silyltetraisocyanat, einem Sulfonylisocyanat, z.B. Chlorsulfonylisocyanat, oder Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono oder N,N-disubstituierten Carbaminsäure-Verbindungen, wie einem entsprechenden Carbaminsäurehalogenid, z.B. -chlorid, umsetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatmosphäre, arbeitet.

Ferner kann man eine Verbindung der Formel I, worin $R_1$ eine Gruppe $—CH_2—R_2$ bedeutet, wobei $R_2$ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedingungen, bevorzugt bei einem pH-Wert von etwa 6,5, in Gegenwart von Wasser und gegebenenfalls in einem, mit Wasser mischbaren organischen Lösungsmittel umsetzen und so zu Verbindungen der Formel I gelangen, worin $R_1$ den Rest der Formel $—CH_2—R_2$ darstellt und $R_2$ für eine quaternäre Ammoniumgruppe steht. Die schwach-sauren Bedingungen können durch Zugabe einer geeigneten organischen oder anorganischen Säure, beispielsweise Essigsäure, Chlorwasserstoffsäure, Phosphorsäure oder auch Schwefelsäure eingestellt werden. Als organische Lösungsmittel können beispielsweise die vorstehend genannten, mit Wasser mischbaren Lösungsmittel verwendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäuren, z.B. Chlorwasserstoff- und insbesondere Jodwasserstoffsäure, sowie der Thiocyansäure, oder organischen Säuren, wie Niederalkancarbonsäuren, z.B. Essigsäure. Vertreter solcher Salze sind beispielsweise Kaliumjodid und Kaliumthiocyanat. Auch Salze von geeigneten Anionenaustauschern, z.B. flüssige Ionenaustauscher in Salzform, wie z.B. Amberlite LA-1 (flüssige sekundäre Amine mit einem Molekulargewicht von 351—393; Oel-löslich und Wasserunlöslich; mAeq./g = 2,5—2,7, z.B. in Acetatform), mit Säuren, z.B. Essigsäure, können für diesen Zweckverwendet werden.

Quaternäre Ammoniumgruppen $R_2$ können vorteilhafterweise unter Verwendung eines Zwischenprodukts der Formel I, in welchem $R_2$ für eine substituierte, insbesondere für eine aromatisch substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin $R_2$ im Rest $R_1$ eine veresterte Hydroxygruppe, insbesondere eine Niederalkanoyloxy- z.B. Acetyloxygruppe bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegebenenfalls substituierten Pyridin, umgesetzt, wobei man die quaternäre Ammoniumverbindung erhält. Die Reaktion wird üblicherweise in Gegenwart eines geeigneten Entschwefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatmosphäre, durchgeführt.

Die Ueberführung einer freien Carboxylgruppe in einer erhaltenen Verbindung der Formel I in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere funktionelle Gruppen, wie Amino-, Hydroxy- oder Sulfogruppen, gegebenenfalls in geschützter

Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

Salze von Verbindungen der Formel I können in an sich bekannter Weise hergestellt werden. So kann man Salze von Verbindungen der Formel I mit sauren Gruppen, z.B. durch Behandeln mit Metallverbindungen, wie Alkalimetallsalzen von geeigneten Carbonsäuren, z.B. dem Natriumsalz der $\alpha$-Aethyl-capronsäure oder Natriumbicarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Die Ausgangsverbindungen der Formel I, worin mindestens eine der funktionellen Gruppen in geschützter Form vorliegt, und Verfahren zu ihrer Herstellung sind ebenfalls Gegenstand der vorliegenden Erfindung. Diese Verbindungen können in an sich bekannter Weise hergestellt werden, indem man z.B.

a) in einer Verbindung der Formel

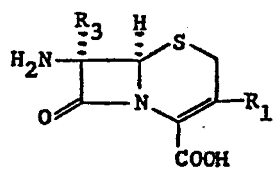

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einer Säure der Formel

$$HOOC-\underset{\displaystyle NH_2}{\underset{|}{CH}}-(C_nH_{2n})-X-W-NH-(C_mH_{2m})-A-\overset{\displaystyle Y}{\overset{|}{\underset{\displaystyle Z}{\underset{|}{C}}}}-\overset{\displaystyle O}{\overset{\|}{C}}-OH \qquad (III),$$

worin die Aminocarbonsäuregruppierung $HOOC-CH(NH_2)-$ und gegebenenfalls in der Gruppierung $-A-C(Y)(Z)-$ vorhandene weitere funktionelle Gruppen in geschützter Form vorliegen, oder mit einem reaktionsfähigen funktionellen Säurederivat oder einem Salz davon acyliert, oder

b) in einer Verbindung der Formel

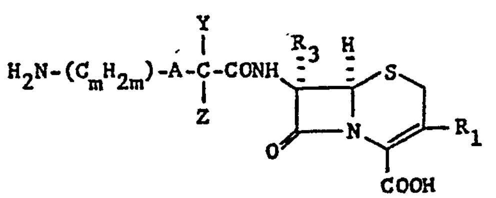

(IV),

worin die Aminogruppe gegebenenfalls durch eine, die Acylierung erlaubende Gruppe substituiert sein kann, und worin die 4-Carboxylgruppe und gegebenenfalls im Rest $R_1$ und in der Gruppierung $-A-C(Y)(Z)-$ vorhandene weitere funktionelle Gruppen in geschützter Form vorliegen können, die

Aminogruppe durch Behandeln mit einem reaktionsfähigen funktionellen Derivat einer Säure der Formel

$$HOOC—CH—(C_nH_{2n})—X—W—OH$$
$$|$$
$$NH_2$$
(V),

worin die Aminocarbonsäuregruppierung $HOOC—CH(NH_2)—$ in geschützter Form vorliegt, oder wenn X—W zusammen eine Gruppe —CO— bedeuten, auch mit einer entsprechenden freien Säure oder mit einem Salz davon, acyliert, oder

c) in einer Verbindung der Formel

$$HOOC—CH—(C_nH_{2n})—X—H$$
$$|$$
$$NH_2$$
(VI),

worin X Sauerstoff, Schwefel oder die Gruppe —NH— bedeutet, und worin die Aminocarbonsäuregruppierung $HOOC—CH(NH_2)—$ in geschützter Form vorliegt, die Gruppe —X—H mit einem reaktionsfähigen funktionellen Derivat einer Verbindung der Formel

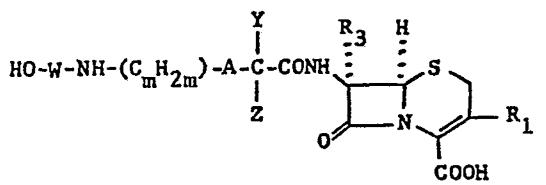
(VII),

worin die 4-Carboxylgruppe und gegebenenfalls im Rest $R_1$ und in der Gruppierung —A—C(Y)(Z)— vorhandene funktionelle Gruppen in geschützter Form vorliegen können, acyliert, und, wenn gewünscht, in einer erhaltenen Verbindung noch nicht geschützte funktionelle Gruppen schützt oder eine Schutzgruppe gegen eine andere austauscht, und/oder wenn gewünscht, in einem Rest $R_1$ eine Gruppe $R_2$ gegen eine andere Gruppe $R_2$ austauscht, und/oder, wenn gewünscht, eine erhaltene Verbindung, worin $R_3$ Wasserstoff ist, in eine Verbindung überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in dei einzelnen Isomeren auftrennt.

Gegebenenfalls vorhandene, die Aminogruppe substituierende und deren Acylierung erlaubende Reste in einem Ausgangsmaterial der Formel II oder IV sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. bei der Silylierung oder Stannylierung einer Carboxylgruppe in einem Ausgangsmateral der Formel II oder IV, kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht; solche Arylmethylengruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Die übrigen in den Ausgangsstoffen der Formel II bis VII vorhandenen funktionellen Gruppen können durch die bereits unter den Verbindungen der Formel I genannten Schutzgruppen geschützt sein. Bevorzugt sind alle nicht an der Acylierungsreaktion teilnehmenden reaktionsfähigen funktionellen Gruppen, insbesondere aber gegebenenfalls vorhandene, acylierbare Amino-, Hydroxy- und Mercaptogruppen, geschützt.

Falls eine freie Säure der Formel III oder V, worin alle funktionellen Gruppen ausser der reagierenden Carboxylgruppe geschützt sind, zur Acylierung eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazolinium-perchlorat, oder eine Acylaminoverbindung, z.B. 2-Aethoxy-1-äthoxycarbonyl-1,2-dihydro-chinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder

Erwärmen und/oder in einer Inertgasatmosphäre, durchgeführt.

Ein reaktionsfähiges, d.h. Amid-bildendes, bzw. Esterbildendes, funktionelles Derivat einer Säure der Formel III, V oder VII, worin alle funktionelle Gruppen ausser der reagierenden Säuregruppe geschützt sind bzw. sein können, ist in erster Linie ein Anhydrid einer solchen Säure, inklusive und vorzugsweise ein gemischtes Anhydrid, aber auch ein inneres Anhydrid, d.h. ein entsprechendes Keten oder, in der Säure V, wenn W die Gruppe —$SO_2$NH—CO— ist, oder wenn X die Gruppe —NH— und W die Gruppe —CO— oder —CO—$NHSO_2$— ist, ein entsprechendes Isocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhaltigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure. Von der Säure III, wenn Z Wasserstoff ist und Y Hydroxy bedeutet, kann auch ein gemischtes inneres Anhydrid mit dem Kohlensäurehalbester der $\alpha$-Hydroxygruppe verwendet werden.

Weitere, zur Reaktion mit der Amino-, Hydroxy- oder Mercaptogruppe geeignete Säurederivate in einer Säure der Formel III, V, oder VIII, worin alle funktionellen Gruppen ausser der reagierenden Carboxylgruppe geschützt sind, bzw. sein können, sind aktivierte Ester, wie Ester mit vinylogen Alkoholen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-benztriazolester, oder Diacyliminoester, wie Succinylimino- oder Phthalyliminoester.

Die Acylierung mit einem Säurederivat, wie einem Anhydrid, insbesondere mit einem Säurehalogenid, kann in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt werden.

Die obigen Acylierungen können in einem inerten, vorzugsweise wasserfreien Lösungsmittel oder Lösungsmittelgemisch vorgenommen werden, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.G. Essigsäureäthylester, oder einem Nitril, z.B. Acetonitril, oder Mischungen davon, und, wenn notwendig, bei erniedrigter oder erhöhter Temperatur, etwa bei —40° bis etwa 100°, bevorzugt bei —10° bis +40°, und/oder in einer Inertgas-, z.B. Stickstoffatmosphäre.

In einer acylierenden Säure der Formel III, V oder VII oder in einem Säurederivat davon kann eine geschützte Aminogruppe auch in ionischer Form vorliegen, d.h. das Ausgangsmaterial der Formel III, V oder VII kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, *in situ* gebildet werden. So erhält man z.B. ein gemischtes Anhydrid durch Behandeln einer Säure der Formel III, oder einer Säure der Formel V, worin X—W die Gruppe —CO— bedeutet, mit entsprechend geschützten funktionellen Gruppen, oder eines geeigneten Salzes davon, wie eines Ammoniumsalzes, z.B. mit einem organischen Amin, wie 4-Methylmorpholin oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Ein Säurechlorid einer Säure der Formel V, worin X—W eine Gruppierung —O—CO—, —S—CO— oder —NH—CO— ist und worin die Aminocarbonsäuregruppierung HOOC—CH($NH_2$)— in geschützter Form vorliegt, kann, z.B. *in situ*, gebildet werden, indem man eine Aminocarbonsäure der Formel VI, worin X Sauerstoff, Schwefel oder die Gruppe —NH— bedeutet, und die Gruppierung HOOC—CH($NH_2$)— in geschützter Form vorliegt, in Gegenwart eines Salzsäurereakzeptors in einem inerten organischen Lösungsmittel oder Lösungsmittelgemisch mit Phosgen behandelt. Die Salzsäurereakzeptoren, Lösungsmittel und Reaktionsbedingungen sind die gleichen, die für die Acylierung von Verbindungen der Formel II oder IV genannt wurden; beispielsweise kann die Reaktion in Gegenwart von Pyridin in Methylenchlorid und Toluol bei etwa 0° bis eta +10° stattfinden.

Auf die gleiche Weise kann, z.B. *in situ,* ein Säurechlorid einer Säure der Formel VII, worin W die Gruppe —CO— bedeutet, und worin die 4-Carboxylgruppe und in den Gruppen —A—C(Y)(Z)— und $R_1$ gegebenenfalls vorhandene weitere funktionelle Gruppen geschützt sind, aus einer entsprechend

geschützten Verbindung der Formel IV, durch Behandlung mit Chlorsulfonylisocyanat hergestellt werden.

In einem erhaltenen Zwischenprodukt der Formel I, worin mindestens eine der funktionellen Gruppen in geschützter Form vorliegt, können auf übliche, an sich bekannte Weise, noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

In einem erhaltenen Zwischenprodukt der Formel I, worin mindestens eine der funktionellen Gruppen in geschützter Form vorliegt, kann eine im Rest $R_1$ vorhandene Gruppe $R_2$ gegen eine andere Gruppe $R_2$ ausgetauscht werden, wie für diese Reaktion bei den Endprodukten der Formel I angegeben ist.

In einem erhaltenen Zwischenprodukt der Formel I, worin $R_3$ Wasserstoff ist und alle funktionellen Gruppen in geschützter Form vorliegen, kann die $7\alpha$-Methoxygruppe $R_3$ auf an sich bekannte Weise eingeführt werden, beispielsweise indem man das genannte Zwischenprodukt nacheinander mit einem Anionen-bildenden Mittel, einem N-Halogenierungsmittel und Methanol behandelt.

Ein geeignetes Anion-bildendes Mittel ist in erster Linie eine metallorganische Base, insbesondere eine Alkalimetall-, in erster Linie eine Lithium-organische Base. Solche Verbindungen sind insbesondere entsprechende Alkoholate, wie geeignete Lithium-niederalkanolate, in erster Linie Lithiummethylat, oder entsprechende Metall-Kohlenwasserstoffbasen, wie Lithium-niederalkane und Lithiumphenyl. Die Umsetzung mit der Anion-bildenden metallorganischen Base wird üblicherweise unter Kühlen, z.B. von etwa 0°C bis etwa —80°C, und in Gegenwart eines geeigneten Lösungs- oder Verdünnungsmittels, z.B. eines Aethers, wie Tetrahydrofuran, bei Verwendung von Lithiummethylat auch in Gegenwart von Methanol, und, wenn erwünscht, in einem geschlossenen Gefäss und/oder in einer Inertgas-, z.B. Stickstoffatmosphäre, vorgenommen.

Als N-halogenierendes Mittel verwendet man üblicherweise ein sterisch gehindertes, organische Hypohalogenit, insbesondere -chlorit, und in erster Linie ein entsprechendes aliphatisches Hypohalogenit, z.B. -chlorit, wie ein tert.-Niederalkyl-hypohalogenit, z.B. -chlorit. In erster Linie wendet man das tert.-Butylhypochlorit an, das man mit dem nichtisolierten Produkt der Anionisierungsreaktion umsetzt.

Die N-halogenierte Zwischenverbindung wird bei Anwesenheit eines Ueberschusses der Anion-bildenden Base, insbesondere von Lithiummethylat, unter den Reaktionsbedingungen und ohne isoliert zu werden in eine 7-Acyliminocephemverbindung umgewandelt, und diese durch Zugabe von Methanol in eine $7\alpha$-Methoxy-cephem-Verbindung übergeführt. Falls notwendig, müssen aus dem N-halogenierten Zwischenprodukt die Elemente der Halogenwasserstoff-, insbesondere der Chlorwasserstoffsäure, abgespalten werden; dies geschieht unter zugabe einer Halogenwasserstoff-abspaltenden Base, wie eines geeigneten Alkalimetall-niederalkanolats, z.B. Lithium-tert.-butylat, wobei diese Reaktion üblicherweise unter den Bedingungen der Anion- und N-Halogenverbindung-bildenden Reaktion stattfindet, wobei man in Gegenwart von Methanol arbeiten und anstelle der Acyliminoverbindung direkt die $7\alpha$-Methoxy-cephem-Verbindung erhalten kann. Man geht üblicherweise aus von einer Verbindung der Formel I, worin funktionelle Gruppen in geschützter Form vorliegen, setzt diese mit einem Ueberschuss des Anion-bildenden Mittels, z.B. Lithiummethylat oder Phenyllithium, in Gegenwart von Methanol um, behandelt dann mit dem N-Halogenierungsmittel, z.B. tert.-Butylhypochlorit, und erhält so direkt die gewünschte Verbindung der Formel I, worin funktionelle Gruppen geschützt sind. Man kann auch das Methanol nachträglich zugeben wobei man die Dehydrohalogenierung und die Zugabe von Methanol bei etwas höheren Temperaturen als die Anion- und N-Halogenverbindung-bildenden Reaktionen, z.B. bei etwa 0°C bis etwa —20°C, wenn notwendig, in einem geschlossenen Gefäss und/oder in einer Inertgas-, z.B. Stickstoffatmosphäre, durchführen kann.

Bei vorstehenden Reaktionen, die unter basischen Bedingungen durchgeführt werden, können 3-Cephem-verbindungen, gegebenenfalls teilweise, zu 2-Cephemverbindungen isomerisieren. Eine erhaltene 2-Cephemverbindung oder ein Gemisch aus einer 2- und einer 3-Cephemverbindung kann in an sich bekannter Weise zur gewünschten 3-Cephemverbindung isomerisiert werden.

Diese Isomerisierung kann beispielsweise durchgeführt werden, indem man die erhaltene 2-Cephemverbindung oder das erhaltene Gemisch der 2- und 3-Cephemverbindung in 1-Stellung oxidiert und die so erhältlichen 1-Oxide der entsprechenden 3-Cephem-Verbindungen reduziert.

Als geeignete Oxydationsmittel für die Oxydation in 1-Stellung von Cephemverbindung kommen beispielsweise anorganische Persäuren, die ein Reduktionspotential von wenigstens +1,5 Volt aufweisen und aus nicht-metallischen Elementen bestehen, organische Persäuren oder Gemischen aus Wasserstoffperoxyd und Säuren, insbesondere organische Carbonsäuren mit einer Dissoziationskonstante von wenigstens $10^{-5}$, in Frage. Geeignete anorganische Persäuren sind Perjod- und Perschwefelsäure. Organische Persäuren sind entsprechende Percarbon- und Persulfonsäuren, die als solche zugesetzt oder durch Verwendung von wenigstens einem Aequivalent Wasserstoffperoxyd und einer Carbonsäure *in situ* gebildet werden können. Dabei ist es zweckmässig, einen grossen Ueberschuss der Carbonsäure zu verwenden, wenn z.B. Essigsäure als Lösungsmittel verwendet wird. Geeignete Persäuren sind z.B. Perameisensäure, Peressigsäure, Pertrifluoressigsäure, Permaleinsäure, Perbenzoesäure, 3-Chlorperbenzoesäure, Monoperphthalsäure oder p-Toluolpersulfonsäure.

Die Oxydation kann ebenfalls unter Verwendung von Wasserstoffperoxyd mit katalytischen

Mengen einer Säure mit einer Dissoziationskonstante von wenigstens $10^{-5}$ durchgeführt werden, wobei man niedrige Konzentrationen, z.B. 1—2% und weniger, aber auch grössere Mengen der Säure einsetzen kann. Dabei hängt die Wirksamkeit des Gemisches in erster Linie von der Stärke der Säure ab. Geeignete Gemische sind z.B. solche von Wasserstoffperoxyd mit Essigsäure, Perchlorsäure oder Trifluoressigsäure.

Die obige Oxydation kann in Gegenwart von geeigneten Katalysatoren durchgeführt werden. So kann z.B. die Oxydation mit Percarbonsäuren durch die Anwesenheit einer Säure mit einer Dissoziationskonstante von wenigstens $10^{-5}$ katalysiert werden, wobei ihre Wirksamkeit von ihrer Stärke abhängt. Als Katalysatoren geeignete Säuren sind z.B. Essigsäure, Perchlorsäure und Trifluoressigsäure. Ueblicherweise verwendet man mindestens äquimolare Mengen des Oxydationsmittels, vorzugsweise einen geringen Ueberschuss von etwa 10% bis etwa 20%. Die Oxydation wird unter milden Bedingungen, z.B. bei Temperaturen von etwa —50°C bis etwa +100°C, vorzugsweise von etwa —10°C bis etwa +40°C durchgeführt.

Die Oxydation von 2-Cephem-Verbindungen zu den 1-Oxyden der entsprechenden 3-Cephemverbindungen kann auch durch Behandeln mit Ozon, ferner mit organischen Hypohalogenitverbindungen, wie Niederalkyl-hypochloriten, z.B. tert.-Butylhypochlorit, die man in Gegenwart von inerten Lösungsmitteln, wie gegebenenfalls halogenierten Kohlenwasserstoffen, z.B. Methylenchlorid, und bei Temperaturen von etwa —10°C bis etwa +30°C verwendet, mit Perjodatverbindungen, wie Alkalimetallperjodaten, z.B. Kaliumperjodat, die man vorzugsweise in einem wässrigen Medium bei einem pH-Wert von etwa 6 und bei Temperaturen von etwa —10°C bis etwa +30°C verwendet, mit Jodbenzoldichlorid, das man in einem wässrigen Medium, vorzugsweise in Gegenwart einer organischen Base, z.B. Pyridin, und unter Kühlen, z.B. bei Temperaturen von etwa —20°C bis etwa 0°, verwendet, oder mit irgendeinem anderen Oxydationsmittel durchgeführt werden, das sich zur Umwandlung einer Thioin eine Sulfoxydgruppierung eignet.

Die Reduktion der 1-Oxyde von 3-Cephem-Verbindungen kann in an sich bekannter Weise durch Behandeln mit einem Reduktionsmittel, wenn notwendig, in Anwesenheit eines aktiviertenden Mittels, durchgeführt werden. Als Reduktionsmittel kommen beispielsweise in Betracht: Katalytisch aktivierter Wasserstoff, wobei Edelmetallkatalysatoren verwendet werden, welche Palladium, Platin oder Rhodium enthalten, und die man gegebenenfalls zusammen mit einem geeigneten Trägermaterial, wie Kohle oder Bariumsulfat, einsetzt; reduzierende Zinn-, Eisen-, Kupfer- oder Mangankationen, welche in Form von entsprechenden Verbindungen oder Komplexen anorganischer oder organischer Art, z.B. als Zinn-II-chlorid, -fluorid, -acetat oder formiat, Eisen-II-chlorid, -sulfat, -oxalat oder -succinat, Kupfer-I-chlorid, -benzoat oder -oxyd, oder Mangan-II-chlorid, -sulfat, -acetat oder -oxyd, oder als Komplexe, z.B. mit Aethylendiamintetraessigsäure oder Nitrolotriessigsäure, verwendet werden; reduzierende Dithionit-, Jod- oder Eisen-II-cyanid-anionen, welche in Form von entsprechenden anorganischen oder organischen Salzen, wie Alkalimetall-, z.B. Natrium- oder Kaliumdithionit, Natrium- oder Kaliumjodid oder -eisen-II-cyanid, oder in Form der entsprechenden Säuren, wie Jodwasserstoffsäure, verwendet werden; reduzierende trivalente anorganische oder organische Phosphorverbindungen, wie Phosphine, ferner Ester, Amide und Halogenide der phosphinigen, phosphonigen oder phosphorigen Säure, sowie diesen Phosphorsäurestoffverbindungen entsprechenden Phosphor-Schwefelverbindungen, worin organische Reste in erster Linie aliphatische, aromatische oder araliphatische Reste, z.B. gegebenenfalls substituierte Niederalkyl-, Phenyl oder Phenylniederalkylgruppen darstellen, wie z.B. Triphenylphosphin, Tri-n-butylphosphin, Diphenylphosphinigsäuremethylester, Diphenylchlorphosphin, Phenyldichlorphosphin, Benzolphosphonigsäuredimethylester, Butanphosphonigsäuremethylester, Phosphorigsäuretriphenylester, Phosphorigsäuretrimethylester, Phosphortrichlorid, Phosphortribromid, etc.; reduzierende Halogensilanverbindungen, die mindestens ein an das Siliciumatom gebundenes Wasserstoffatom aufweisen, und die ausser Halogen, wie Chlor, Brom oder Jod, auch organische Reste, wie aliphatische oder aromatische Gruppen, z.B. gegebenenfalls substituierte Niederalkyl- oder Phenylgruppen aufweisen können, wie Chlorsilan, Bromsilan, Di- oder Trichlorsilan, Di- oder Tribromsilan, Diphenylchlorsilan, Dimethylchlorsilan, etc.; reduzierende quaternäre Chlormethylen-iminiumsalze, insbesondere -chloride oder -bromide, worin die Iminiumgruppe durch einen bivalenten oder zwei monovalente organische Reste, wie gegebenenfalls substituierte Niederalkylen- oder Niederalkylgruppen substituiert ist, wie N-Chlormethylen-N,N-diäthyliminiumchlorid oder N-Chlormethylenpyrrolidiniumchlorid; und komplexe Metallhydride, wie Natriumborhydride, in Gegenwart von geeigneten Aktivierungsmitteln, wie Cobalt-II-chlorid, sowie Borandichlorid.

Als aktivierende Mittel, die zusammen mit denjenigen der obgenannten Reduktionsmittel verwendet werden, welche selber nicht Lewissäure-Eigenschaften aufweisen, d.h. die in erster Linie zusammen mit den Dithionit-, Jod- oder Eisen-II-cyanid- und den nicht-halogenhaltigen trivalenten Phosphor-Reduktionsmitteln oder bei der katalytischen Reduktion eingesetzt werden, sind insbesondere organische Carbon- und Sulfonsäurehalogenide, ferner Schwefel-, Phosphor- oder Siliciumhalogenide mit gleicher oder grösserer Hydrolysenkonstante zweiter Ordnung als Benzoylchlorid, z.B. Phosgen, Oxalylchlorid, Essigsäurechlorid oder -bromid, Chloressigsäurechlorid; Pivalinsäurechlorid, 4-Methoxybenzoesäurechlorid, 4-Cyanbenzoesäurechlorid, p-Toluolsulfonsäurechlorid, Methansulfonsäurechlorid, Thionylchlorid, Phosphoroxychlorid, Phosphortrichlorid, Phosphortribromid, Phenyldichlorphosphin, Benzolphosphonigsäuredichlorid, Dimethylchlorsilan oder Trichlorsilan, ferner geeignete Säureanhydride, wie Trifluoressigsäureanhydrid, oder cyclische Sultone, wie Aethansulton, 1,3-Propansulton, 1,4-Butansulton oder 1,3-Hexansulton zu erwähnen.

Die Reduktion wird vorzugsweise in Gegenwart von Lösungsmitteln oder Gemischen davon durchgeführt, deren Auswahl in erster Linie durch die Löslichkeit der Ausgangsstoffe und die Wahl des Reduktionsmittels bestimmt wird, so z.B. Niederalkancarbonsäuren oder Ester davon, wie Essigsäure und Essigsäureäthylester, bei der katalytischen Reduktion, und z.B. gegebenenfalls substituierte, wie halogenierte oder nitrierte aliphatische, cycloaliphatische, aromatische oder araliphatische Kohlenwasserstoffe, z.B. Benzol, Methylenchlorid, Chloroform oder Nitromethan, geeignete Säurederivate, wie Niederalkancaronsäureester oder -nitrile, z.B. Essigsäureäthylester oder Acetonitril, oder Amide von anorganischen oder organischen Säuren, z.B. Dimethylformamid oder Hexamethylphosphoramid, Aether, z.B. Diäthyläther, Tetrahydrofuran oder Dioxan, Ketone, z.B. Aceton, oder Sulfone, insbesondere aliphatische Sulfone, z.B. Dimethylsulfon oder Tetramethylensulfon, etc., zusammen mit den chemischen Reduktionsmitteln, wobei diese Lösungsmittel vorzugsweise kein Wasser enthalten. Dabei arbeitet man gewöhnlicherweise bei Temperaturen von etwa —20°C bis etwa 100°C, wobei bei Verwendung von sehr reaktionsfähigen Aktivierungsmitteln die Reaktion bei tieferen Temperaturen durchgeführt werden kann.

Ausgangsverbindungen der Formel II sowie entsprechende Verbindungen mit geschützten funktionellen Gruppen sind bekannt oder können auf an sich bekannte Weise hergestellt werden.

Verbindungen der Formel III, worin die Aminocarbonsäuregruppierung $HOOC—CH(NH_2)—$ und gegebenenfalls in der Gruppierung —A—C(Y)(Z)— vorhandene weitere funktionelle Gruppen in geschützter Form vorliegen, sind neu.

Solche Verbindungen der Formel III, mit entsprechend geschützten funktionellen Gruppen, werden beispielsweise hergestellt, indem man eine Verbindung der Formel

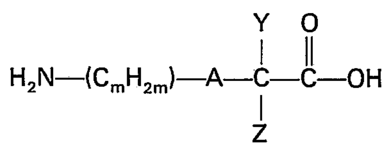

(VIII),

worin die Aminogruppe gegebenenfalls durch eine die Acylierung erlaubende Gruppe substituiert sein kann, und gegebenenfalls in der Gruppierung —A—C(Y)(Z)— vorhandene funktionelle Gruppen geschützt sind, unter intermediärem Schutz der Carboxylgruppe, mit einem reaktionsfähigen funktionellen Derivat einer Säure der Formel V, worin die Aminocarbonsäuregruppierung $HOOC—CH(NH_2)—$ in geschützter Form vorliegt, oder, wenn X—W zusammen eine Gruppe —CO— bedeuten, auch mit einer entsprechenden freien Säure oder mit einem Salz davon, acyliert, und wenn erwünscht, eine erhaltene Verbindung in eine andere Verbindung der Formel III mit entsprechend geschützten funktionellen Gruppen überführt.

Die die Acylierung erlaubenden Gruppen, sowie die Schutzgruppen der Aminocarbonsäuregruppierung $HOOC—CH(NH_2)—$ und der Gruppierung —A—C(Y)(Z)—, sind die gleichen wie die unter den Verbindungen der Formeln II oder IV, sowie I erwähnten. Zum intermediären Schutzt der Carboxylgruppe in der Verbindung der Formel VIII können an sich ebenfalls die bereits erwähnten Carboxylschutzgruppen verwendet werden, jedoch müssen sich die zum intermediären Schutz in der vorliegenden Acylierung benützten Carboxylschutzgruppen von den übrigen Schutzgruppen, die in den Verbindungen der Formel III notwendigerweise vorhanden bleiben sollen, in der Art ihrer Abspaltung unterscheiden, so dass sie nach der vorliegenden Acylierungsreaktion selektiv abgespalten werden können. Wenn beispielsweise zum intermediären Schutz der Carboxylgruppe eine hydrogenolytisch abspaltbare Schutzgruppe, wie eine der genannten gegebenenfalls substituierten Benzylgruppen, z.B. die Benzyl- oder p-Nitrobenzylgruppe, verwendet wird, dann dürfen die anderen Schutzgruppen hydrogenolytisch nicht abspaltbar sein; sie können beispielsweise die genannten, nur acidolytisch abspaltbaren tert.-Niederalkylgruppen, wie tert.Butyl bzw. tert.-Niederalkoxycarbonylgruppen, wie tert.-Butoxycarbonyl, sein.

Die Acylierung kann im übrigen analog ausgeführt werden wie die Acylierung von Verbindungen der Formel IV mit einer Säure der Formel V, bzw. einem entsprechend geschützten und reaktionsfähigen funktionellen Derivat davon.

In einer erhaltenen Verbindung der Formel III, mit entsprechend geschützten funktionellen Gruppen, kann eine Schutzgruppe, gegebenenfalls selektiv, abgespalten, oder eine, gegebenenfalls bi der Acylierungsreaktion frei gewordene, funktionelle Gruppe geschützt werden. In einer erhaltenen Verbindung der Formel III, worin Z Wasserstoff und Y Hydroxy bedeutet, kann die $\alpha$-Hydroxygruppe oxidativ, wie für die Oxidation von Verbindungen XI zu $\alpha$-Ketosäuren IX angegeben, z.B. unter vorübergehendem Schutz der Carboxylgruppe als Ester, durch Behandeln mit Mangandioxid, in eine $\alpha$-Oxogruppe übergeführt werden und in einer erhältlichen Verbindung der Formel III, worin Z und Y zusammen die Oxogruppe bedeuten, kann diese durch Behandeln mit einem Hydroxylamin der Formel $H_2N—O—R°$, in die entsprechende Oximinogruppe übergeführt werden, welche Reaktion

analog der Ueberführung von $\alpha$-Ketosäuren der Formel IX in Oximinoverbindungen der Formel VIIIa ausgeführt werden kann.

Verbindungen der Formel IV, worin die Aminogruppe gegebenenfalls durch eine die Acylierung erlaubende Gruppe substituiert sein kann, und worin die 4-Carboxylgruppe und gegebenenfalls im Rest $R_1$ und in der Gruppierung —A—C(Y)(Z)— vorhandene weitere funktionelle Gruppen in geschützter Form vorliegen können, sind zum Teil bekannt und zum Teil neu. Sie können nach an sich bekannten Verfahren hergestellt werden.

In den neuen Verbindungen der Formel IV haben der Index m, A, $R_1$ und $R_3$ die unter der Formel I genannten Bedeutungen und X und Z bedeuten zusammen eine Gruppe =N—O—$R^o$, worin $R^o$ Wasserstoff oder gegebenenfalls substituiertes Niederalkyl darstellt. Diese neuen Verbindungen, worin die Carboxylgruppe gegebenenfalls in physiologisch spaltbarer Form verestert ist, sowie ihre pharmazeutisch verwendbaren Salze, haben ebenfalls antibiotische Wirksamkeit. Zusammen mit dem Verfahren zu ihrer Herstellung sind sie ebenfalls Gegenstand der vorliegenden Erfindung.

Die genannten neuen, antibiotisch wirksamen Verbindungen der Formel IV können als antibakterielle Antibiotika verwendet werden. Beispielsweise sind sie *in vitro* gegen Enterobacteriaceen, z.B. *Escherichia coli*, in Minimalkonzentrationen von 0,8 mcg/ml und gegen Kokken in Minimalkonzentrationen von 0,1 mcg/ml wirksam. *In vivo*, bei subcutaner Applikation an der Maus, sind sie beispielsweise gegen Enterobacteriaceen, wie *Escherichia coli*, in Minimaldosen von 6 mg/kg und gegen Kokken in Minimaldosen von 15 mg/kg wirksam. Die neuen Verbindungen können deshalb entsprechend, z.B. in Form von antibiotisch wirksamen Präparaten zur Behandlung von durch Enterobakterien, wie *Escherichia coli*, und Kokken verursachten Infektionen Verwendung finden.

Bevorzugt sind solche Verbindungen der Formel IV, worin der Index m den Wert 1 hat, A Thienylen, insbesondere 2,5-Thienylen, oder Furylen, insbesondere 2,5-Furylen, bedeutet, Y und Z zusammen eine Gruppe =N—O—$R^o$ darstellen, insbesondere in syn-Konfiguration, worin $R^o$ Wasserstoff, Niederalkyl, insbesondere Methyl, oder substituiertes Niederalkyl, insbesondere Carboxyniederalkyl, wie 2-Carboxyäthyl oder 3-Carboxypropyl, bedeutet, $R_1$ eine Gruppe —$CH_2R_2$ bedeutet, worin $R_2$ Acetoxy, Carbamoyloxy oder Heterocyclylthio, insbesondere substituiertes Tetrazolylthio, wie 1-Methyltetrazol-5-ylthio, darstellt, und $R_3$ Wasserstoff ist, sowie Salze davon.

Verbindungen der Formel IV, worin gegebenenfalls die Aminogruppe und die anderen funktionellen Gruppen wie angegeben substituiert bzw. geschützt sein können, sowie ihre Salze, können hergestellt werden, indem man eine Verbindung der Formel II, worin die Aminogruppe gegebenenfalls durch eine die Acylierung erlaubende Gruppe substituiert ist und worin die 4-Carboxylgruppe und gegebenenfalls im Rest $R_1$ vorhandene weitere funktionelle Gruppen in geschützter Form vorliegen können, mit einer Säure der Formel VIII, worin die Aminogruppe gegebenenfalls geschützt sein kann, und gegebenenfalls in der Gruppierung —A—C(Y)(Z)— vorhandene funktionelle Gruppen geschützt sind, oder mit einem reaktionsfähigen funktionellen Derivat einer solchen Säure oder einem Salz davon, acyliert, wenn gewünscht in einer erhaltenen Verbindung die Schutzgruppen abspaltet und/oder, wenn gewünscht, in einer erhaltenen Verbindung eine Gruppe $R_1$ in eine andere Gruppe $R_1$ umwandelt, und/oder, wenn gewünscht, eine erhaltene Verbindung, worin $R_3$ Wasserstoff ist, in eine Verbindung überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in die Isomeren auftrennt, und/oder wenn gewünscht, eine erhaltene Verbindung mit salzbildender Gruppe in ein Salz oder ein erhaltenes Salz in die freie Verbindung überführt.

Die die Acylierung erlaubenden Gruppen in den Verbindungen der Formel II und VIII, sowie die Schutzgruppen, sind die gleichen, die bereits oben erwähnt wurden.

Die Acylierung von entsprechenden geschützten Verbindungen der Formel II mit einer Säure der Formel VIII, oder mit einem reaktionsfähigen funktionellen Derivat davon, die Abspaltung der Schutzgruppen, die Ueberführung einer Gruppe $R_1$ in eine andere Gruppe $R_1$, und die Einführung der Methoxygruppe $R_3$, sowie die Salzbildung können analog ausgeführt werden wie bei der Acylierung von entsprechend geschützten Verbindungen der Formel II oder IV mit einer Säure der Formel III bzw. V und den entsprechenden Nachoperationen angegeben ist.

Säuren der Formel V, reaktionsfähige funktionelle Derivate davon, die Vorstufen der Formel·

$$HOOC—CH(NH_2)—(C_nH_{2n})—X—H \qquad (VI)$$

und entsprechend geschütze Derivate sind bekannt oder können nach an sich bekannten Methoden, beispielsweise *in situ*, hergestellt werden.

Reaktionsfähige funktionelle Derivate von Säuren der Formel VII, worin die 4-Carboxylgruppe und gegebenenfalls im Rest $R_1$ und in der Gruppierung —A—C(Y)(Z)— vorhandene funktionelle Gruppen in geschützter Form vorliegen können, werden auf an sich bekannte Weise aus entsprechend geschützten Verbindungen der Formel IV hergestellt.

Säuren der Formel VIII und entsprechende reaktionsfähige funktionelle und geschützte Derivate davon sind zum Teil bekannt oder können nach an sich bekannten Methoden hergestellt werden.

Die unter die Formel VIII fallenden Säuren der Formel

$$H_2N—(C_mH_{2m})—A—\underset{}{C}(=N—O—R^o)—COOH \quad (VIIIa),$$

worin der Index m den Wert 1 hat, und A und R° die unter Formel I genannten Bedeutungen haben, sowie entsprechende an der Aminogruppe geschützte, sowie bezüglich der Carboxylgruppe reaktionsfähige funktionelle Derivate davon sind neu und können auf an sich bekannte Weise hergestellt werden, indem man eine $\alpha$-Ketosäure der Formel

$$H_2N—(C_mH_{2m})—A—C(=O)—COOH \quad (IX)$$

worin m und A die unter Formel VIIIa genannten Bedeutungen haben und worin die Aminogruppe in geschützter Form vorliegt, mit einer Hydroxylaminoverbindung der Formel $H_2N—O—R^o$, worin R° die unter Formel VIIIa genannte Bedeutung hat, behandelt und, wenn gewünscht, in einer erhaltenen Verbindung der Formel VIIIa mit geschützter Aminogruppe, diese in die freie Aminogruppe und/oder, wenn gewünscht, die erhaltene freie Aminogruppe in eine andersartig geschützte Aminogruppe überführt, und/oder, wenn gewünscht, ein erhaltenes Isomerengemisch in die Isomeren auftrennt, und/oder, wenn gewünscht, in einer erhaltenen Verbindung der Formel VIIIa, worin die aminogruppe geschützt ist, die Carboxylgruppe in ein reaktionsfähiges funktionelles Derivat davon überführt.

In einer $\alpha$-Ketosäure der Formel IX ist A insbesondere eine der unter Formel I genannten bevorzugten Gruppen, in erster Linie 2,5-Furylen, 2,5-Thienylen oder 1,4-Phenylen. Die Aminoschutzgruppe in einer Verbindung der Formel IX ist eine der zuvor genannten, beispielsweise, eine der unter Formel I genannten, während der Reaktion stabilen und anschliessend leicht abspaltbaren Acylgruppen, insbesondere die Trifluoracetylgruppe.

Die Reaktion der $\alpha$-Ketosäure mit der Hydroxylaminverbindung $H_2N—O—R^o$ wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Trinierderalkylamin, wie Triäthylamin oder Aethyl-diisopropylamin, oder einer heterocyclischen tertiären Base, wie Pyridin, in Freiheit gesetzt werden.

In einer erhaltenen Verbindung der Formel VIIIa kann die Schutzgruppe nach üblichen, vorher genannten Methoden abgespalten werden, eine Trifluoracetylgruppe beispielsweise durch alkalische Verseifung.

In einer erhaltenen Verbindung der Formel VIIIa kann die freie Aminogruppe nach üblichen, vorher genannten Methoden in eine der genannten geschützten Aminogruppen übergeführt werden, beispielsweise durch Behandeln mit einem Säureanhydrid, wie dem Anhydrid des Kohlensäure-mono-tert.butylesters in Gegenwart von Base, in die entsprechende N-Acyl-, z.B. N-tert.-Butoxycarbonylverbindung.

Verbindungen der Formel VIIIa und ihre geschützten Derivate, fallen bei ihrer Bildung aus der $\alpha$-Ketosäure IX und der Hydroxylaminverbindung $H_2N—O—R^o$ gewöhnlich als Gemisch von syn- und anti-Isomeren an, wobei das syn-Isomere üblichwerweise in grösserer Menge gebildet wird. Die beiden Isomeren können in die syn- und anti-Isomeren getrennt werden, beispielsweise durch Kristallisation, Chromatographie, Destillation und dergleichen.

Bezüglich der Carboxylgruppe reaktionsfähige, funktionelle Derivate von Verbindungen der Formel VIIIa, worin die Aminogruppe geschützt ist, sind die gleichen, wie die unter den Säuren der Formel III genannten, und sind insbesondere Anhydride, wie gemischte Anhydride mit den genannten anorganischen oder organischen Säuren, oder aktivierte Ester, wie diejenigen mit den genannten Alkoholen.

Die Ueberführung einer erhaltenen Verbindung der Formel VIIIa, worin die Aminogruppe geschützt ist, in ein entsprechendes reaktionsfähiges funktionelles Derivat bezüglich der Carboxylgruppe erfolgt auf an sich bekannte Weise, gegebenenfalls in situ, beispielsweise wie bei der Herstellung von reaktionsfähigen funktionellen Derivaten von Säuren der Formel III angegeben oder gemäss den bekannten Herstellungsmethoden für die reaktionsfähigen funktionellen Derivate der Formel III.

Säurehalogenide werden z.B. hergestellt, indem man eine Verbindung der Formel VIIIa, mit geschützter Aminogruppe, oder ein Salz davon mit einem Halogenierungsmittel, beispielsweise Phosphorpentachlorid, Thionylchlorid oder Oxalylchlorid, umsetzt. Die Umsetzung wird bevorzugt in einem nicht-wässrigen Lösungsmittel oder Lösungsmittelgemisch, wie einem Carbonsäureamid, z.B. Dimethylformamid, und/oder in Gegenwart einer Base, wie einem tertiären Amin, wie Triniederalkylamin, z.B. Triäthylamin, oder einem tertiären cyclischen Amin, wie N-Methyl-morpholin, durchgeführt.

Symmetrische Anhydride oder von Halogeniden verschiedene gemischte Anhydride von

Verbindungen der Formel VIIIa mit geschützter Aminogruppe können z.B. hergestellt werden, indem man eine entsprechende Verbindung mit einer freien Carboxylgruppe, vorzugsweise ein Salz, insbesondere Alkalimetall-, z.B. Natrium-, oder Ammonium-, z.B. Triäthylammoniumsalz davon, mit einem reaktionsfähigen Derivat, wie einem Halogenid, z.B. dem Chlorid, einer der genannten Säuren, z.B. einem Niederalkylhalogenformiat, wie Isobutylchlorformiat, oder einem gegebenenfalls halogenierten Niederalkancarbonsäurechlorid, z.B. Trichloracetylchlorid, umsetzt.

Aktivierte Ester von Verbindungen der Formel VIIIa mit geschützter Aminogruppe können z.B. hergestellt werden, indem man eine entsprechende Verbindung mit freier Carboxylgruppe in Gegenwart eines Carbodiimids, z.B. eines der oben genannten Carbodiimide, wie N,N'-Dicyclohexylcarbodiimid, mit einem gegebenenfalls, z.B. durch Nitro oder Halogen, wie Chlor, substituierten Phenol, wie u.a. Nitrophenol, z.B. 4-Nitrophenol oder 2,4-Dinitrophenol, oder Polyhalogenphenol, z.B. 2,3,4,5,6-Pentachlorphenol, oder mit 1-Hydroxybenzotriazol umsetzt.

Die $\alpha$-Ketosäuren der Formel IX sind neu und ebenfalls Gegenstand der vorliegenden Erfindung. Sie können auf an sich bekannte Weise hergestellt werden, indem man in einer Verbindung der Formel

$$H_2N—(C_mH_{2m})—A—C(=O)—CH_3 \qquad (X),$$

worin m und A die unter Formel VIIIa genannten Bedeutungen haben und worin die Aminogruppe in geschützter Form vorliegt, die Methylgruppe zu einer Carboxylgruppe oxidiert, oder in einer $\alpha$-Hydroxysäure der Formel

$$H_2N—(C_mH_{2m})—A—CH(OH)—COOH \qquad (XI),$$

worin m und A die unter Formel VIIIa genannten Bedeutungen haben und worin die aminogruppe in geschützter Form vorliegt, die $\alpha$-Hydroxygruppe zu einer $\alpha$-Oxogruppe oxidiert.

In einer Ausgangsverbindung der Formel X oder XI, ist die Aminogruppe beispielsweise durch eine der genannten Acylschutzgruppen, insbesondere durch die Trifluoracetylgruppe, geschützt. Die Oxidation einer Verbindung X wird mit einem der für die Ueberführung von aromatischen Acetylverbindungen, d.h. Arylmethylketonen, in entsprechende $\alpha$-Ketosäuren, d.h. Arylglyoxylsäuren, geeigneten Oxidationsmittel auf übliche Weise durchgeführt. Geeignete Oxidationsmittel sind beispielsweise oxidierende Oxide oder sauerstoffhaltige Säuren, wie solche des Selens, Schwefels, Mangans, Chroms oder Stickstoff, oder Salze entsprechender Säuren, insbesondere Alkalimetall-, wie Kaliumsalze davon, wobei die Salze gegebenenfalls in Gegenwart von Mineralsäuren, wie Salzsäure oder Schwefelsäure, verwendet werden, Wasserstoffperoxid, oder auch Sauerstoff in Gegenwart eines Katalysators, wie Platin auf Kohle. Hervorzuhebende Oxidationsmittel sind Selendioxid oder die selenige Säure, permangansaure Salze, wie Kaliumpermanganat, dichromsaure Salze, wie Kaliumdichromat, und salpetrige Säure, die in situ aus einem anorganischen Nitritsalz, wie einem entsprechenden Alkalimetallsalz oder Erdalkalimetallsalz, z.B. Natriumsalz, und einer Säure, wie Salzsäure oder Schwefelsäure, gebildet wird.

Die Oxidation wird in Wasser oder einem gegebenenfalls mit Wasser mischbaren, gegebenenfalls wasserhaltigen organischen Lösungsmittel, wie Pyridin, Essigsäure, einem Aether, wie Tetrahydrofuran oder Dioxan, oder auch einem Alkohol, wie einem Niederalkanol, z.B. Methanol oder Aethanol, bei Temperaturen von etwa 0° bis etwa 100° durchgeführt, wobei gewöhnlich, bei erhöhten Temperaturen, d.h. bei etwa 60—90°, gearbeitet wird.

Bevorzugt wird eine Verbindung der Formel X mit Selendioxid in einem Lösungsmittel, wie Pyridin, bei etwa 80—90°, oder auch gemäss der Deutschen Offenlegungsschrift Nr. 2 528 786, mit Natrium- oder Kaliumnitrit in wässriger Salz-, Schwefel- oder Phosphorsäure oxidiert.

Die Oxidation von $\alpha$-Hydroxysäuren der Formel XI, worin die Aminogruppe in geschützter Form vorliegt, zu den entsprechenden $\alpha$-Ketosäuren der Formel IX kann ebenfalls auf an sich bekannte, d.h. wie für die Oxidation von Hydroxy- zu Oxogruppen bekannte Weise durchgeführt werden. Als Oxidationsmittel kommen wiederum oxidierende Oxide, wie solche des Mangans, Chroms, Stickstoffs oder Schwefels, wie Mangandioxid, Chromtrioxid, z.B. Jones Reagens oder Chromtrioxid in Gegenwart von Essigsäure, Schwefelsäure oder Pyridin, Distickstofftetroxid, Dimethylsulfoxid gegebenenfalls in Gegenwart von Dicyclohexylcarbodiimid oder Sauerstoff, und Peroxide, wie Wasserstoffperoxid, sauerstoffhaltige Säuren, wie Permangansäure, Chromsäure oder Hypochlorsäure oder Salze davon, wie Kaliumpermanganat, Natrium- oder Kaliumdichromat oder Kaliumhypochlorit, in Frage. Die $\alpha$-Hydroxygruppe kann auch durch die Oppenauer-Oxidation in die $\alpha$-Oxogruppe übergeführt werden, d.h. durch Behandeln mit dem Salz eines sterisch gehinderten Alkohols, wie Aluminium- oder Kalium-tert.-butoxid-, -isopropoxid oder -phenoxid in Gegenwart eines Ketones, wie Aceton, Cyclohexanon oder Fluorenon. Eine weitere Möglichkeit die $\alpha$-Hydroxygruppe in die $\alpha$-Oxogruppe überzuführen besteht in der Dehydrierung, z.B. mit Raney-Nickel.

Die Oxidation wird je nach Oxidationsmittel in Wasser oder einem gegebenenfalls mit Wasser mischbaren, gegebenenfalls wasserhaltigen Lösungsmittel bei Temperaturen von etwa 0° bis etwa 100° durchgeführt.

Verbindungen der Formel XI sind bekannt oder können auf an sich bekannte Weise hergestellt werden.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eigenen.

Vorzugsweise verwendet man die pharmakologisch wirksamen Verbindungen der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wiksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung: Temperaturen werden in Celsiusgraden angegeben.

In der Dünnschichtchromatographie werden die folgenden Systeme verwendet:

| | | |
|---|---|---|
| System 52A | : | n-Butanol-Eisessig-Wasser (67:10:23) |
| System 96 | : | sec. Butanol-Eisessig-Wasser (67:10:23) |
| System 101 | : | n-Butanol-Pyridin-Eisessig-Wasser (38:24:8:30) |
| System 101A | : | n-Butanol-Pvridin-Eisessig-Wasser (42:24:4:30) |

Rf-Angaben für Dünnschichtchromatographie:
DS: Auf Silicagel-Fertigplatten SL 254 der Fa. Antec, Birsfelden.
DC: Auf Cellulose-Fertigplatten der Fa. Merck, Darmstadt.

In den Beispielen werden folgenden Abkürzungen verwendet:

BOC tert.-Butyloxycarbonyl,

Z Carbobenzoxy.

Beispiel 1

a) Ein Gemisch von 1,60 g 7$\beta$-{2-[4-((2R)-2-BOC-Amino-2-t.butoxycarbonyläthoxycarbonylamino)phenyl]acetylamino} - 3 - [(1 - methyl - 1H - tetrazol - 5 - yl) - thiomethyl] - 3 - cephem - 4-carbonsäurediphenylmethylester, 1,0 g Anisol, 2,50 ml Methylenchlorid und 25 ml Trifluoressigsäure wird 1 Stunde bei Raumtemperatur unter Ausschluss von Luftfeuchtigkeit gerührt. Aus der anfänglich klaren Lösung fällt ein voluminöser Niederschlaa aus. Am Ende der Reaktionszeit wird die Suspension auf ein eiskaltes Gemisch von Petroläther (300 ml) und Diäthyläther (150 ml) gegossen, das anfallende Trifluoracetat abgenutscht,mit Diäthyläther gewaschen und im Hochvakuum bei Raumptemperatur getrocknet.

Das rohe 7$\beta$-{2-[4-((2R)-2-Amino-2-carboxyäthoxycarbonylamino)phenyl]acetylamino}-3-[(1-methyl - 1H - tetrazol - 5 - yl) - thiomethyl] - 3 - cephem - 4 - carbonsäure - trifluoracetat wird in 25 ml Wasser aufgeschlämmt, durch Zusatz von 1N Natriumbicarbonatlösung ein pH-Wert von 6,0 eingestellt, die Suspension 15 Min. bei Raumtemperatur gerührt, dann das ungelöste Produkt (wenig) durch Behandlung mit Aktivkohle und Filtrieren durch Celite abgetrennt. Das klare Filtrat wird mit viel Aethanol versetzt, der pH-Wert auf 5,5 korrigiert und die Lösung am Rotationsverdampfer (Hockvakuum) bei 45° eingeent. Die wässrige Lösung wird noch zweimal mit Aethanol eingedampft, schliesslich das 7$\beta$-{2-[4-((2R)-2-Amino-2-carboxyäthoxycarbonylamino)phenyl]acetylamino}-3-[(1-methyl-1H-tetrazol-5-yl)-thiomethyl]-3-cephem-4-carbonsäure-natriumsalz durch Zugabe von Aethanol gefällt, abgenutscht und sukzessive mit Aethanol, Aethanol-Diäthyläther (1:1) und Diäthyläther gewaschen. Das Produkt kann durch Lösen in Wasser und Eindampfen bzw. Fällen mit Aethanol weiter gereinigt werden. F. 185—190° (Zersetzung); $[\alpha]_D = +26° \pm 1°$ (c = 3.409; in Wasser); DS: $Rf_{52A} = 0,05$; $Rf_{101} = 0,32$; $Rf_{101A} = 0,45$.

Das Ausgangsmaterial kann wie folgt erhalten werden:

b) (2R)-N-BOC-Serin-tert.-butylester wird aus (2R)-N-BOC-Serin (Smp. 89—90°, $\alpha_D = -2 \pm 1°$ (c = 3; in Methanol)) durch Umsetzung mit O-tert.-Butyl-isoharnstoff in Methylenchlorid [(E. Vowinkel, Chem. Ber. *100,* 16 (1967)] gewonnen. Smp. 75—76°. $[\alpha]_D = -8 \pm 1°$ (c = 5,2 in Chloroform).

c) Eine Lösung von 13,0 g (2R)-N-BOC-Serin-tert.-butylester in 125 ml Methylenchlorid wird auf 0° abgekühlt, mit 28 ml Phosgenlösung (20% in Toluol) versetzt und anschliessend unter Rühren und Kühlen bei +5° bis +10° ein Gemisch von 6 ml Pyridin und 45 ml Methylenchlorid innert 30 Minuten zugetropft. Die Suspension wird 90 Minuten im Eisbad gerührt und darauf eine Lösung von 12,0 g 4-Aminophenylessigsäure-benzylester in 65 ml Methylenchlorid und 6 ml Pyridin bei 0° bis +5° innert 20 Minuten dazu getropft und das Reaktionsgemisch 3 Stunden bei Raumtemperatur gerührt. Die Suspension wird mit Methylenchlorid verdünnt, sukzessive mit Wasser, 10%-iger Zitronensäure, Wasser, 1N Natriumbicarbonatlösung und Wasser gewaschen. Die organische Phase wird über Natriumsaulfat getrocknet und das Methylenchlorid am Rotationsverdampfer bei 50° abgedampft. Der erhaltene 4-((2R)-2-BOC-Amino-2-t.butoxycarbonyläthoxycarbonylamino)phenylessigsäure-benzylester an Kieselgel gereinigt und aus Petroläther-Aethylacetat (20:1) umkristallisiert. F. 74—76°; $[\alpha]_D = -22 \pm 1°$ (c = 2,85, in Chloroform).

d) Eine Lösung von 25,0 g 4-((2R)-2-BOC-Amino-2-t.butoxycarbonyläthoxycarbonylamino)-phenylessigsäurebenzylester in 300 ml Aethylacetat wird in Gegenwart von 3,0 g Palladium auf Aktivkohle (10%) hydriert. Der Katalysator wird abfiltriert, mit Aethylacetat, gewaschen, das Filtrat am Rotationsverdampfer bei 50° eingeengt und die 4-((2R)-2-BOC-Amino-2-t.butoxycarbonyläthoxycarbonylamino)phenylessigsäure durch Zusatz von Petroläther kristallisiert. F. 133—137°; $\alpha_D = -36 \pm 1°$ (c = 1,06, in Chloroform).

e) Eine Lösung von 5,0 g 7β-Amino-3-[(1-methyl-1H-tetrazol-5-yl)-thiomethyl]-3-cephem-4-carbonsäure-diphenylmethylester und 4,50 g 4-((2R)-2-BOC-Amino-2-t.butoxycarbonyläthoxycarbonylamino)phenylessigsäure in einem Gemisch von 30 ml Tetrahydrofuran und 15 ml N,N-Dimethylformamid wird mit einem Eisbad auf +5° abgekühlt, eine Lösung von 2,0 g N,N'-Dicyclohexylcarbodiimid in 10 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird die Suspension mit weiteren 2,0 g festem N,N'-Dicyclohexylcarbodiimid versetzt. Nach insgesamt 6,5 Stunden Rühren wird die Suspension abgenutscht, das Nutschgut mit Tetrahydrofuran gewaschen, das Filtrat mit viel Aethylacetat versetzt und das Tetrahydrofuran durch Einengen am Rotationsverdampfer bei 45° entfernt. Die resultierende Lösung wird mit Aethylacetat verdünnt, sukzessive mit Wasser, 1N Natriumbicarbonatlösung und Wasser gewaschen, die organische Phase wird abgetrennt, über Natriumsulfat getrocknet und am Rotationsverdampfer bei 45° eingedampft. Der zurückbleibende Schaum wird durch Chromatographieren an der 20-fachen Menge Kieselgel gereinigt. Die Fraktionen mit Methylenchlorid-Methylacetat (7:1) und (5:1) als Eluiermittel werden vereinigt und der 7β-{2-[4-((2R)-2-BOC-Amino-2-t.butoxycarbonyläthoxycarbonylamino) phenyl]acetylamino} - 3 - [(1 - methyl - 1H - tetrazol - 5 - yl) - thiomethyl] - 3 - cephem - 4 - carbonsäure-diphenylmethylester aus wenig Aethylacetat kristallisiert. F. 155—160°. DS (Fliessmittel: Toluol-Chloroform-Aethylacetat-Aethanol-(16:16:16:1): Rf = 0,51.

Beispiel 2

a) Ein Gemisch von 7,20 g 7β - {2 - [4 - ((2S) - 2 - BOC - Amino - 2 - t.butoxycarbonyläthoxycarbonylamino)phenyl]acetylamino} - 3 - [(1 - methyl - 1H - tetrazol - 5 - yl) - thiomethyl] - 3-cephem-4-carbonsäure-diphenylmethylester, 3.0 ml Anisol, 7,0 ml Methylenchlorid und 70 ml Trifluoressigsäure wird 45 Minuten bei Raumtemperatur unter Ausschluss von Luftfeuchtigkeit gerührt. Aus der anfänglich klaren Lösung fällt ein voluminöser Niederschlag aus. Am Ende der Reaktionszeit wird die Suspension auf ein eiskaltes Gemisch von Petroläther (1000 ml) und Diäthyläther (500 ml) gegossen, das anfallende Trifluoracetat abgenutscht, mit Diäthyläther gewaschen und im Hockvakuum bei Raumtemperatur getrocknet.

Das rohe 7β-{2-[4-((2S-2-Amino-2-carboxyäthoxycarbonylamino)phenyl]acetylamino}-3-[(1-methyl-1H-tetrazol-5-yl)-thiomethyl]-3-cephem-4-carbonsäure-trifluoracetate wird in 40 ml Wasser aufgeschlämmt, durch Zusatz von 1N Natriumbicarbonatlösung ein pH-Wert von 6,0 eingestellt, die Suspension 15 Min. bei Raumtemperatur gerührt, dann das ungelöste Produkt (wenig) durch Behandlung mit Aktivkohle und Filtrieren durch Celit abgetrennt. Das klare Filtrat wird mit viel Aethanol versetzt, der pH-Wert auf 5,5 korrigiert und die Lösung am Rotationsverdampfer (Hochvakuum) bei 45° eingeengt. Die wässrige Lösung wird noch zweimal mit Aethanol eingedampft, schliesslichu das 7β - {2 - [4 - ((2S) - 2 - Amino - 2 - carboxyäthoxycarbonylamino)phenyl]acetylamino} - 3 - [(1-methyl-1H-tetrazol-5-yl)-thiomethyl]-3-cephem-4-carbonsäure-natriumsalz durch Zugabe von Aethanol gefällt, abgenutscht und sukzessive mit Aethanol, Aethanol-Diäthyläther (1:1) und Diäthyläther gewaschen. Das Produkt kann durch Lösen in Wasser und Eindampfen bzw. Fällen mit Aethanol weiter gereinigt werde. F. 218—225° (Zersetzung); $[\alpha]_D = +23 \pm 1°$ (c = 3,002; in Wasser); DS: $Rf_{52A} = 0,05$; $Rf_{101} = 0,33$; $Rf_{101A} = 0,28$.

Das Ausgangsmaterial kann wie folgt erhalten werden:

b) (2S)-N-BOC-Serin-tert.-butylester wird aus (2S)-N-BOC-Serin (Smp. 85—95°, $\alpha_D = +3 \pm 1°$ (c = 3; in Methanol)) durch Umsetzung mit O-tert.-Butyl-isoharnstoff in Methylenchlorid [(E. Vowinkel,

Chem. Ber. *100,* 16 (1967)] gewonnen. Smp. 95—103°. $[\alpha]_D = +7 \pm 1°$ (c = 4,96 in Chloroform).

c) Eine Lösung von 12,1 g (2S)-N-BOC-Serin-tert.-butylester in 125 ml Methylenchlorid wird auf 0° abgekühlt, mit 30 ml Phosgenlösung (20% in Toluol) versetzt und anschliessend unter Rühren und Kühlen bei +5° bis +10° ein Gemisch von 5 ml Pyridin und 20 ml Methylenchlorid innert 30 Minuten zugetropft. Die Suspension wird 90 Minuten im Eisbad gerührt und darauf eine Lösung von 13,0 g 4-Aminophenylessigsäure-benzylester in 50 ml Methylenchlorid und 6 ml Pyridin bei 0° bis +5° innert 20 Minuten dazu getropft und das Reaktionsgemisch 3 Stunden bei Raumtemperatur gerührt. Die Suspension wird mit Methylenchlorid verdünnt, sukzessive mit Wasser, 10%-iger Zitronensäure, Wasser, 1N Natriumbicarbonatlösung und Wasser gewaschen. Die organische Phase wird über Natriumsulfat getrocknet und das Methylenchlorid an Rotationsverdampfer bei 50° abgedampft. Der erhaltene 4-((2S)-2-BOC-Amino-2-t.butoxycarbonyläthoxycarbonylamino)phenylessigsäure-benzylester an Kieselgel gereinigt und aus Petroläther-Aethylacetat (20:1) umkristallisiert. F. 74—75°; $[\alpha]_D = +21 \pm 1°$ (c = 3,02, in Chloroform).

d) Eine Lösung von 13,0 g 4 - ((2S) - 2 - BOC - Amino - 2 - t.butoxycarbonyläthoxycarbonylamino)phenylessigsäure - benzyl - ester in 200 ml Aethylacetat wird in Gegenwart von 1,50 g Palladium auf Aktivkohle (10%) hydriert. Der Katalysator wird abfiltriert, mit Aethylacetat gewaschen, das Filtrat am Rotationsverdampfer bei 50° eingeengt und die 4 - ((2S) - 2 - BOC - Amino - 2 - t.butoxycarbonyläthoxycarbonylamino)phenylessigsäure durch Zusatz von Petroläther kristallisiert. F. 150—156°; $\alpha_D = +37 \pm 1°$ (c = 2,96, in Chloroform).

e) Eine Lösung von 7,50 g 7β - Amino - 3 - [(1 - methyl - 1H - tetrazol - 5 - yl) - thiomethyl] - 3 - cephem - 4 - carbonsäure - diphenylmethylester und 7,00 g 4 - ((2S) - 2 - BOC - Amino - 2 - t.butoxycarbonyläthoxycarbonylamino)phenylessigsäure in einem Gemisch von 50 ml Tetrahydrofuran und 15 ml N,N-Dimethylformamid wird mit einem Eisbad auf +5° abgekühlt, eine Lösung von 3,0 g N,N'-Dicyclohexylcarbodiimid in 20 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird die Suspension mit weiteren 2,0 g festem N,N'-Dicyclohexylcarbodiimid versetzt. Nach insgesamt 6,5 Stunden Rühren wird die Suspension abgenutscht, das Nutschgut mit Tetrahydrofuran gewaschen, das Filtrat mit viel Aethylacetat versetzt und das Tetrahydrofuran durch Einengen am Rotationsverdampfer bei 45° entfernt. Die resultierende Lösung wird mit Aethylacetat verdünnt, sukzessive mit Wasser, 1N Natriumbicarbonatlösung und Wasser gewaschen, die organische Phase wird abgetrennt, über Natriumsulfat getrocknet und am Rotationsverdampfer bei 45° eingedampft. Der zurückbleibende Schaum wird durch Chromatographieren an der 20-fachen Menge Kieselgel fereinigt. Die Fraktionen mit Methylenchlorid-Methylacetat (7:1) und (5:1) als Eluiermittel werden vereinigt und der 7β - {2 - [4 - ((2S) - 2 - BOC - Amino - 2 - t.butoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester aus wenig Aethylacetat kristallisiert. F. 125—132°. DS (Fliessmittel: Toluol - Chloroform - Aethylacetat - Aethanol - (16:16:16:1)): Rf = 0,50.

## Beispiel 3

a) Ein Gemisch von 4,0 g 7β - {2 - [4 - ((2R) - 2 - BOC - Amino - 2 - t.butoxycarbonyläthoxycarbonylamino)phenyl]acetylamino} - 3 - methyl - 3 - cephem - 4 - carbonsäure - diphenylmethylester, 3,2 ml Anisol, 4,0 ml Methylenchlorid und 40 ml Trifluoressigsäure wird 1,5 Stunden bei Raumtemperatur unter Ausschluss von Luftfeuchtigkeit gerührt. Aus der anfänglich klaren Lösung fällt ein voluminöser Niederschlag aus. Die Suspension wird auf ein eiskaltes Gemisch von Petroläther (500 ml) und Aether (250 ml) gegossen, das anfallende Trifluoracetat abgenutscht, mit Aether gewaschen und im Hochvakuum bei Raumtemperatur getrocknet. Das 7β - {2 - [4 - ((2R) - 2 - Amino - 2 - carboxyäthoxycarbonylamino)phenyl]acetylamino} - 3 - methyl - 3 - cephem - 4 - carbonsäure - natriumsalz wird aus dem Trifluoracetat nach dem Verfahren von Beispiel 1 gewonnen. Das Natriumsalz wird durch Umfällen aus wässriger Lösung mit Aethanol gereinigt. F. 211—216° (Zersetzung); DS: $Rf_{52A} = 0{,}17$; $Rf_{101} = 0{,}34$; $Rf_{101A} = 0{,}25$.

Das Ausgangsmaterial kann wie folgt hergestellt werden:

b) Eine Lösung von 5,0 g 7β - Amino - 3 - methyl - 3 - cephem - 4 - carbonsäure - diphenylmethylester und 5,75 g 4 - ((2R) - 2 - BOC - Amino - 2 - t.butoxycarbonyläthoxycarbonylamino)-phenylessigsäure in 50 ml Tetrahydrofuran wird mit einer Lösung von 2,7 N,N'-Dicyclohexylcarbodiimid in 10 ml Tetrahydrofuran bzw. mit 1,5 g festem N,N'-Dicyclohexylcarbondiimid nach dem Verfahren von Beispiel 1 umgesetzt. Das Rohprodukt wird durch Chromatographieren an der 15-fachen Menge Kieselgel gereinigt. Die Fraktionen mit Methylenchlorid-Methylacetat (9:1) als Eluiermittel werden vereinigt und der 7β - {2 - [4 - ((2R) - 2 - BOC - Amino - 2 - t.butoxycarbonyläthoxycarbonylamino)phenyl]acetylamino} - 3 - methyl - 3 - cephem - 4 - carbonsäure - diphenylmethylester aus Aethylacetatlösung mit einem Gemisch von Petroläther und Aether gefällt. DS (Fliessmittel: Toluol - Chloroform - Aethylacetat - Aethanol-(33:33:33:1)): Rf = 0,32.

Beispiel 4

a) Ein Gemisch von 2,3 g 7β - {2 - [4 - ((2R) - 2 - BOC - Amino - 2 - t.butoxycarbonyläthoxycarbonylamino)phenyl]acetylamino} - cephalosporansäure - diphenylmethylester, 1,5 g Anisol, 15 ml Methylenchlorid und 120 ml Trifluoressigsäure wird 1 Stunde bei Raumtemperatur unter Ausschluss von Luftfeuchtigkeit gerührt. Die Suspension wird am Rotationsverdampfer bei 40° innert 15 Minuten auf ca. 50 ml eingeengt und auf ein eiskaltes Gemisch von Petroläther (500 ml) und Aether (250 ml) gegossen. Das anfallende Trifluoracetat wird abgenutscht, mit Aether gewaschen und bei Raumtemperatur im Hochvakuum getrocknet.

Das 7β - {2 - [4 - ((2R) - 2 - Amino - 2 - carboxyäthoxycarbonylamino)phenyl]acetylamino} - cephalosporansäure - natriumsalz wird aus dem Trifluoracetat nach dem Verfahren von Beispiel 1 erhalten. F. 166—170° (Zersetzung). DS: $Rf_{52A} = 0,07$; $Rf_{101} = 0,34$; $Rf_{101} = 0,46$.

Das Ausgangsmaterial kann wie folgt hergestellt werden:

b) Eine Lösung von 5,0 g 7β - Amino - cephalosporansäure - diphenylmethylester und 5,0 g 4 - ((2R) - 2 - BOC - Amino - 2 - t.butoxycarbonyläthoxycarbonylamino)phenylessigsäure in 50 ml Tetrahydrofuran wird mit einer Lösung von 2,35 g N,N'-Dicyclohexylcarbodiimid in 10 ml Tetrahydrofuran bzw. mit 1,18 g festem N,N'-Dicyclohexylcarbodiimid nach dem Verfahren von Beispiel 1 umgesetzt. Das Rohprodukt wird durch Chromatographieren an der 20-fachen Menge Kieselgel gereinigt. Die Fraktionen mit Methylenchlorid-Methylacetat (9:1) und (5:1) als Eluiermittel werden vereinigt und der 7β - {2 - [4 - ((2R) - 2 - BOC - Amino - 2 - t.butoxycarbonyläthoxycarbonylamino)phenyl]-acetylamino} - cephalosporansäurebenzylester aus Aethylacetatlösung mit einem Gemisch von Petroläther und Aether gefällt. DS (Fliessmittel: Toluol - Chloroform - Aethylacetat - Aethanol - (33:33:33:1)): Rf = 0,28.

Beispiel 5

a) Ein gemisch von 3,7 g 7β - {2 - [4 - ((2R) - 2 - BOC - Amino - 2 - t.butoxycarbonyläthoxycarbonylamino)phenyl]acetylamino} - 3 - carbamoyloxymethyl - 3 - cephem - 4 - carbonsäure - diphenylmethylester, 3,0 ml Anisol, 4,0 ml Methylenchlorid und 40 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (800 ml) und Aether (400 ml) gegossen, das anfallende Trifluoracetat abgenutscht, mit Diäthyläther gewaschen und bei Raumtemperatur im Hochvakuum getrocknet.

Das 7β - {2 - [4 - ((2R) - 2 - Amino - 2 - carboxyäthoxycarbonylamino)phenyl]acetylamino} - 3 - carbamoyloxymethyl - 3 - cephem - 4 - carbonsäure - natriumsalz wird aus dem obigen Trifluoracetat nach dem Verfahren von Beispiel 1 gewonnen. Smp. 237—242° (Zersetzung). DS: $Rf_{52A} = 0,07$; $Rf_{101} = 0,30$.

Das Ausgangsmaterial kann wie folgt hergestellt werden:

b) Eine Lösung von 5,0 g 7β - Amino - 3 - carbamoyloxy - 3 - cephem - 4 - carbonsäure - diphenylmethylester und 4,9 g 4 - ((2R) - 2 - BOC - Amino - 2 - t.butoxycarbonyläthoxycarbonylamino)phenyl - essigsäure in 50 ml Tetrahydrofuran wird mit einer Lösung von 2,8 g N,N'-Dicyclohexylcarbodiimid in 10 ml Tetrahydrofuran bzw. mit 1,5 g festem N,N'-dicyclohexylcarbodiimid nach dem Verfahren von Beispiel 1 umgesetzt. Das Rohprodukt wird durch Chromatographieren an der 30-fachen Menge Kieselgel gereinigt Die Fraktionen mit Methylenchlorid-Methylacetat (7:3) und (3:2) als Eluiermittel werden vereinigt und der 7β - {2 - [4 - ((2R) - 2 - BOC - Amino - 2 - t.butoxycarbonyläthoxycarbonylamino)phenyl]acetylamino} - 3 - carbamoyloxymethyl - 3 - cephem - 4 - carbonsäure - diphenylmethylester aus Aethylacetatlösung mit einem Gemisch von Petroläther und Aether gefällt. F. 95—114° (langsame Zersetzung); DS (Fliessmittel: Aethylacetat - Toluol - Aethanol - (47:47:5)): Rf = 0,34.

Die gemäss Beispiel 5a) erhaltene Verbindung kann auch wie folgt hergestellt werden:

ai) Eine Suspension von 24 g 7β - {2 - [4 - ((2R) - 2 - BOC - Amino - 2 - diphenylmethoxycarbonyläthoxycarbonylamino)phenyl]acetylamino} - 3 - carbamoyloxymethyl - 3 - cephem - 4 - carbonsäure - diphenylmethylester in 130 ml Methylenchlorid und 6,6 ml Anisol wird bei 0° mit 130 ml Trifluoressigsäure versetzt und die resultierende klare Lösung 45 Minuten bei 0° gerührt. Durch Zugabe von 740 ml Diäthyläther-Hexan 1:1 wird das Trifluoracetat der 7β - {2 - [4 - ((2R) - 2 - Amino - 2 - carboxyäthoxycarbonylamino)phenyl]acetylamino} - 3 - carbamoyloxymethyl - 3 - cephem - 4 - carbonsäure ausgefällt. Der Niederschlag wird abfiltriert, in 140 ml Wasser gelöst und die Lösung durch Zugabe von 1N Natriumbicarbonat auf pH 5,5 gestellt und filtriert. Das im Vakuum eingedampfte klare Filtrat gibt einen Rückstand, der mit Aethanol zu einem nahezu farblosen Pulver digeriert wird, das abgenutscht und getrocknet wird. Man erhält das Natriumsalz der 7β - {2 - [4 - ((2R) - 2 - Amino - 2 - carboxyäthoxycarbonylamino)phenyl]acetylamino} - 3 - carbamoyloxymethyl - 3 - cephem - 4 - carbonsäure: $[\alpha]_D^{20} = +110° \pm 1°$ (c = 3, $H_2O$); $[\alpha]_D^{20} = +90° \pm 1°$ (c = 3, 0,1 N Natriumbicarbonat). DC: $Rf_{101} = 0,38$.

Das Ausgangsmaterial kann wie folgt erhalten werden.

c) Eine Suspension von 100 g (2R)-Serin in 1 l Wasser wird durch langsame Zugabe von 100 g festem, wasserfreiem Natriumcarbonat in Lösung gebracht, dann versetzt man mit 2 l Dioxan sowie 346 g Di-tert.-butyl-pyrocarbonat und rührt 30 Minuten bei +20°. Das Dioxan wird im Vakuum abgedampft und die wässrige Phase bei 0° und pH 2,0 mit Essigester extrahiert. Man erhält 236,7 g amorphes, rohes (2R)—N—BOC-Serin, das ca. 195 g reines Produkt enthält. Beim Verreiben mit Petroläther und Kühlen kann daraus die reine Verbindung erhalten werden; F. 78—83° (Zersetzung).

d) Zu einer Lösung von 236,7 g des erhaltenen rohen (2R)—BOC-Serins in 700 ml Methylenchlorid tropft man bei 22° unter Kühlen eine Lösung von ca. 214 g Diphenyldiazomethan in 1 Liter Methylenchlorid und rührt $1\frac{1}{2}$ Stunden bei 20°. Das Gemisch wird im Vakuum eingedampft, der Rückstand in Aethylacetat aufgenommen und bei 0° mit Phosphat-Puffer von pH 2,0 dann von pH 7,0 gewaschen. Die getrocknete, organische Phase gibt beim Eindampfen einen kristallinen Rückstand, der in Hexan digeriert wird und man erhalt nach Filtrieren den (2R)—N—BOC-serin-diphenylmethylester; F. 116—117° (korr.). $[\alpha]_D^{20} = -6° \pm 1°$ (c = 1, Chloroform).

e) Eine Suspension von 46,4 g (2R)—N—BOC-serin-diphenylmethylester in 340 ml absolutem Methylenchlorid wird bei 0° zu einer Mischung von 62 ml 20%-igem Phosgen in Toluol und 10 ml Pyridin getropft und 1 Stunde bei 0° gerührt (Lösung A).

Eine Suspension von 18,9 g p-Aminophenylessigsäure in 75 ml Pyridin wird mit 37,5 ml Trimethylchlorsilan versetzt, wobei unter Selbsterwärmung auf 33° eine klare Lösung entsteht. Nach 15 Minuten Rühren verdünnt man mit 70 ml Methylenchlorid und rührt weitere 15 Minuten (Lösung B).

Bei 0° wird die Lösung B innerhalb 3 Minuten zur Lösung A getropft und anschliessend 30 Minuten bei 0° sowie 30 Minuten bei +20° gerührt. Das Gemisch wird auf 1,3 Liter 0,3-m. Phosphatpuffer pH 7 gerührt und bei pH 6 mit Essigester extrahiert. Die getrocknete und eingedampfte organische Phase wird mit Toluol digeriert und ergibt nach Filtration die 4 - ((2R) - 2 - BOC - Amino - 2 - diphenylmethoxycarbonyläthoxycarbonylamino)phenylessigsäure; F. 165—166° (korr.), $[\alpha]_D^{20} = +2° \pm 1°$ (c = 1, Chloroform).

f) Eine Suspension von 38,4 g 4 - ((2R) - 2 - BOC - Amino - 2 - diphenylmethoxycarbonyläthoxycarbonylamino)phenylessigsäure und 7,1 g N—Methylmorpholin in 500 ml absolutem Methylenchlorid wird bei 0° mit 8,8 g Chlorameisensäureisobutylester versetzt und anschliessend 90 Minuten bei 0° gerührt. Diese Lösung des Anhydrids tropft man bei 0° zu einer Lösung von 35,9 g 3 - Carbamoyloxymethyl - 7 - ammonium - 3 - cephem - 4 - carbonsäure - diphenylmethylester - tosylat und 7,1 N—Methylmorpholin in 300 ml Methylenchlorid und rührt 45 Minuten bei 22°. Der Ansatz wird zu 600 ml 0,5 m. Dikaliumhydrogen-phosphat-Puffer gerührt. Nach Abdestillieren des Methylenchlorids im Vakuum extrahiert man in der Kälte mit Aethylacetat. Die organische Phase wird mit Phosphat-Puffer von pH 2,0 gewaschen, getrocknet, im Vakuum, eingeengt und ergibt den 7β - {2 - [4 - ((2R) - 2 - BOC - Amino - 2 - diphenylmethoxycarbonyläthoxycarbonylamino)phenyl]-acetylamino} - 3 - carbamoyloxymethyl - 3 - cephem - 4 - carbonsäure - diphenylmethylester; F. 167—169°; $[\alpha]_D^{20} = +46° \pm 1°$ (c = 3, Dimethylsulfoxyd).

Beispiel 6

a) Ein Gemisch von 2,2 g 7β - {2 - [4 - ((5R,S) - 5 - BOC - Amino - 5 - t.butoxycarbonylphenylaminocarbonylamino)phenyl]acetylamino} - cephalosporansäure - diphenylmethylester, 1,9 g Anisol, 15 ml Methylenchlorid und 120 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (600 ml) und Aether (300 ml) gegossen, das anfallende Trifluoracetat abgenutscht, mit Aether gewaschen und bei Raumtemperatur im Hochvakuum getrocknet.

Das 7β - {2 - [4 - ((5R,S) - 5 - Amino - 5 - carboxypentylaminocarbonylamino)phenyl]-acetylamino} - cephalosporansäure - natriumsalz wird aus dem obigen Trifluoracetat nach dem Verfahren von Beispiel 1 erhalten. F. 248—255° (Zersetzung); DS: $Rf_{52A} = 0{,}05$; $Rf_{101} = 0{,}32$; $Rf_{101A} = 0{,}47$.

Das Ausgangsmaterial kann wie folgt hergestellt werden:

b) Der ölige (2R,S)—$N^2$—BOC—$N^6$—Z-Lysin-tert.-butylester wird aus (2R,S)—$N^2$—BOC—$N^6$—Z-Lysin durch Umsetzung mit O-tert.-Butyl-isoharnstoff in Methylenchlorid (E. Vowinkel, Chem. Ber. *100,* 16 (1967)) gewonnen. Das Rohprodukt wird durch Chromatographieren an der 20-fachen Kieselgel gereinigt und die Fraktionen mit Toluol-Aethylacetat (5:1) als Eluiermittel vereinigt. DS (Fliessmittel: Toluol-Aethylacetat (2:1)). Rf = 0,56.

c) Eine Lösung von 30,0 g (2R,S)—$N^2$—BOC—$N^6$—Z-Lysin-tert.-butylester in 300 ml Methanol wird in Gegenwart von 4,0 g Palladium auf Aktivkohle (10%) hydriert. Nach ca. 4 Stunden kommt die Wasserstoffaufnahme zum Stillstand. Der Katalysator wird abfiltriert, mit Methanol gewaschen und das Filtrat am Rotationsverdampfer bei 50° eingedampft. Der (2R,S)-$N^2$—BOC-Lysin-tert.-butylester ist ölig.

d) Ein Gemisch von 66 ml Phosgenlösung (20% in Toluol) und 450 ml Methylenchlorid wird mit einem Eisbad auf 0° abgekühlt und unter Rühren und Kühlen bei +5 bis +8° eine Lösung von 36,0 g (2R,S)—$N^2$—BOC-Kysin-tert.-butylester in 180 ml Methylenchlorid und 10 ml Pyridin innert 1 Stunde zugetropft. Die Suspension wird 1 Stunde im Eisbad gerührt und darauf eine Lösung von 26,1 g 4-Aminophenylessigsäure-benzylester in 280 ml Methylenchlorid und 22 ml Pyridin bei 0° bis+5° dazu getropft. Das Reaktionsgemisch wird 2 Stunden bei Raumtemperatur gerührt. Die Suspension wird mit Methylenchlorid verdünnt und sukzessive mit Wasser, 10%-iger Zitronensäure, Wasser, 1N Natriumbicarbonatlösung und Wasser gewaschen. Die organische Phase wird über Natriumsulfat getrocknet

und das Lösungsmittel am Rotationsverdampfer bei 50° abgedampft. Das Rohprodukt wird durch Chromatographieren an der 25-fachen Menge Kieselgel gereinigt und die mit Methylenchlorid-Methylacetat (4:1) als Eluiermittel erhaltenen Fraktionen vereinigt. Der erhaltene 4 - ((5R,S) - 5 - BOC - Amino - 5 - t.butoxycarbonylpentylaminocarbonylamino)phenylessigsäure - benzylester ist ölig. DS (Fliessmittel: Toluol - Chloroform - Aethylacetat - Aethanol - (3:3:3:0,3)): Rf: 0,32.

e) Eine Lösung von 7,60 g 4 - ((5R,S) - 5 - BOC - Amino - 5 - t.butoxycarbonylpentylaminocarbonylamino)phenylessigsäure - benzylester in 200 ml Aethylacetat wird in Gegenwart von 1,0 g Palladium auf Aktivkohle (10%) hydriert. Nach ca. 2 Stunden ist die berechnete Wasserstoffmenge aufgenommen. Der Katalysator wird abfiltriert, mit Aethylacetat gewaschen, das Filtrat am Rotationsverdampfer bei 45° eingeengt und die 4 - ((5R,S) - 5 - t.butoxycarbonylpentylaminocarbonylamino)-phenylessigsäure durch Zugabe von Petroläther kristallisiert. F: 121—124°.

f) Eine Lösung von 4,40 g 7β - Amino - cephalosporansäure - diphenylmethylester und 4,80 g 4 - ((5R,S) - 5 - BOC - Amino - 5 - t.butoxycarbonylpentylaminocarbonylamino)phenylessigsäure in 40 ml Tetrahydrofuran wird mit einer Lösung von 2,0 g N,N'-Dicyclohexylcarbodiimid in 10 ml Tetrahydrofuran bzw. mit 1,0 g festem N,N'-Dicyclohexylcarbodiimid nach dem Verfahren von Beispiel 1 umgesetzt. Das Rohprodukt wird durch Chromatographieren an der 30-fachen Menge Kieselgel gereinigt. Die Fraktionen mit Methylenchlorid-Methylacetat (1:1) werden vereinigt und der 7β - {2 - [4 - ((5R,S) - 5 - BOC - Amino - 5 - t.butoxycarbonylpentylaminocarbonylamino)phenyl]acetylamino} - cephalosporansäure - diphenylmethylester aus Aethylacetatlösung mit einem Gemisch von Petroläther und Aether gefällt. F. 120—128° (Zersetzung). DS (Fliessmittel: Toluol-Aethanol-(12:1)) Rf = 0,17.

Beispiel 7

a) Ein Gemisch von 1,60 g 7β - {2 - [4 - ((3R) - 3 - BOC - Amino - 3 - t.butoxycarbonylpropionylamino)phenyl]acetylamino} - 3 - [(1 - methyl - 1H - tetrazol - 5 - yl) - thiomethyl] - 3 - cephem - 4 - carbonsäure - diphenylmethylester, 1,0 g Anisol, 2,50 ml Mthylenchlorid und 25 ml Trifluoressigsäure wird 40 Minuten bei Raumtemperatur unter Ausschluss von Luftfeuchtigkeit gerührt. Die erhaltene Suspension wird auf ein eiskaltes Gemisch von Petroläther (400 ml) und Diäthyläther (200 ml) gegossen, das anfallende Trifluoracetat abgenutscht, mit Diäthyläther gewaschen und bei Raumtemperatur im Hochvakuum getrocknet.

Das 7β - {2 - [4 - ((3R) - 3 - Amino - 3 - carboxypropionylamino) - phenyl]acetylamino} - 3 - [(1 - methyl - 1H - tetrazol - 5 - yl) - thiomethyl] - 3 - cephem - 4 - carbonsäure - natriumsalz wird aus dem Trifluoracetat nach den Verfahren von Beispiel 1 erhalten. F. 248—252° (Zersetzung). DS: $Rf_{52A}$ = 0,09, $Rf_{101}$ = 0,33, $Rf_{101A}$ = 0,28.

Das Ausgangsmaterial kann wie folgt hergestellt werden:

b) (2R)—N—BOC-Asparaginsäure - α - butylester wird durch Verseifen von (2R)—N—BOC-Asparaginsäure - (β - methylester) - α - tert. - butylester [F. 50—51° (Zersetzung); $[\alpha]_D = -17° \pm 1°$ (c = 2,93 in Chloroform)] mit 1 N Natriumhydroxid (P. M. Hardy et al., J. Chem. Soc., Perkin. Trans. 1, 605 (1972)) in Aceton erhalten. F. 96—100° (Zersetzung); $[\alpha]_D = -15° \pm 1°$ (c = 2,88 in Chloroform).

c) Eine Lösung von 10,0 g (2R)—N—BOC-Asparaginsäure - α - tert. - butylester und 8,35 g 4-Aminophenylessigsäurebenzylester in 125 ml Tetrahydrofuran wird mit einem Eisbad auf +5° abgekühlt, eine Lösung von 7,15 g N,N'-Dicyclohexylcarbodiimid in 30 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird das Reaktionsgemisch mit 4,0 g festem N,N'-Dicyclohexylcarbodiimid versetzt. Nach insgesamt 7 Stunden Rühren wird die Suspension abgenutscht, das Nutschgut mit Aethylacetat gewaschen, das Filtrat mit viel Aethylacetat versetzt und das Tetrahydrofuran durch Einengen am Rotationsverdampfer bei 45° entfernt. Die erhaltene Lösung wird mit Aethylacetat verdünnt und sukzessive mit Wasser, 1N Natriumbicarbonatlösung und Wasser gewaschen. Die organische Phase wird über Natriumsulfat getrocknet und am Rotationsverdampfer bei 45° eingedampft. Der erhaltene 4 - ((3R) - 3 - BOC - Amino - 3 - t.butoxycarbonylpropionylamino)phenylessigsäure - benzylester wird aus einem Gemisch von Benzol und Petroläther umkristallisiert. F. 130—133° (Zersetzung); $[\alpha]_D = -15° \pm 1°$ (c = 1,51 in Chloroform).

d) Eine Lösung von 12,2 g 4 - ((3R) - 3 - BOC - Amino - 3 - t.butoxycarbonylpropionylamino)phenylessigsäure - benzylester in einem Gemisch von Aethylacetat (360 ml) und Aethanol (40 ml) wird in Gegenwart von 4,0 g Palladium auf Aktivkohle (10%) bei Raumtemperatur hydriert. Nach ca. 1 Stunde ist die berechnete Wasserstoffmenge aufgenommen. Der Katalysator wird abfiltriert, mit einem Gemisch von Aethylacetat-Aethanol-(9:1) gewaschen und das Filtrat am Rotationsverdampfer bei 45° eingedampft. Die 4 - ((3R) - 3 - BOC - Amino - 3 - t.butoxycarbonylpropionylamino) - phenylessigsäure wird durch Vermischen mit Petroläther gefällt. F. 160—166° (Zersetzung); $[\alpha]_D = +2° \pm 1°$ (c = 1,92, in Methanol).

e) Eine Lösung von 5,0 g 7β - Amino - 3 - [(1 - methyl - 1H - tetrazol - 5 - yl) - thiomethyl] - 3 - cephem - 4 - carbonsäure - diphenylmethylester und 4,40 g 4 - ((3R) - 3 - BOC - Amino - 3 - t.butoxycarbonylpropionylamino)phenylessigsäure in einem Gemisch von 30 ml Tetrahydrofuran und 10 ml N,N'-Dimethylformamid wird mit einer Lösung von 2,5 g N,N'-Dicyclohexylcarbodiimid in 10 ml Tetrahydrofuran bzw. 2,0 g festem N,N'-Dicyclohexylcarbodiimid nach dem Verfahren von Beispiel 1 umgesetzt. Das Rohprodukt wird durch Chromatographieren an der 20-fachen menge Kieselgel gereinigt. Die Fraktionen mit Methylenchlorid-Methylacetat (4:1) werden vereinigt und der 7β - {2 - [4 - ((3R) - 3 - BOC - Amino - 3 - t.butoxycarbonylpropionylamino)phenyl]acetylamino} - 3 - [(1 - methyl - 1H - tetrazol - 5 - yl) - thiomethyl] - 3 - cephem - 4 - carbonsäure - diphenylmethylester aus Aethylacetat kristallisiert. F. 155—160° (Zersetzung); DS (Fliessmittel: Toluol - Aethylacetat - Chloroform - Aethanol - (16:16:16:1): Rf = 0,26.

Beispiel 8

a) Ein Gemisch von 1,80 g 7β - {2 - [4 - ((3R) - 3 - BOC - Amino - 3 - t.butoxycarbonylpropionylamino)phenyl]acetylamino} - 3 - carbamoyloxymethyl - 3 - cephem - 4 - carbonsäure - diphenylmethylester, 0,8 ml Anisol und 20 ml Trifluoressigsäure wird 1 Stunde bei Raumtemperatur unter Ausschluss von Luftfeuchtigkeit gerührt. Die suspension wird auf ein eiskaltes Gemisch von Petroläther (400 ml) und Diäthyläther (200 ml) gegossen, das anfallende Trifluoracetat abgenutscht, mit Diäthyläther gewaschen und bei Raumtemperatur im Hochvakuum getrocknet. Das 7β - {2 - [4 - ((3R) - 3 - Amino - 3 - carboxypropionylamino)phenyl]acetylamino} - 3 - carbamoyloxymethyl - 3 - cephem - 4 - carbonsäure - natriumsalz wird aus dem Trifluoracetat nach dem Verfahren von beispiel 1 erhalten. F. 250—254° (Zersetzung); DS: $Rf_{52A}$ = 0,045; $Rf_{101}$ = 0,25; $Rf_{101}$ = 0,17.

b) Eine Lösung von 5,0 g 7β - Amino - 3 - carbamoyloxy - methyl - 3 - cephem - 4 - carbonsäurediphenylmethylester und 4,90 g 4 - ((3R) - 3 - BOC - Amino - 3 - t.butoxycarbonylpropionylamino)phenylessigsäure in einem Gemisch von 40 ml Tetrahydrofuran und 40 ml Methylenchlorid wird mit einer Lösung von 3,0 g N,N'-Dicyclohexylcarbodiimid bzw. 1,50 g festem N,N'-Dicyclohexylcarbodiimid in 20 ml Tetrahydrofuran nach dem Verfahren von Beispiel 1 umgesetzt. Nach 7,5 Stunden Reaktionszeit wird die Suspension abgenutscht, das Nutschgut mit einem Gemisch von Aethylacetat-Methylacetat (1:1) gewaschen und das Filtrat am Rotationsverdampfer bei 45° eingeengt. Die Lösung wird mit viel Aethylacetat-Methylacetat (1:1) verdünnt und sukzessive mit Wasser, 1 Natriumbicarbonatlosung und Wasser gewaschen. Die organische Phase wird über Natriumsulfat getrocknet. Beim Einengen der Lösung am Rotationsverdampfer bei 45° fällt der feste 7β - {2 - [4 - ((3R) - 3 - BOC - Amino - 3 - t.butoxycarbonylpropionylamino)phenyl]acetylamino} - 3 - carbamoyloxymethyl - 3 - cephem - 4 - carbonsäure - diphenylmethylester aus. Das Produkt wird abgenutscht und mit wenig Aethylacetat gewaschen. F. 158—165° (Zersetzung): DS (Fliessmittel: Aethylacetat-Chloroform-Aethanol 12:12:1): Rf = 0,51.

Beispiel 9

a) Eine Aufschlämmung von 1.07 g (1 mMol) 7β - {2 - [5 - ((2R) - 2 - BOC - Amino - 2 - diphenylmethoxycarbonyl - äthoxycarbonylaminomethyl) - 2 - furyl] - 2 - syn - methoximinoacetylamino} - 2-3 - [(1 - methyl - 1H - tetrazol - 5 - yl) - thiomethyl] - 3 - cephem - 4 - carbonsäure - diphenylmethylester in 3 ml Anisol wird auf 0° gekühlt, mit 12 ml Trifluoressigsäure versetzt und während 20 Minuten bei 0° gerührt. Anschliessend wird zweimal unter Zusatz von Toluol im Vakuum eingedampft. Der Rückstand, enthaltend das Trifluoracetat des Endproduktes, wird in Aethylacetat/Wasser aufgenommen und die organische Phase abgetrennt. Die wässrige Phase wird mit 1N Natronlauge auf pH 6,1 eingestellt, im Vakuum auf ca. 1,5 ml eingeengt und mit 60 ml Aethanol versetzt, worauf das Natriumsalz der 7β - {2 - [5 - ((2R) - 2 - Amino - 2 - carboxyäthoxycarbonylaminomethyl)-2 - furyl] - 2 - syn - methoximinoacetylamino} - 3 - [(1 - methyl - 1H - tetrazol - 5 - yl) - thiomethyl] - 3 - cephem - 4 - carbonsäure ausfällt. DS: $Rf_{52A}$ = 0,05.

Das Ausgangsmaterial kann wie folgt hergestellt werden:

b) Zu 50 g (0.51 Mol) Furfurylamin werden unter Eiskühlung 150 ml Trifluoressigsäure getropft. Die Lösung wird auf 45° erwärmt, tropfenweise mit 150 ml Acetanhydrid versetzt und anschliessend 3 Stunden bei 50°—55° und über Nacht bei Raumtemperatur gerührt. Die Mischung wird im Vakuum eingeengt und noch zweimal unter Zusatz von Toluol im Vakuum eingedampft. Das kristallisierende 2-Acetyl-5-trifluoracetylaminomethyl-furan wird abfiltriert und mit Diäthyläther gewaschen. F. 96°; IR-Spektrum (in $CHCl_3$): Absorptionsbanden bei 2,90; 3,03; 5,78; 5,97; 6,57 μ.

c) Eine Lösung von 100 g (0,42 Mol) 2-Acetyl-5-trifluoracetylaminomethylfuran und 72 g (0,65 Mol) Selendioxid in 300 ml Pyridin wird vorsichtig unter gutem Rühren auf 85° erwärmt und bei 90° während 3 Stunden gerührt. Dann wird durch Celite filtriert, das Filtrat im Vakuum eingeengt, der Rückstand in Aethylacetat aufgenommen und mit verdünnter Schwefelsäure und gesättigter Natriumchloridlösung gewaschen. Nach Trocknen der organischen Phase über Natriumsulfat wird zur Trockene eingeengt und die erhaltene 2-(5-Trifluoracetylaminomethyl-2-furyl)-2-oxoessigsäure durch Chromatographie an Silicagel (System Methylenchlorid/Aethylacetat 9:1) gereinigt. IR-Spektrum (Dioxan): Absorptionsbanden bei 3,08; 5,67; 5,81; 5,98; 6,45 μ; DS: $Rf_{52A}$ = 0,35; F. 126—128°.

d) Eine Suspension von 27 g (102 mMol) 2-(5-Trifluoracetylaminomethyl-2-furyl)-2-oxoessigsäure in 250 ml Methanol wird mit 8,07 ml Pyridin (102 mMol) und 8,5 g O-Methylhydroxylaminhydrochlorid (102 mMol) versetzt. Die erhaltene Lösung wird $1\frac{1}{2}$ Stunden bei Raumtemperatur unter Stickstoff gerührt, anschliessend im Vakuum zur Trockene eingeengt, der Rückstand in Aethylacetat aufgenommen und die Lösung mit verdünnter Schwefelsäure und gesättigter Natriumchloridlösung gewaschen. Die organische Phase wird über Natriumsulfat getrocknet und eingeengt. Die 2 - (5 - Trifluoracetylaminomethyl - 2 - furyl) - 2 - syn-methoxyiminoessigsäure wird als teilweise kristallisierender Schaum isoliert (über 85% syn-Isomeres). Nach Umkristallisation aus Aethylacetat/Hexan erhält man das reine syn-Isomere. F. 115—117°; IR-Spektrum (Nujol): Absorptions-banden bei 3,2; 5,67; 5,82; 6,36 $\mu$; DS: Rf = 0,41 (n-Butanol:Essigsäure:Wasser = 67:10:23).

e) Eine Lösung von 31 g (102 mMol) 2 - (5 - Trifluoracetylaminomethyl - 2 - furyl) - 2 - syn - methoximinoessigsäure in 150 ml Wasser und 200 ml Dioxan wird mit 27 ml (202 mMol) 7,5 N Natronlauge bei Raumtemperatur während 1 Stunde gerührt. Die Lösung wird auf pH 6 gestellt und im Vakuum auf 30 ml eingeengt. Das kristallisierte Zwitterion der 2 - (5 - Aminomethyl - 2 - furyl) - 2 - syn - methoximinoessigsäure wird abfiltriert und mit Aceton gewaschen. F. 230° (Zersetzung) IR-Spektrum (Nujol) Absorptionsbanden bei 3,25; 6,12; 6,29; 6,52 $\mu$; DS: $Rf_{52A}$ = 0,1.

f) Eine Lösung von 560 mg (1,5 mMol) (2R)—N—BOC-Serin-diphenylmethylester (hergestellt aus (2R)—N—BOC-Serin und Diphenyldiazomethan) in 10 ml Methylenchlorid wird auf 0° gekühlt und nacheinander mit 0,12 ml (1,5 mMol) Pyridin und 0,72 ml Phosgen (20%-ig in Toluol) versetzt. Die Lösung lässt man bei 0° unter Stickstoff während 1 Stunde Rühren (Lösung I).

Eine Suspension von 250 mg (1,25 mMol) 2-(5-Aminomethyl-2-furyl)-2-syn-methoximinoessigsäure in 1 ml Acetonitril wird mit 0,93 ml Bis-trimethylsilylacetamid (3,75 mMol) versetzt und 3/4 Stunden bei Raumtemperatur unter Stickstoff gerührt (Lösung II).

Lösung II wird nun zu Lösung I pipettiert und nach Zufügen von 0,2 ml (1,4 mMol) Triäthylamin $1\frac{1}{2}$ Stunden gerührt. Nach Schütteln mit 2 N Schwefelsäure und Kochsalzlösung wird die organische Phase über Natriumsulfat getrocknet und im Vakuum eingeengt. Der ölige Rückstand wird an Silicagel (System Methylenchlorid/Aethylacetat 9:1) chromatographiert.

Die 2 - [5 - ((2R) - 2 - BOC - Amino - diphenylmethoxycarbonyläthoxycarbonylaminomethyl) - 2 - furyl] - 2 - syn - methoximinoessigsäure wird als Schaum isoliert. IR-Spektrum (in $CHCl_3$): Absorptionsbanden bei 2,94; 5,78; 5,84; 5,95; 6,15; 6,66 $\mu$. DS: $Rf_{52A}$ = 0,53.

g) Eine Lösung von 10 g (16,8 mMol) 2 - [5 - ((2) - 2 - BOC - Amino - 2 - diphenylmethoxycarbonyläthoxycarbonylaminomethyl) - 2 - furyl] - 2 - syn - methoximinoessigsäure in 50 ml Methylenchlorid wird auf —20° gekühlt, mit 1,87 ml (16,8 mMol) N-Methylmorpholin und 1,9 ml (14 mMol) Chlorameisensäureisobutylester versetzt. Nach $1\frac{1}{2}$ stündiger Reaktionsdauer bei —15° werden 6,92 g 7$\beta$ - Amino - 3 - [(1 - methyl - 1H - tetrazol - 5 - yl) - thiomethyl] - 3 - cephem - 4 - carbonsaure - diphenylmethylester zugefügt und die Lösung auf 0° gebracht Nach 3 Stunden wird die Reaktionsmischung nacheinander mit 2N Schwefelsäure, verdünnter wässriger Natriumbicarbonatlösung und konzentrierter, wässriger Natriumchloridlösung gewaschen, über Natriumsulfat getrocknet, filtriert und eingedampft. Durch Chromatographie des Rückstandes an Silicagel (System Toluol/Aethylacetat 3:1) wird der 7$\beta$ - {2 - [5 - ((2R) - 2 - BOC - Amino - 2 - diphenylmethoxycarbonyläthoxycarbonylaminomethyl) - 2 - furyl] - 2 - syn - methoximinoacetylamino} - 3 - [(1 - methyl - 1H - tetrazol - 5 - yl) - thiomethyl] - 3 - cephem - 4 - carbonsäure - diphenylmethylester rein isoliert. IR-Spektrum ($CHCl_3$): Absorptionsbaden bei 2,92; 5,61; 5,74; 5,84; 6,66 $\mu$; DS: Rf = 0,41 (Toluol/Aethylacetat 1:1).

Die gleiche Verbindung kann auch wie folgt erhalten werden:

h) Eine Lösung von 31 g (102 mMol) 2-(5-Trifluoracetylaminomethyl-2-furyl-2-syn-methoximinoessigsäure in 150 ml Wasser und 200 ml Dioxan wird mit 27 ml (202 mMol) 7,5 N Natronlauge versetzt und 1 Stunde bei Raumtemperatur gerührt. Man fügt 32,4 g (306 mMol) Natriumcarbonat und 66,7 g (306 mMol) Di-tert.butyl-pyrocarbonat zu und lässt bei Raumtemperatur während $2\frac{1}{2}$ Stunden ausrühren. Die Lösung wird eingeengt und der Rückstand in Aethylacetat/Wasser gelöst. Die wässrige Phase wird abgetrennt, mit konzentrierter Phosphorsäure auf pH 2,4 eingestellt und mit Aethylacetat extrahiert. Nach Trocknen der organischen Phase über Natriumsulfat und Einengen der Lösung im Vakuum erhält man die 2 - (5 - BOC - Aminomethyl - 2 - furyl) - 2 - syn - methoximinoessigsäure als amorphen Rückstand. IR-Spektrum (in $CHCl_3$): Absorptionsbanden bei 2,99; 3,10; 5,88; 6,71 $\mu$; DS:$Rf_{52A}$ = 0,48.

i) Eine Lösung von 5 g (16,8 mMol) 2 - (5 - BOC - Aminomethyl - 2 - furyl) - 2 - syn - methoximinoessigsäure in 50 ml Methylenchlorid wird aur —20° gekühlt, mit 1,87 ml (16,8 mMol) N-Methylmorpholin und 1,90 ml (14 mMol) Chlorameisensäureisobutylester versetzt. Nach $1\frac{1}{2}$ Stunden Reaktionsdauer bei —15° werden 6,92 g 7$\beta$ - Amino - 3 - [(1 - methyl - 1H - tetrazol - 5 - yl) - thiomethyl] - 3 - cephem - 4 - carbonsäure - diphenylmethylester zugefügt und die Lösung auf 0° gebracht. Nach 3 Stunden wird nacheinander mit 2N Schwefelsäure, verdünnter wässriger Natriumbicarbonatlösung und konzentrierter wässriger Natriumchloridlösung gewaschen. Die organische Phase wird über Natriumsulfat getrocknet, filtriert und eingedampft. Der Rückstand wird an Silicagel (System Hexan/Toluol/Aceton = 3:4:2) chromatographiert und ergibt den amorphen, reinen 7$\beta$ - {2- (5 - BOC - Aminomethyl - 2 - furyl) - 2 - syn - methoximinoacetylamino] - 3 - [(1 - methyl - 1H - tetrazol - 5 - yl) - thiomethyl] - 3 - cephem - 4 - carbonsäurediphenylmethylester. IR-Spektrum (in $CHCl_3$): Absorptionsbanden bei 2,89; 2,94; 5,58; 5,84; 6,66 $\mu$; DS: Rf = 0,11 (Hexan:Toluol:Aceton = 3:4:2); Rf = 0,1 (Toluol:Essigester = 3:1).

j) Eine Lösung von 1,7 g (2,2 mMol) 7$\beta$ - [2 - (5 - BOC - Aminomethyl - 2 - furyl) - 2 - syn - methoximinoacetylamino] - 3 - [(1 - methyl - 1H - tetrazol - 5 - yl) - thiomethyl] - 3 - cephem - 4 - carbonsäure - diphenylmethylester und 0,83 (4,4 mMol) p-Toluolsulfonsäure (Monohydrat) in 17

ml Acetonitril wird während 2½ Stunden bei Raumtemperatur gerührt, dann im Vakuum eingeengt und der Rückstand in Aethylacetat und Wasser aufgenommen. Die organische Phase wird mit Natriumbicarbonat- und Kochsalzlösung gewaschen, über Natriumsulfat getrocknet und zur Trockne eingeengt. Der erhaltene 7β - [2 - (5 - Aminomethyl - 2 - furyl) - 2 - syn - methoximinoacetylamino] - 3 - [(1 - methyl - 1H - tetrazol - 5 - yl) - thiomethyl] - 3 - cephem - 4 - carbonsäure - diphenylmethylester wird in ca.15 ml Methylenchlorid aufgenommen (Lösung I).

Eine Lösung von 0,98 g (2,64 mMol) (2R)—N—BOC-Serin-diphenylmethylester in 17 ml Methylenchlorid wird bei 0° mit 0,188 ml (2,64 mMol) Pyridin und 1,43 ml Phosgen in Toluol (20%-ig) (2,64 mMol) versetzt und während 1¾ Stunden bei 0° unter Stickstoff gerührt (Lösung II).

Lösung I wird der Lösung II zugefügt und das Gemisch während 1 Stunde bei 0° unter Stickstoff gerührt. Nach Schütteln mit 2 N Schwefelsäure, Natriumbicarbonat- und Kochsalzlösung wird die organische Phase über Natriumsulfat getrocknet, filtriert und im Vakuum eingedampft. Der Rückstand wird durch Chromatographie an Silicagel (System Toluol/Aethylacetat 3:1) gereinigt und ergibt den 7β - {2 - [5 - ((2R) - 2 - BOC - Amino - 2 - diphenylmethoxycarbonyläthoxycarbonylaminomethyl) - 2 - furyl] - 2 - syn - methoximinoacetylamino} - 3 - [(1 - methyl - 1H - tetrazol - 5 - yl) - thiomethyl] - 3 - cephem - 4 - carbonsäure - diphenylmethylester (farbloser Schaum). IR-Spektrum (in $CHCl_3$): Absorptionsbanden bei 2,92; 5,61; 5,74; 5,84; 6,66 μ; DS: Rf = 0,41 (Toluol/Aethylacetat 1:1).

Beispiel 10

a) Eine Aufschlämmung von 0,36 g (0,35 mMol) 7β - {2 - [5 - ((2R) - 2 - BOC - Amino - 2 - diphenylmethoxycarbonyläthoxycarbonylaminomethyl) - 2 - furyl] - 2 - syn - methoximinoacetylamino} - 3 - carbamoyloxymethyl - 3 - cephem - 4 - carbonsäure - diphenylmethylester in 1 ml Anisol wird auf 0° gekühlt, mit 4 ml Trifluoressigsäure versetzt und während 20 Minuten bei 0° gerührt. Anschliessend wird zweimal mit Toluol versetzt und im Vakuum eingedampft. Der Rückstand, enthaltend das Trifluoracetat des Endproduktes, wird in Aethylacetat/Wasser aufgenommen und die organische Phase abgetrennt. Die wässerige Phase wird mit 1N Natronlauge auf pH 6,1 eingestellt, im Vakuum auf ca. 1 ml eingeengt und mit Aethanol (ca. 20 ml) versetzt, worauf das Natriumsalz der 7β - {2 - [5 - ((2R) - 2 - Amino - 2 - carboxyäthoxycarbonylaminomethyl) - 2 - furyl] - 2 - syn - methoximinoacetylamino} - 3 - carbamoyloxymethyl - 3 - cephem - 4 - carbonsäure ausfällt. DS: $Rf_{52A}$ = 0,05.

Das Ausgangsmaterial kann wie folgt hergestellt werden:

b) Eine Lösung von 2,05 g (3,45 mMol) 2 - [5 - ((2R) - 2 - BOC - Amino - 2 - diphenylmethoxycarbonyläthoxycarbonylaminomethyl) - 2 - furyl] - 2 - syn - methoximinoessigsäure in 10 ml Methylenchlorid wird nacheinander bei —15° mit 0,385 ml (3,45 mMol) N-Methylmorpholin und 0,39 ml (2,88 mMol) Chlorameisensäureisobutylester versetzt und 1½ Stunden bei —15° unter Stickstoff gerührt. Nach Zugabe von 1,26 g (2,88 mMol) 7β - Amino - 3 - carbamoyloxymethyl - 3 - cephem - 4 - carbonsäure - diphenylmethylester wird die Lösung auf 0° gebracht und während 2½ Stunden bei dieser Temperatur gerührt. Die Reaktionsmischung wird mit Methylenchlorid verdünnt, nacheinander mit 2N Schwefelsäure, Natriumbicarbonat- und Natriumchloridlösung gewaschen, über Natriumsulfat getrocknet, filtriert und eingedampft. Das Rohprodukt wird an Silicagel (System Toluol/Aethylacetat 1:1) chromatographiert und ergibt den reinen 7β - {2 - [5 - ((2R) - 2 - BOC - Amino - 2 - diphenylmethoxycarbonyläthoxycarbonylaminomethyl) - 2 - furyl] - 2 - syn - methoximinoacetylamino} - 3 - carbamoyloxymethyl - 3 - cephem - 4 - carbonsäure - diphenylmethylester. IR-Spektrum (in $CHCl_3$): Absorptionsbanden bei 2,93; 5,58; 5,81; 6,66 μ; DS: Rf = 0,21 (Toluol/Aethylacetat: 1:1).

Die gleiche Verbindung kann auch wie folgt hergestellt werden:

c) Eine Lösung von 10,3 g (34,5 mMol) 2 - (5 - BOC - Aminomethyl - 2 - furyl) - 2 - syn - methoximinoessigsäure in 100 ml Methylenchlorid wird nacheinander bei —15° mit 3,85 ml 34,5 mMol) N-Methylmorpholin und 3,9 ml (28,8 mMol) Chlorameisensäureisobutylester versetzt, dann 1½ Stunden bei —15° unter Stickstoff gerührt. Nach Zugabe von 12,6 g (28,8 mMol) 7β - Amino - 3 - carbamoyloxymethyl - 3 - cephem - 4 - carbonsäure - diphenylmethylester wird die Lösung auf 0° gebracht und während 2½ Stunden bei dieser Temperatur gerührt. Die Reaktionsmischung wird mit Methylenchlorid verdünnt, nacheinander mit 2N Schwefelsäure, Natriumbicarbonat- und Natriumchloridlösung gewaschen, über Natriumsulfat getrocknet, filtriert und eingedampft. Das Rohprodukt wird an Silicagel (System Toluol/Aethylacetat 2:1) chromatographiert und ergibt den 7β - [2 - (5 - BOC - Aminomethyl - 2 - furyl) - 2 - syn - methoximinoacetylamino] - 3 - carbamoyloxymethyl - 3 - cephem - 4 - carbonsäure - diphenylmethylester, der aus Aethylacetat/Diäthyläther umkristallisiert wird. F. 145—148°; IR-Spektrum ($CHCl_3$): Absorptionsbanden bei 2,93; 3,00; 5,61; 5,84; 6,33; 6,66 μ; DS: RF = 0,1 (Toluol/Aethylacetat = 2:1).

d) Eine Lösung von 0,8 g (1,11 mMol) 7β - [2 - (5 - BOC - Aminomethyl - 2 - furyl - 2 - syn - methoximinoacetylamino] - 3 - carbamoyloxymethyl - 3 - cephem - 4 - carbonsäure - diphenylmethylester, und 0,42 g (2,22 mMol) p-Toluolsulfonsäure-monohydrat in 8 ml Acetonitril wird während 2½ Stunden bei Raumtemperatur gerührt, im Vakuum eingeengt und der Rückstand in Aethylacetat und Wasser aufgenommen. Die organische Phase wird mit Natriumbicarbonat- und Kochsalzlösung gewaschen, über Natriumsulfat getrocknet und zur Trockne eingeengt. Der erhaltene 7β - [2 - (5 - Aminomethyl - 2 - furyl) - 2 - syn - methoximinoacetylamino] - 3 - carbamoyloxymethyl - 3 - cephem - 4 - carbonsäure - diphenylmethylester wird in 5 ml Methylenchlorid aufgenommen (Lösung I).

Eine Lösung von 0,493 g (1,33 mMol) (2R)—N—BOC-Serin-diphenylmethylester in 9 ml Methylenchlorid wird bei 0° mit 0,095 ml (1,33 mMol) Pyridin und 0,752 ml Phosgen in Toluol (20%-ig) (1,33 mMol) versetzt und während 1 Stunde bei 0° gerührt (Lösung II).

Lösung I wird der Lösung II zugefügt und das Gemisch während 1 Stunde bei 0° unter Stickstoff stehen gelassen. Nach Schütteln mit 2N Schwefelsäure, Natriumbicarbonat- und Kochsalzlösung wird die organische Phase über Natriumsulfat getrocknet, filtriert und im Vakuum eingedampft. Der Rückstand wird durch Chromatographie an Silicagel (System Toluol/Aethylacetat 1.1) gereinigt und ergibt den 7β - {2 - [5 - ((2R) - 2 - BOC - Amino - 2 - diphenylmethoxycarbonyläthoxycarbonylaminomethyl) - 2 - furyl] - 2 - syn - methoximinoacetylamino} - 3 - carbamoyloxymethyl - 3 - cephem - 4 - carbonsäure - diphenylmethylester. IR-Spektrum (in $CHCl_3$): Absorptionsbanden bei 2,93; 5,58; 5,81; 6,66 μ; DS: Rf = 0,21 (Toluol/Aethylacetat = 1:1).

Beispiel 11

a) Eine Aufschlämmung von 1,06 g (1 mMol) 7β - {2 - [5 - ((2R) - 2 - BOC - Amino - 2 - diphenylmethoxycarbonyl - äthoxycarbonylaminomethyl) - 2 - thienyl] - 2 - syn - methoximinoacetylamino} - 3 - acetoxymethyl - 3 - cephem - 4 - carbonsäure - diphenylmethylester in 3 ml Anisol wird auf 0° gekühlt, mit 12 ml Trifluoressigsäure versetzt und während 20 Minuten bei 0° gerührt. Anschliessend wird zweimal unter Zusatz von Toluol im Vakuum eingedampft. Der Rückstand, enthaltend das Trifluoracetat des Endproduktes, wird in Aethylacetat/Wasser aufgenommen und die organische Phase abgetrennt. Die wässrige Phase wird mit 1N Natronlauge auf pH 6,1 eingestellt, im Vakuum auf ca. 1,5 ml eingeengt und mit 60 ml Aethanol versetzt, worauf das Natriumsalz der 7β - {2 - [5 - ((2R) - 2 - Amino - 2 - carboxyäthoxycarbonylaminomethyl) - 2 - thienyl] - 2 - syn - methoximinoacetylamino} - 3 - acetoxymethyl - 3 - cephem - 4 - carbonsäure ausfällt. DS: $Rf_{52A}$ = 0,05.

Das Ausgangsmaterial kann wie folgt hergestellt werden:

b) Eine Lösung von 100 g (0.40 Mol) 2-Acetyl-5-trifluoracetylaminomethylthiophen (herstellbar analog Beispiel 9b) aus 2-Aminomethylthiophen, Trifluoressigsäure und Acetanhydrid) und 72 g (0,65 mMol) Selendioxid in 300 ml Pyridin wird vorsichtig unter gutem Rühren auf 85° erwärmt und bei 90° während 3 Stunden gerührt. Dann wird durch Celite filtriert, das Filtrat im Vakuum eingeengt, der Rückstand in Aethylacetat aufgenommen und mit verdünnter Schwefelsäure und gesättigter Natriumchloridlösung gewaschen. Nach Trocknen der organischen Phase über Natriumsulfat wird zur Trockne eingeengt und die erhaltene 2 - (5 - Trifluoracetylaminomethyl - 2 - thienyl) - 2 - oxoessigsäure durch Chromatographie an Silicagel (System Methylenchlorid/Aethylacetat 9:1) gereinigt. IR-Spektrum (Dioxan): Absorptionsbanden bei 3,08; 5,67; 5,81; 5,90; 6,45 μ; DS: $Rf_{52A}$ = 0,35.

c) Eine Suspension von 28 g (100 mMol) 2-(5-Trifluoracetylaminomethyl-2-thienyl)-2-oxoessigsäure in 250 ml Methanol wird mit 8,07 ml Pyridin (102 mMol) und 8,5 g O-Methylhydroxylaminhydrochlorid (102 mMol) versetzt. Die erhaltene Lösung wird $1\frac{1}{2}$ Stunden bei Raumtemperatur unter Stickstoff gerührt, anschliessend im Vakuum zur Trockene eingeengt, der Rückstand in Aethylacetat aufgenommen und die Lösung mit verdünnter Schwefelsäure und gesättigter Natriumchloridlösung gewaschen. Die organische Phase wird über Natriumsulfat getrocknet und eingeengt. Die 2-(5-Trifluoracetylaminomethyl-2-thienyl)-2-syn-methoximinoessigsäure wird als Schaum isoliert (ca. 70% syn-Isomeres). IR-Spektrum (Nujol): Absorptionsbanden bei 3,2; 5,67; 5,82; 6,36 μ; DS: $Rf_{52A}$ = 0,41.

d) Eine Lösung von 31 g (100 mMol) 2-(5-Trifluoracetylaminomethyl-2-thienyl)-2-syn-methoximinoessigsäure in 150 ml Wasser und 200 ml Dioxan wird mit 27 ml (202 mMol) 7,5 N Natronlauge bei Raumtemperatur während 1 Stunde gerührt. Die Lösung wird auf pH 6 gestellt und im Vakuum auf 30 ml eingeengt. Das ausfallende Zwitterion der 2-(5-Aminomethyl-2-thienyl)-2-syn-methoximinoessigsäure wird abfiltriert und mit Aceton gewaschen. IR-Spektrum (Nujol): Absorptionsbanden bei 3,25; 6,12; 6,29; 6,52 μ; DS: $Rf_{52A}$ = 0,1.

e) Eine Lösung von 560 mg (1,5 mMol) (2R)—N—BOC-Serin-diphenylmethylester (hergestellt aus (2R)—N—BOC-Serin und Diphenyldiazomethan) in 10 ml Methylenchlorid wird auf 0° gekühlt und nacheinander mit 0,12 ml (1,5 mMol) Pyridin und 0,72 ml Phosgen (20%-ig in Toluol) versetzt. Die Lösung lässt man bei 0° unter Stickstoff während 1 Stunde Rühren (Lösung I).

Eine Suspension von 250 mg (1,25 mMol) 2-(5-Aminomethyl-2-thienyl)-2-syn-methoximinoessigsäure wird mit 0,93 ml Bis-Trimethylsilylacetamid (3,75 mMol) versetzt und 3/4 Stunden bei Raumtemperatur unter Stickstoff gerührt (Lösung II).

Lösung II wird nun zu Lösung I pipettiert und nach Zufügen von 0,2 ml (1,4 mMol) Triäthylamin $1\frac{1}{2}$ Stunden gerührt. Nach Schütteln mit 2 N Schwefelsäure und Kochsalzlösung wird die organische Phase über Natriumsulfat getrocknet und im Vakuum eingeengt. Der ölige Rückstand wird an Silicagel (System Methylenchlorid/Aethylacetat 9:1) chromatographiert.

Die 2 - [5 - ((2R) - 2 - BOC - Amino - 2 - diphenylmethoxycarbonyläthoxycarbonylaminomethyl) - 2 - thienyl] - 2 - syn - methoximinoessigsäure wird als Schaum isoliert. IR-Spektrum (in

$CHCl_3$): Absorptionsbanden bei 2,94; 5,78; 5,84; 5,95; 6,15; 6,66 $\mu$; DS: $Rf_{52A} = 0,53$.

f) Eine Lösung von 10 g (16,0 mMol) 2 - [5 - ((2R) - 2 - BOC - Amino - 2 - diphenylmethoxycarbonylaminomethyl) - 2 - thienyl] - 2 - syn - methoximinoessigsäure in 50 ml Methylenchlorid wird auf —20° gekühlt, mit 1,87 ml (16,8 mMol) N-Methylmorpholin und 1,9 ml (14 mMol) Chlorameisensäureisobutylester versetzt. Nach $1\frac{1}{2}$ stündiger Reaktionsdauer bei —15° werden 6,92 g 7$\beta$ - Amino - 3 - acetylmethyl - 3 - cephem - 4 - carbonsäure - diphenylmethylester zugefügt und die Lösung auf 0° gebracht. Nach 3 Stunden wird die Reaktionsmischung nacheinander mit 2N Schwefelsäure, verdünnter wässriger Natriumbicarbonatlösung und konzentrierter, wässriger Natriumchloridlösung gewaschen, über Natriumsulfat getrocknet, filtriert und eingedampft. Durch Chromatographie des Rückstandes an Silicagel (System Toluol/Aethylacetat 3:1) wird der 7$\beta$ - {2 - [5 - ((2R) - 2 - BOC - Amino - 2 - diphenylmethoxycarbonyläthoxycarbonylaminomethyl) - 2 - thienyl] - 2 - syn - methoximinoacetylamino} - 3 - acetoxymethyl - 3 - cephem - 4 - carbonsäurediphenyl - methylester rein isoliert. IR-Spektrum ($CHCl_3$): Absorptionsbanden bei 2,92; 5,61; 5,74; 5,84; 6,66 $\mu$; DS: Rf = 0,41 (Toluol/Aethylacetat 1:1).

Die gleiche Verbindung kann auch wie folgt erhalten werden:

g) Eine Lösung von 31 g (100 mMol) 2 - (5 - Trifluoracetylaminomethyl - 2 - thienyl) - 2 - syn - methoximinoessigsäure in 150 ml Wasser und 200 ml Dioxan wird mit 27 ml (202 mMol) 7,5 N Natronlauge versetzt und 1 Stunde bei Raumtemperatur gerührt. Man fügt 32,4 g (306 mMol) Natriumcarbonat und 66,7 g (306 mMol) Di-tert.Butyl-pyrocarbonat zu und lässt bei Raumtemperatur während $2\frac{1}{2}$ Stunden ausrühren. Die Lösung wird eingeengt und der Rückstand in Aethylacetat/Wasser gelöst. Die wässrige Phase wird abgetrennt, mit konzentrierter Phosphorsäure auf pH 2,4 eingestellt und mit Aethylacetat extrahiert. Nach Trocknen der organischen Phase über Natriumsulfat und Einengen der Lösung im Vakuum erhält man die 2 - (5 - BOC - Aminomethyl - 2 - thienyl) - 2 - syn - methoximinoessigsäure als amorphen Rückstand. IR-Spektrum (in $CHCl_3$): Absorptionsbanden bei 2,99; 3,10 5,88; 6,71 $\mu$; DS: Rf = 0,48 (n-Butanol:Essigsäure: Wasser = 67:10:23).

h) Eine Lösung von 5 g (16,0 mMol) 2 - (5 - BOC - Aminomethyl - 2 - thienyl) - 2 - syn - methoximinoessigsäure in 50 ml Methylenchlorid wird auf —20° gekühlt, mit 1,87 ml (16,8 mMol) N-Methylmorpholin und 1,90 ml (14 mMol) Chlorameisensäureisobutylester versetzt. Nach $1\frac{1}{2}$ Stunden Reaktionsdauer bei —15° werden 6,92 g 7$\beta$ - Amino - 3 - acetoxymethyl - 3 - cephem - 4 - carbonsäure - diphenylmethylester zugefügt und die Lösung auf 0° gebracht. Nach 3 Stunden wird nacheinander mit 2N Schwefelsäure, verdünnter wässriger Natriumbicarbonatlösung und konzentrierter wässriger Natriumchloridlösung gewaschen. Die organische Phase wird über Natriumsulfat getrocknet, filtriert und eingedampft. Der Rückstand wird an Silicagel (System Hexan/Toluol/Aceton = 3:4:2) chromatographiert und ergibt den amorphen, reinen 7$\beta$ - [2 - (5 - BOC - Aminomethyl - 2 - thienyl) - 2 - syn - methoximinoacetylamino] - 3 - acetoxymethyl - 3 - cephem - 4 - carbonsäurediphenylmethylester. IR-Spektrum (in $CHCl_3$): Absorptionsbanden bei 2,89; 2,94; 5,58; 5,84; 6,66 $\mu$; DS: Rf = 0,11 (Hexan:Toluol:Aceton = 3:4:2); Rf = 0,1 (Toluol: Essigester = 3:1).

i) Eine Lösung von 1,7 g (2,2 mMol) 7$\beta$ - [2 - (5 - BOC - Aminomethyl - 2 - thienyl) - 2 - syn - methoximinoacetylamino] - 3 - acetoxymethyl - 3 - cephem - 4 - carbonsäurediphenylmethylester und 0,83 g (4,4 mMol) p-Toluolsulfonsäure (Monohydrat) in 17 ml Acetonitril wird während $2\frac{1}{2}$ Stunden bei Raumtemperatur gerührt, dann im Vakuum eingeengt und der Rückstand in Aethylacetat und Waser aufgenommen. Die organische Phase wird mit Natriumbicarbonat- und Kochsalzlösung gewaschen, über Natriumsulfat getrocknet und zur Trockne eingeengt. Der erhaltene 7$\beta$ - [2 - (5 - Aminomethyl - 2 - thienyl) - 2 - syn - methoximinoacetylamino] - 3 - acetoxymethyl - 3 - cephem - 4 - carbonsäure - diphenylmethylester wird in ca. 15 ml Methylenchlorid aufgenommen (Lösung I).

Eine Lösung von 0,98 g (2,64 mMol) (2R)—N—BOC-Serin-diphenylmethylester in 17 ml Methylenchlorid wird bei 0° mit 0,188 ml (2,64 mMol) Pyridin und 1,43 ml Phosgenin Toluol (20%-ig) (2,64 mMol) versetzt und während $1\frac{3}{4}$ Stunden bei 0° unter Stickstoff gerührt (Lösung II).

Lösung I wird der Lösung II Zugefügt und das Gemisch während 1 Stunde bei 0° unter Stickstoff gerührt. Nach Schütteln mit 2 N Schwefelsäure, Natriumbicarbonat- und Kochsalzlösung wird die organische Phase über Natriumsulfat getrocknet, filtriert und im Vakuum eingedampft. Der Rückstand wird durch Chromatographie an Silicagel (System Toluol/Aethylacetat 3:1) gereinigt und ergibt den 7$\beta$ - {2 - [5 - ((2R) - 2 - BOC - Amino - 2 - diphenylmethoxycarbonyläthoxycarbonylaminomethyl) - 2 - thienyl] - 2 - syn - methoximinoacetylamino} - 3 - acetoxymethyl - 3 - cephem - 4 - carbonsäure - diphenylmethylester (farbloser Schaum). IR-Spektrum (in $CHCl_3$): Absorptionsbanden bei 2,92; 5,61; 5,74; 5,84; 6,66 $\mu$; DS: Rf = 0,41 (Toluol/Aethylacetat 1:1).

## Beispiel 12

a) Eine Suspension von 0,58 g 7$\beta$ - {2 - [4 - ((2R) - 2 - BOC - Amino - 2 - diphenylmethoxycarbonyläthoxycarbonylaminosulfonylamino)phenyl]acetylamino} - 3 - carbamoyloxymethyl - 3 - cephem - 4 - carbonsäurediphenylmethylester und 0,15 ml Anisol in 3 ml Methylenchlorid wird bei 0° mit 3 ml Trifluoressigsäure versetzt und 45 Minuten bei 0° gerührt. Durch Zugabe von 17 ml Diäthyläther-Hexan 1:1 wird das Trifluoracetat des Endproduktes gefällt, das nach 10 Minuten bei 0° abfiltriert und in 15 ml Wasser gelöst wird. Die wässrige Lösung wird dreimal mit je

20 ml Aethylacetat gewaschen, mit 1 N Natronlauge auf pH 4,6 gestellt, mit Aceton verdünnt und im Vakuum eingeengt. Das ausfallende 7β - {2 - [4 - ((2R) - 2 - Amino - 2 - carboxyäthoxy - carbonylaminosulfonylamino)phenyl]acetylamino} - 3 - carbamoyloxymethyl - 3 - cephem - 4 - carbonsäure - natriumsalz wird abfiltriert und im Vakuum getrocknet; $[\alpha]_D^{20} = +79° \pm 1°$ (c = 3; Wasser).

Das Ausgangsmaterial wird wie folgt hergestellt:

b) Zu einer Losung von 0,69 g Chlorsulfonylisocyanat in 1,3 ml Methylenchlorid wird bei 20° eine Lösung von 1,94 g (2R)—N—BOC-Serin-diphenylmethylester und 0,69 g Pyridin in 10 ml Methylenchlorid getropft. Nach 20 Minuten Rühren bei 40° wird die klare Lösung auf 0° abgekühlt (Lösung A).

Eine Suspension von 0,75 g Aminophenylessigsäure in 2,91 g Pyridin wird mit 1,29 g Trimethylchlorsilan versetzt, wobei eine klare Lösung entsteht und die Temperatur auf 38° ansteigt. Nach 20 Minuten Rühren bei 40—45° wird auf 0° gekühlt (Lösung B).

Bei 0° wird Lösung B zu Lösung A getropft und anschliessend 30 Minuten bei 0° und 1 Stunde bei +22° weitergerührt. Das Gemisch wird unter Rühren auf 80 ml 0,2-m Dikaliumhydrogenphosphatlösung gegossen, im Vakuum vom Methylenchlorid befreit und die verbleibende wässrige Phase bei pH2 in der Kälte mit Aethylacetat extrahiert. Durch Eindampfen der organischen Phase word die 2 - [4 - ((2R) - 2 - BOC - Amino - 2 - diphenylmethoxycarbonyläthoxycarbonylaminosulfonylamino)-phenyl]essigsäure in Form eines Schaumes erhalten.

c) Eine Lösung von 2,08 g 2 - [4 - ((2R) - 2 - BOC - Amino - 2 - diphenylmethoxycarbonyläthoxycarbonylaminosulfonylamino) - phenyl]essigsäure und 0,32 g N-Methylmorpholin in 28 ml Methylenchlorid wird bei 0° mit 0,43 g Chlorameisensäureisobutylester versetzt und anschliessend 1 Stunde bei 0° gerührt. Diese Lösung des entstehenden gemischten Anhydrids wird bei 0° zu einer Lösung von 1,62 g 7β - Amonio - 3 - carbamoyloxymethyl - 3 - cephem - 4 - carbonsäure - diphenylmethylester - p - tosylat und 0,34 g N-Methylmorpholin in 14 ml Methylenchlorid getropft. Nach 1½ Stunden Rühren bei 0° und 1 Stunde bei +22° wird das Gemisch auf 100 ml 0,3-m Kaliumdihydrogenphosphatlösung gerührt und im Vakuum von organischen Lösungsmitteln befreit. Die verbleibende wässrige Phase wird bei 0° und pH 2,0 mit Aethylacetat extrahiert Die organische Phase wird mit 0,5-m Dikaliumhydrogenphosphatlösung gewaschen, mit Natriumsulfat getrocknet, im Vakuum eingedampft und ergibt den 7β - {2 - [4 - ((2R) - 2 - BOC - Amino - 2 - diphenylmethoxycarbonyläthoxycarbonylaminosulfonylamino)-phenyl]acetylamino} - 3 - carbamoyloxymethyl - 3 - cephem - 4 - carbonsäurediphenylmethylester.

Beispiel 13

a) Eine Mischung von 1,00 g (1,29 mMol) 7β - [2 - (5 - BOC - Aminomethyl - 2 - furyl) - 2 - syn - methoximinoacetylamino] - 3 - [(1 - methyl - 1H - tetrazol - 5 - yl) - thiomethyl] - 3 - cephem - 4 - carbonsäure - diphenylmethylester in 2 ml Anisol wird auf 0° gekühlt, mit 8 ml Trifluoressigsäure versetzt und während 15 Minuten bei 0° gerührt. Anschliessend wird zweimal mit Toluol versetzt und im Vakuum eingedampft. Der Rückstand, enthaltend das Trifluoracetat des Endprodktes, wird in Aethylacetat/Wasser aufgenommen und die organische Phase abgetrennt, und zweimal mit Wasser ausgeschüttelt. Die vereinigten wässrigen Phasen werden dreimal mit Aethylacetat gewaschen, mit Triäthylamin (20%-ige Lösung in Aethanol) auf pH 5,7 gestellt, im Vakuum etwas eingeengt und mit Aceton versetzt, worauf die 7β - [2 - (5 - Aminomethyl - 2 - furyl) - 2 - syn - methoximinoacetylamino] - 3 - [(1 - methyl - 1H - tetrazol - 5 - yl) - thiomethyl] - 3 - cephem - 4 - carbonsäure ausfällt. DS: $Rf_{52A} = 0,096$; IR-Spektrum (Nujol): Absorptionsbanden bei 2,85 (Schulter); 3,11; 5,66; 6,00; 6,25; 6,45 μ.

Beispiel 14

a) Eine Mischung von 3 g (4,25 mMol) 7β - [2 - (5 - BOC - Aminomethyl - 2 - furyl) - 2 - syn - methoxyiminoacetylamino] - 3 - carbamoyloxymethyl - 3 - cephem - 4 - carbonsäure - diphenylmethylester in 7,5 ml Anisol wird auf 0° gekühlt, mit 30 ml Trifluoressigsäure versetzt und während 20 Minuten bei 0° gerührt. Anschliessend wird dreimal mit Toluol versetzt und im Vakuum eingedampft. Der Rückstand, enthaltend das Trifluoracetat des Endproduktes, wird in Aethylacetat/Wasser aufgenommen, die organische Phase abgetrennt und zweimal mit Wasser ausgeschüttelt. Die vereinigten wässrigen Phasen werden dreimal mit Aethylacetat gewaschen, filtriert, mit Triäthylamin (20%-ige Lösung in Aethanol) auf pH 5,7 gestellt, im Vakuum auf ca. 5 ml eingeengt und mit Aceton versetzt, worauf die 7β - [2 - (5 - Aminomethyl - 2 - furyl) - 2 - syn - methoximinoacetylamino] - 3 - carbamoyl - oxymethyl - 3 - cephem - 4 - carbonsäure ausfällt. DS: $Rf_{52A} = 0,062$; IR-Spektrum (Nujol): Absorpitonsbanden bei 2,87 (Schulter): 3,09; 5,65; 5,87; 5,98; 6,23; 6,47 μ.

Beispiel 15

a) Eine Lösung von 10,5 g 7β - {(2R,S) - 2 - [4 - ((2R) - 2 - BOC - Amino - 2 - tert. - butoxycarbonyläthoxycarbonylamino)phenyl] - 2 - (2,2,2 - trichloräthoxycarbonyloxy)acetylamino} - 3 - carbamoyloxymethyl - 3 - cephem - 4 - carbonsäure - diphenylmethylester in 100 ml Acetonitril und 100 ml Eisessig wird im Eisbad abgekühlt und unter starkem Rühren bei +3° während 2 Stunden

portionenweise mit insgesamt 20,5 g Zinkpulver in gleichen Zeitabständen versetzt. Anschliessend wird die Suspension weitere 3 Stunden im Eisbad gerührt, dann durch Celit abgenutscht und das Nutschgut mit viel Aethylacetat gewaschen. Das Filtrat wird sukzessive mit viel Wasser, 1N Natriumhydrogencarbonat und Wasser gewaschen. Die Aethylacetatlösung wird über Natriumsulfat getrocknet, das Lösungsmittel am Rotationsverdampfer bei 45° abgedampft und der Rückstand durch Chromatographieren an der 25-fachen Menge Kieselgel gereinigt. Die Fraktionen mit Methylenchlorid-Methylacetat-(7:3) als Eluiermittel werden vereinigt und der 7β - {(2R,S) - 2 - [4 - ((2R) - 2 - BOC - Amino - 2 - tert. - butoxycarbonyläthoxycarbonylamino)phenyl] - 2 - hydroxyacetylamino} - 3 - carbamoyloxymethyl - 3 - cephem - 4 - carbonsäure - diphenylmethylester aus Aethylacetatlösung mit einem Gemisch von Petroläther und Diäthyläther gefällt. DS (Fliessmittel: Chloroform-Aethylacetat-Aethanol-(5:5:0,1)): Rf = 0,20 und 0,32 (Diastereomerengemisch).

Ein Gemisch von 2,70 g 7β - {(2R,S) - 2 - [4 - ((2R) - 2 - BOC - Amino - 2 - tert. - butoxycarbonyläthoxycarbonylamino)phenyl] - 2 - hydroxyacetylamino} - 3 - carbamoyloxymethyl - 3 - cephem - 4 - carbonsäure - diphenylmethylester, 2,70 ml Methylenchlorid, 1,70 ml Anisol und 27,0 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (500 ml) und Diäthyläther (250 ml) gegossen, das anfallende Trifluoracetat abgenutscht, mit Diäthyläther gewaschen und im Hochvakuum bei Raumtemperatur gerocknet. Das erhaltene Trifluoracetat der 7β - {(2R,S) - 2 - [4 - ((2R) - 2 - Amino - 2 - carboxyäthoxycarbonylamino)phenyl] - 2 - hydroxyacetylamino} - 3 - carbamoyloxymethyl - 3 - cephem - 4 - carbonsäure wird analog Beispiel 1a) in das entsprechende Natriumsalz übergeführt. F. ab 220° (Zersetzung). DS: $Rf_{101} = 0{,}24$; $Rf_{101A} = 0{,}17$.

Das Ausgangsmaterial kann wie folgt hergestellt werden:

b) 16 g (R,S)-4-Amino-mandelsäure werden in 80 ml Wasser suspendiert, unter Rühren durch Zutropfen von 2N Natronlauge ein pH-Wert von 7,5 eingestellt und die Lösung mittels Eisbad auf +3 bis +5° abgekühlt. Darauf wird eine Lösung von 22,2 g Chlorameisensäure-2,2,2-trichloräthylester in 50 ml Tetrahydrofuran unter starkem Rühren und Kühlen innert 20 Minuten bei +5° zugetropft und durch Zutropfen von 2N Natronlauge der pH-Wert des Reaktionsgemisches zwischen 7—8 gehalten. Anschliessend wird das Gemisch 1 Stunde bei +5° gerührt, dann mit 100 ml Wasser verdünnt und zweimal mit Aethylacetat ausgezogen. Die wässrige Phase wird abgetrennt, mit Aethylacetat überschichtet, im Eisbad abgekühlt, unter Rühren durch Zugabe von verdünnter Salzsäure-(1:1) sauer gestellt (pH 2) und zweimal mit Aethylacetat ausgezogen. Die organischen Extrakte werden vereinigt, einmal mit Solelösung gewaschen, über Natriumsulfat getrocknet und das Lösungsmittel am Rotationsverdampfer bei 50° abgedampft. Die (R,S)-4-(2,2,2-Trichloräthoxycarbonylamino)-mandelsäure wird aus Aethylacetat-Petroläther umkristallisiert. F. 202—205° (Zersetzung).

c) In eine Lösung von 64 g (R,S)-4-(2,2,2-Trichloräthoxycarbonylamino)mandelsäure in 140 ml N,N-Dimethylformamid und 38,4 ml Triäthylamin werden innert 2 Stunden unter Rühren bei Raumtemperatur 27 ml Benzylbromid getropft. Das Reaktionsgemisch wird 18 Stunden bei Raumtemperatur gerührt, die Suspension auf viel Wasser gegossen und zweimal mit einem Gemisch von Aethylacetat-Diäthyläther-(1:1) ausgezogen. Die organischen Extrakte werden vereinigt, zweimal mit Solelösung gewaschen und über Natriumsulfat getrocknet. Nach Abdampfen des Lösungsmittels wird das Produkt mit Petroläther digeriert und der (R,S) - 4 - (2,2,2-Trichloräthoxycarbonylamino)mandelsäure-benzylester kristallin erhalten. F. 72—76° (Zersetzung).

d) Eine Lösung von 20 g (R,S) - 4 - (2,2,2-Trichloräthoxycarbonylamino)mandelsäure-benzylester in 200 ml Eisessig wird unter Rühren bei Raumtemperatur während 3 Stunden portionenweise mit insgesamt 21 g Zinkpulver in gleichen Zeitabständen versetzt. Anschliessend wird die Suspension weitere 2 Stunden bei Raumtemperatur gerührt, dann durch Celit abgenutscht, das Nutschgut mit Aethylacetat gewaschen und das Filtrat am Rotationsverdampfer bei 50° stark eingeengt. Der Rückstand wird auf Wasser gegossen, mit Aethylacetat überschichtet, durch Zugabe von festem Natriumhydrogencarbonat leicht alkalisch gestellt (pH 7,5—8,0) und zweimal mit Aethylacetat ausgezogen. Die organischen Extrakte werden vereinigt, sukzessive mit Solelösung, 1N Natriumhydrogencarbonat und Solelösung gewaschen, über Natriumsulfat getrocknet und das Lösungsmittel am Rotationsverdampfer bei 45° abgedampft. Der erhaltene (R,S)-4-Amino-mandelsäure-benzylester hat den Schmelzpunkt 88—92° (Zersetzung).

e) Eine Lösung von 2,50 ml Pyridin in 10 ml Methylenchlorid wird unter Rühren und Kühlen innert 4 Minuten bei 0° bis +5° in ein Gemisch von 16 ml Phosgenlösung (20% in Toluol) und 10 ml Methylenchlorid getropft. Darauf wird eine Lösung von 8,0 g N-BOC-(R)-Serin-tert.-butylester in 40 ml Methylenchlorid innert 15 Minuten in die obige Suspension getropft und anschliessend das Reaktionsgemisch 1 Stunde bei 0° bis +5° gerührt. Nach Zugabe einer Lösung von 7,8 g (R,S)-4-Amino-mandelsäure-benzylester in 80 ml Methylenchlorid und 2,7 ml Pyridin innert 3 Minuten bei 0° wird die Suspension 30 Minuten bei 0° und 30 Minuten bei Raumtemperatur gerührt, darauf mit 400 ml Diäthyläther verdünnt und sukzessive mit Wasser, Zitronensäurelösung (5%), Wasser, 0,1 N Salzsäure und Wasser gewaschen. Nach Trocknen und Abdampfen des Lösungsmittels wird der ölige (R,S) - 4 - ((2R) - BOC - Amino - 2 - tert. - butoxycarbonyläthoxycarbonylamino)mandelsäure-benzylester durch Verreiben mit Petroläther zum Kristallisieren gebracht. F. 98—100°; $[\alpha]_D = -17 \pm 1°$ (c = 2,83 in Chloroform); DS (Fliessmittel: Toluol-Chloroform-Aethylacetat-(1:1:1)): Rf = 0,46.

f) Eine Lösung von 20,0 g (R,S) - 4 - ((2R) - 2 - BOC - Amino - 2 - tert. - butoxycarbonyläthoxycarbonylamino)mandelsäure - benzylester in 40 ml Tetrahydrofuran und 40 ml Pyridin wird im Eisbad abgekühlt und unter Rühren innert 20 Minuten bei 0 bis +5° ein Lösung von 6,90 ml Chlorameisensäure-2,2,2-trichloräthylester in 40 ml Tetrahydrofuran zugetropft. Das Reaktionsgemisch wird 3 Stunden bei 0° gerührt, mit Diäthyläther verdünnt und die organische Phase sukzessive mit Wasser, Zitronensäurelösung (15%), Wasser, 1N Natriumhydrogencarbonat und Wasser gewaschen. Die Lösung wird über Natriumsulfat getrocknet und das Lösungsmittel am Rotationsverdampfer bei 45° abgedampft. Durch Vermischen mit Petroläther wird der ($\alpha$R,S) - 4 - ((2R) - 2 - BOC - Amino - 2 - tert. - butoxycarbonyläthoxycarbonylamino) - $\alpha$ - (2,2,2 - trichloräthoxycarbonyloxy) - phenylessig - säure - benzylester fest. F. 85—90° (Zersetzung). $[\alpha]_D = -15° + 1°$ (c = 3,02 in Chloroform); DS (Fliessmittel: Toluol-Chloroform-Aethylacetat-(4,5:4,5:1)): Rf = 0,42.

g) Eine Lösung von 18,0 g ($\alpha$R,S) - 4 - ((2R) - 2 - BOC - Amino - 2 - tert. - butoxycarbonyläthoxycarbonylamino) - $\alpha$ - (2,2,2 - trichloräthoxycarbonyloxy) - phenylessigsäure-benzylester in 250 ml Aethylacetat wird in Gegenwart von 3,0 g Palladium auf Aktivkohle (10%) hydriert. Nach ca. 2 Stunden ist die berechnete Wasserstoffmenge aufgenommen. Der Katalysator wird abfiltriert, mit Aethylacetat gewaschen, das Filtrat am Rotationsverdampfer bei 45° eingeengt und die ($\alpha$R,S) - 4 - ((2R) - 2 - BOC - Amino - 2 - tert. - butoxycarbonyläthoxycarbonylamino) - $\alpha$ - (2,2,2 - trichloräthoxycarbonyloxy) - phenylessigsäure durch Zugabe von Petroläther gefällt. F. 94—100° (Zersetzung); $[\alpha] = -18° \pm 1°$ (c = 0,93 in Chloroform).

h) Eine Lösung von 7,25 g 7$\beta$-Amino-3-carbamoyloxymethyl-3-cephem-4-carbonsäure-diphenylmethylester und 10,0 g ($\alpha$R,S) - 4 - ((2R) - 2 - BOC - Amino - 2 - tert. - butoxycarbonyläthoxycarbonylamino) - $\alpha$ - (2,2,2 - trichloräthoxycarbonyloxy) - phenylessigsäure in 125 ml Tetrahydrofuran wird mit einer Lösung von 3,75 g N,N'-Dicyclohexylcarbodiimid in 25 ml Tetrahydrofuran und später mit einer zweiten Portion von 1,75 g festem N,N'-Dicyclohexylcarbodiimid nach dem Verfahren von Beispiel 1 umgesetzt. Das Rohprodukt wird durch Chromatographieren an der 25-fachen Menge Kieselgel gereinigt. Die Fraktionen mit Methylenchlorid-Methylacetat-(17:3) als Eluiermittel werden vereinigt und der 7$\beta$ - {(2R,S) - 2 - [4 - ((2R) - 2 - BOC - Amino - 2 - tert. - butoxycarbonyläthoxycarbonylamino)phenyl] - 2 - (2,2,2 - trichloräthoxycarbonyloxy)acetylamino} - 3 - carbamoyloxymethyl - 3 - cephem - 4 - carbonsäure - diphenylmethylester aud Aethylacetatlösung mit einem Gemisch von Petroläther und Diäthyläther gefällt. F. 138—145° (Zersetzung). DS (Fliessmittel: Toluol-Aethylacetat-Aethanol-(5:5:0,1)): Rf = 0,54.

## Beispiel 16

a) Eine Lösung von 8,50 g 7$\beta$ - {(2R,S) - 2 - [4 - ((2R) - 2 - BOC - Amino - 2 - tert. - butoxy - carbonyläthoxycarbonylamino)phenyl] - 2 - (2,2,2 - trichloräthoxycarbonyloxy) - acetylamino} - 3 - [(1 - methyl - 1H - tetrazol - 5 - yl) - thiomethyl] - 3 - cephem - 4 - carbonsäure - diphenylmethylester, in 85 ml Acetonitril und 85 ml Eisessig wird im Eisbad abgekühlt und unter starkem Rühren bei +2° während 90 Minuten portionenweise mit insgesamt 22 g Zinkpulver in gleichen Zeitabständen versetzt. Anschliessend wird die Suspension weitere 2 Stunden im Eisbad gerührt und wie im Beispiel 15a) aufgearbeitet. Das Rohprodukt wird durch Chromatographieren an der 30-fachen Menge Kieselgel gereinigt. Die Fraktionen mit Methylenchlorid-Methylacetat-(17:3) als Eluiermittel werden vereinigt und der 7$\beta$ - {(2R,S) - 2 - [4 - ((2R) - 2 - BOC - Amino - 2 - tert. - butoxycarbonyläthoxycarbonylamino)phenyl] - 2 - hydroxy - acetylamino} - 3 - [(1 - methyl - 1H - tetrazol - 5 - yl) - thiomethyl] - 3 - cephem - 4 - carbonsäure - diphenylmethylester aus Aethylacetatlösung mit einem Gemisch von Petroläther und Diäthyläther gefällt. F. 150—155° (Zersetzung). DS (Fliessmittel: Toluol-Chloroform-Aethylacetat-(1:1:1)): Rf = 0,09 und 0,15 (Diastereomerengemisch).

Ein Gemisch von 2,40 g 7$\beta$ - {(2R,S) - 2 - [4 - ((2R) - 2 - BOC - Amino - 2 - tert. - butoxycarbonyläthoxcarbonylamino)phenyl] - 2 - hydroxyacetylamino} - 3 - [(1 - methyl - 1H - tetrazol - 5 - yl) - thiomethyl] - 3 - cephem - 4 - carbonsäure - diphenylmethylester, 1,60 ml Methylenchlorid, 1,00 ml Anisol und 48 ml Trifluoressigsäure wird 75 Minuten bei Raumtemperatur unter Ausschluss von Luftfeuchtigkeit gerührt. Darauf wird die Suspension auf ein eiskaltes Gemisch von Petroläther (500 ml) und Diäthyläther (250 ml) gegossen, das anfallende Trifluoracetat der 7$\beta$ - {(2R,S) - 2 - [4 - ((2R) - Amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure abgenutscht, mit Diäthyläther gewaschen und im Hochvakuum bei Raumtemperatur getrocknet.

Das entsprechende Natriumsalz wird aus dem Trifluoracetat nach dem Verfahren von Beispiel 1a) erhalten. F. ab 170° (Zersetzung). DS: $Rf_{101} = 0,27$; $Rf_{101A} = 0,21$; $Rf_{52A} = 0,06$.

Das Ausgangsmaterial kann wie folgt hergestellt werden:

b) Eine Lösung von 7,85 g 7$\beta$-Amino-3-[(1-methyl-1H-tetrazol-5-yl)-thiomethyl]-3-cephem-4-carbonsäurediphenylmethylester und 10,0 g ($\alpha$R,S) - 4 - ((2R) - 2 - BOC - Amino - 2 - tert. - butoxycarbonyläthoxycarbonyloxy) - phenylessigsäure in 40 ml Tetrahydrofuran und 20 ml N,N-Dimethylformamid wird mit einer Lösung von 3,25 g N,N'-Dicyclohexylcarbodiimid in 15 ml Tetrahydrofuran bzw. mit 1,60 g festem N,N'-Dicyclohexylcarbodiimid nach dem Verfahren von Beispiel 1 umgesetzt. Das Rohprodukt wird durch Filtrieren an der 15 fachen Menge Kieselgel gereinigt. Die

Fraktionen mit Methylenchlorid-Methylacetat-(93:7) als Eluiermittel werden vereinigt und der 7β - {(2R,S) - 2 - [4 - ((2R) - 2 - BOC - Amino - 2 - tert. - butoxycarbonyläthoxycarbonylamino)phenyl] - 2 - (2,2,2 - trichloräthoxycarbonyloxy)acetylamino} - 3 - [(1 - methyl - 1H - tetrazol - 5 - yl) - thiomethyl] - 3 - cephem - 4 - carbonsäure - diphenylmethylester aus Aethylacetatlösung mit einem Gemisch von Petroläther und Diäthyläther gefällt. DS (Fliessmittel: Toluol-Chloroform-Aethylacetat-(1:1:1)) Rf = 0,49.

Beispiel 17

a) Ein Gemisch von 2,0 g 7β - {(2R) - 2 - [4 - ((2R) - 2 - BOC - Amino - 2 - diphenylmethoxycarbonyläthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure - diphenylmethylester, 2,0 ml Methylenchlorid, 1,2 ml Anisol und 16 ml eiskalter Trifluoressigsäure wird 15 Minuten bei Raumtemperatur unter Ausschluss der Luftfeuchtigkeit gerührt. Aus der anfänglich klaren Lösung fällt ein voluminöser Niederschlag aus. Anschliessend wird die Suspension auf ein eiskaltes Gemisch von Petroläther (400 ml) und Diäthyläther (200 ml) gegossen, das anfallende Trifluoracetat der 7β - {(2R) - 2 - [4 - ((2R) - 2 - Amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure abgenutscht, mit Diäthyläther gewaschen und im Hochvakuum bei Raumtemperatur getrocknet. Das entsprechende Natriumsalz wird aus dem Trifluoracetat nach dem Verfahren von Beispiel 1a) erhalten. F. ab 165° (Zersetzung); $[\alpha]_D = -3° \pm 1°$ (c = 1,83 in 0,1 N Natriumhydrogencarbonat); D9: $Rf_{101} = 0,30$; $Rf_{101A} = 0,23$; $Rf_{52A} = 0,07$.

Das Ausgangsmaterial kann wie folgt hergestellt werden:

b) Eine Lösung von 5,70 g Pyridin in 10 ml Methylenchlorid wird unter Rühren und Kühlen bei 0° bis +3° in 105 ml Phosgenlösung (20% in Toluol) getropft und das Reaktionsgemisch 10 Minuten bei 0° gerührt. Nach Zutropfen einer Lösung von 25,0 g (R)—N—BOC—Serin-diphenylmethylester innert 20 Minuten bei 0°, wird das Gemisch 1 Stunde im Eisbad gerührt und darauf mit Methylenchlorid verdünnt. Die Methylenchloridlösung wird dreimal mit eiskaltem Wasser gewaschen, über Natriumsulfat getrocknet und das Lösungsmittel am Rotationsverdampfer bei 45° abgedampft. Beim Verreiben mit Petroläther wird das erhaltene (2R)-2-BOC-Amino-2-diphenylmethoxycarbonyläthoxycarbonylchlorid fest. F. 79—80° (Zersetzung).

c) 8,86 g (R)-4-Amino-mandelsäure werden in einem Gemisch von 90 ml Acetonitril und 20 ml Tetrahydrofuran aufgeschlämmt, auf 0° abgekühlt und innert 75 Minuten unter Rühren bei 0° portionenweise mit insgesamt 25 ml N,O-Bis-(trimethylsilyl)-acetamid versetzt, wobei eine klare Lösung entsteht. Diese Lösung wird auf —15° abgekühlt und unter Rühren mit 4,2 ml Pyridin anschliessend mit einer Lösung von 23 g (2R)-2-BOC-Amino-2-diphenylmethoxycarbonyläthoxycarbonylchlorid in einem Gemisch von 90 ml Acetonitril und 90 ml Tetrahydrofuran versetzt. Nach 2,5 Stunden Rühren im Eisbad wird das Gemisch mit Aethylacetat verdünnt, dreimal mit Wasser gewaschen, über Natriumsulfat getrocknet und das Lösungsmittel am Rotationsverdampfer bei 45° abdestilliert. Bei Zugabe von Petroläther wird die (R)-4-((2R)-2-BOC-Amino-2-diphenylmethoxycarbonyläthoxycarbonylamino)-mandelsäure fest. F. 65—71°; $[\alpha]_D = -36° \pm 1°$ (c = 2,93 in Dimethylsulfoxid); DS (Fliessmittel: Chloroform-Aethylacetat-Eisessig-(5:5:0,2)): Rf = 0,23.

d) Eine Lösung von 7,55 g 7β - Amino - 3 - [(1 - methyl - 1H - tetrazol - 5 - yl) - thiomethyl] - 3 - cephem - 4 - carbonsäure - diphenylmethylester, 8,50 g (R) - 4 - ((2R) - 2 - BOC - Amino - 2 - diphenylmethoxycarbonyläthoxycarbonylamino)mandelsäure und 2,85 g 1-Hydroxybenzotriazol (angefeuchtet mit 10% Wasser) in einem Gemisch von 30 ml N,N-Dimethylformamid und 45 ml Tetrahydrofuran wird mit einem Eisbad auf 0° bis +3° abgekühlt, unter Kühlen und Rühren innert 30 Minuten eine Lösung von 1,70 g N,N'-Dicyclohexylcarbodiimid in 20 ml Tetrahydrofuran zugetropft und das Reaktionsgemisch im Eisbad weitergerührt. Nach 35 Stunden wird eine weitere Lösung von 1,50 g N,N'-Dicyclohexylcarbodiimid in 20 ml Tetrahydrofuran zugetropft. Nach insgesamt 7,5 Stunden bei 0° wird die Suspension abgenutscht, das Nutschgut mit Aethylacetat gewaschen, das Filtrat mit viel Aethylacetat verdünnt, die Aethylacetlösung sukzessive mit Wasser, 1N Natriumhydrogencarbonat und Wasser gewaschen. Das Rohprodukt wird durth Chromatographieren an der 25-fachen Menge Kieselgel gereinigt. Die Fraktionen mit Chloroform-Methylacetat-(14:1) als Eluiermittel werden vereinigt und der 7β - {(2R) - 2 - [4 - ((2R) - 2 - BOC - Amino - 2 - diphenylmethoxycarbonyläthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure - diphenylmethylester aus Aethylacetat kristallisiert. F. 169—172° (Zersetzung). DS (Fliessmittel: Chloroform-Aethylacetat-Aethanol-(5:5:0,2)): Rf = 0,57. $[\alpha]_D = -52 \pm 1°$ (c = 1,75 in Dimethylsulfoxid).

Beispiel 18

a) Ein Gemisch von 2,0 g 7β - {(2R) - 2 - [4 - ((2R) - 2 - BOC - Amino - 2 - diphenylmethoxycarbonyläthoxycarbonylamino)phenyl] - 2 - hydroxyacetylamino} - 3 - carbamoyloxymethyl - 3 - cephem - 4 - carbonsäure - diphenylmethylester, 2,0 ml Methylenchlorid, 1,20 ml Anisol und 16,0 ml eiskalter Trifluoressigsäure wird 15 Minuten bei Raumtemperatur unter Ausschluss der Luftfeuchtigkeit gerührt. Darauf wird die Suspension auf ein eiskaltes Gemisch von Petroläther (400

ml) und Diäthyläther (200 ml) gegossen, das anfallende Trifluoracetat der 7β - {(2R) - 2 - [4 - ((2R) - 2 - Amino - 2 - carboxyäthoxycarbonylamino)phenyl] - 2 - hydroxyacetylamino} - 3 - carbamoyloxymethyl - 3 - cephem - 4 - carbonsäure abgenutscht, mit Diäthyläther gewaschen und im Hochvakuum bei Raumtemperatur getrocknet. Das entsprechende Natriumsalz wird aus dem Trifluoracetat nach dem Verfahren von Beispiel 1a) erhalten. F. ab 230° (Zersetzung); $[\alpha]_D = +62 \pm 1°$ (c = 1,93 in 0,1 N Natriumhydrogencarbonat); DS: $Rf_{52A} = 0{,}06$; $R_{101} = 0{,}32$; $Rf_{101A} = 0{,}24$.

Das Ausgangsmaterial kann wie folgt hergestellt werden:

b) Eine Lösung von 8,0 g 7β-Amino-3-carbamoyloxymethyl-3-cephem-4-carbonsäure-diphenylmethylester, 10,0 g (R) - 4 - ((2R) - 2 - BOC - Amino - 2 - diphenylmethoxycarbonyläthoxycarbonylamino)mandelsäure und 3,40 g 1-Hydroxybenzotriazol in 80 ml Tetrahydrofuran wird auf +3° abgekühlt, dann unter Rühren und Kühlen innert 30 Minuten eine Lösung von 4,0 g N,N'-Dicyclohexylcarbodiimid in 20 ml Tetrahydrofuran zugetropft und das Reaktionsgemisch 7 Stunden im Eisbad gerührt. Anschliessend wird die Suspension nach dem Verfahren von Beispiel 17d aufgearbeitet. Das Rohprodukt wird durch Chromatographieren an der 30-fachen Menge Kieselgel gereinigt. Die Fraktionen mit Chloroform-Methylacetat-(4:1) als Eluiermittel werden vereinigt und das Produkt aus Chloroform kristallisiert. Der 7β - {(2R) - 2 - [4 - ((2R) - 2 - BOC - Amino - 2 - diphenylmethoxycarbonyläthoxycarbonylamino)phenyl] - 2 - hydroxyacetylamino} - 3 - carbamoyloxymethyl - 3 - cephem - 4 - carbonsäure - diphenylmethylester schmilzt bei 129—133° unter Zersetzung; $[\alpha]_D = +22 \pm 1°$ (c = 3,41 in Dimethylsulfoxid); DS (Fliessmittel: Chloroform Aethylacetat-Aethanol-(5:5:0,1)): Rf = 0,32.

Beispiel 19

a) Ein Gemisch von 2,10 g 7α - Methoxy - 7β - {2 - [4 - ((2R) - 2 - BOC - amino - 2 - diphenylmethoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester, 2,0 ml Methylenchlorid, 1,1 ml Anisol und 15 ml eiskalter Trifluoressigsäure wird 15 Minuten bei Raumtemperatur unter Ausschluss der Luftfeuchtigkeit gerührt. Darauf wird die Suspension auf ein eiskaltes Gemisch von Petroläther (400 ml) und Diäthyläther (200 ml) gegossen, das anfallende Trifluoracetat abgenutscht, mit Diäthyläther gewaschen und im Hockvakuum bei Raumtemperatur getrocknet. Das 7α - Methoxy - 7β - {2 - [4 - ((2R) - 2 - amino - 2 - carboxyäthoxycarbonylamino)phenyl]-acetylamino} - 3 - [(1 - methyl - 1H - tetrazol - 5 - yl) - thiomethyl] - 3 - cephem - 4 - carbonsäure - natriumsalz wird aus dem Trifluoracetat nach dem Verfahren von Beispiel 1 erhalten. F. ab 170° (Zersetzung); DS: $Rf_{52A} = 0{,}05$; $Rf_{101} = 0{,}33$, $Rf_{101A} = 0{,}47$.

Das Ausgangsmaterial kann wie folgt hergestellt werden:

b) Der 7β - {2 - [4 - ((2R) - 2 - BOC - Amino - 2 - diphenylmethoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester wird durch Umsetzung von 7β - Amino - 3 - [(1 - methyl - 1H - tetrazol - 5 - yl) - thiomethyl] - 3 - cephem - 4 - carbonsäure - diphenylmethylester mit 4 - ((2R) - 2 - BOC - Amino - 2 - diphenylmethoxycarbonyläthoxycarbonylamino)phenylessigsäure nach dem Verfahren von Beispiel 5f) erhalten. F. 128—132° (Zersetzung) (kristallisiert aus Chloroform). DS Fliessmittel: Toluol-Aethylacetat-(3:2)): Rf = 0,34.

c) Die folgende Reaktion wird in einer Stickstoff-Atmosphäre ausgeführt. Eine Lösung von 5,0 g 7β - {2 - [4 - ((2R) - 2 - BOC - Amino - 2 - diphenylmethoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester in 400 ml absolutem Tetrahydrofuran wird auf —75° (Trockeneis-Aceton-Bad) abgekühlt und eine vorgekühlte Lösung von Lithiummethoxid in Methanol (130 mg Lithium in 16 ml Methanol gelöst) innert 2 Minuten zugetropft, wobei die Temperatur auf —70° steigt und ein organgegefärbte Lösung eintsteht. Das Reaktionsgemisch wird weitere 2 Minuten bei —75° gerührt, dann 0,90 ml tert.-Butylhypochlorit zugegeben und bei —75° weitergerührt. Nach 5 Minuten wird die braungefärbte Lösung mit weiteren 0,20 ml tert.-Butylhypochlorit versetzt. Nach 20 Minuten werden 3 ml Eisessig in das Reaktionsgemisch getropft. Das Kühlbad wird entfernt und die Lösung auf Raumtemperatur erwärmt. Das Gemisch wird mit Aethylacetat verdünnt, das Tetrahydrofuran am Rotationsverdampfer bei 45° abgedampft, die Aethylacetatlösung sukzessive mit Wasser, 1N Natriumhydrogencarbonat und Wasser gewaschen, über Natriumsulfat getrocknet und das Lösungsmittel abgedampft. Das Rohprodukt wird an der 30-fachen Menge Kieselgel chromatographier. Die Fraktionen mit Methylenchlorid-Methylacetat-(20:1) als Eluiermittel werden vereinigt. Man erhält das Δ2/Δ3 Isomerengemisch des 7α-Methoxy-cephalosporinderivates. DS (Fliessmittel: Toluol-Aethylacetat-(3:2)): Rf = 0,36.

1.30 g Δ2/Δ3 Isomerengemisch des 7α-Methoxy-cephalosporinderivates werden in 6 ml Chloroform gelöst, im Eisbad abgekühlt, unter Rühren und Kühlen eine Lösung von 0,24 g 3-Chlorperbenzoesäure (85%) zugetropft und das Reaktionsgemisch 1,5 Stunden bei 0° gerührt. Die Lösung wird mit Aethylacetat verdünnt, sukzessive mit Wasser, 1N Natriumhydrogencarbonat, Wasser, Natriumhydrogensulfitlösung (5%) und Wasser gewaschen, über Natriumsulfat getrocknet und das Lösungsmittel am Rotationsverdampfer bei 45° abgedampft. Das Rohprodukt wird durch Filtrieren an der 10-fachen Menge Kieselgel gereinigt. Das 7α - Methoxy - 7β - {2 - [4 - ((2R) - 2 - BOC - amino -

2 - diphenylmethoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester - 1$\beta$ - oxid wird mit einem Gemisch von Methylenchlorid-Methylacetat-(7:3) eluiert. DS (Fliessmittel: Aethylacetat): Rf = 0,38.

d) Eine Lösung von 2,40 g 7$\alpha$ - Methoxy - 7$\beta$ - {2 - [4 - ((2R) - 2 - BOC - amino - 3 - diphenylmethoxycarbonylamino)phenyl]acetylamino} - 3 - [(1 - methyl - 1H - tetrazol - 5 - yl) - thiomethyl] - 3 - cephem - 4 - carbonsäure - diphenylmethylester - 1$\beta$ - oxid in einem Gemisch von 150 ml Methylenchlorid und 10 ml N,N-Dimethylacetamid wird auf 0° abgekühlt, unter Rühren und Kühlen mit 0,50 ml Phosphortrichlorid versetzt und 15 Minuten im Eisbad gerührt. Das Reaktionsgemisch wird mit Methylenchlorid verdünnt, sukzessive mit Wasser, 1N Natriumhydrogencarbonat und Wasser gewaschen, über Natriumsulfat getrocknet und das Lösungsmittel am Rotationsverdampfer bei 45° abgedampft. Das Produkt wird durch Chromatographieren an der 20-fachen Menge Kieselgel gereinigt. Die Fraktionen mit Methylenchlorid-Methylacetat-(20:1) als Eluiermittel werden vereinigt. Der 7$\alpha$ - Methoxy - 7$\beta$ - {2 - [4 - ((2R) - 2 - BOC - amino - 2 - diphenylmethoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester wird aus Aethylacetatlösung mit einem Gemisch von Petroläther und Aether gefällt. DS (Fliessmittel: Toluol-Aethylacetat-(3:2)): Rf = 0,36.

## Beispiel 20

a) Eine Suspension von 3,0 g 7$\beta$ - {(2R,S) - 2 - [4 - ((2R) - 2 - Amino - 2 - diphenylmethoxycarbonyläthoxycarbonylamino)phenyl] - 2 - sulfoacetylamino} - 3 - [(1 - methyl - 1H - tetrazol - 5 - yl) - thiomethyl] - 3 - cephem - 4 - carbonsäure - diphenylmethylester in 25 ml Methylenchlorid und 1,0 ml Anisol wird auf 0° abgekühlt, mit 4,65 ml eiskalter Trifluoressigsäure versetzt und 40 Minuten im Eisbad unter Ausschluss der Luftfeuchtigkeit gerührt. Aus der anfänglich klaren Lösung fällt ein schmieriger Niederschlag aus. Nach dem Ablauf der Reaktionszeit wird die Schmiere durch Zugabe von 10 ml eiskalter Trifluoressigsäure rasch in Lösung gebracht, dann auf ein eiskaltes Gemisch von Petroläther (500 ml) und Diäthyläther (250 ml) gegossen. Das anfallende Trifluoracetat wird abgenutscht, mit Diäthyläther gewaschen und im Hockvakuum bei Raumtemperatur getrocknet. Das 7$\beta$ - {(2R,S) - 2 - [4 - ((2R) - 2 - Amino - 2 - carboxyäthoxycarbonylamino)phenyl] - 2 - sulfoacetylamino} - 3 - [(1 - methyl - 1H - tetrazol - 5 - yl) - thiomethyl] - 3 - cephem - 4 - carbonsäure - dinatriumsalz wird aus dem Trifluoracetat nach dem Verfahren von Beispiel 1a) erhalten. F. ab 205° (Zersetzung). DS: $Rf_{101}$ = 0,23; $Rf_{101A}$ = 0,14.

Das Ausgangsmaterial kann wie folgt hergestellt werden:

b) Eine Lösung von 88 g ($\alpha$R,S)-4-Nitro-$\alpha$-sulfophenylessigsäure in 430 ml Wasser wird in Gegenwart von 12,5 g Palladium auf Aktivkohle (10%) hydriert. Nach ca. 12 Stunden kommt die Wasserstoffaufnahme zum Stillstand. Der Katalysator wird abfiltriert, mit wenig Wasser gewaschen, das Filtrat im Eisbad abgekühlt, unter Rühren und Kühlen durch Zutropfen von konzentrierter Salzsäure sauer gestellt (pH) 1,2) und die Suspension einige Stunden im Eisbad stehengelassen. Die anfallende ($\alpha$R.S)-4-Amino-$\alpha$-sulfophenylessigsäure wird abgenutscht und mit eiskaltem Wasser gewaschen. F. über 300° (Zersetzung). DS: $Rf_{52A}$ = 0,03.

c) 17,5 g ($\alpha$R,S)-4-Amino-$\alpha$-sulfo-phenylessigsäure werden in einem Gemisch von 120 ml Methylenchlorid und 40 ml Acetonitril aufgeschlämmt und unter Rühren innert 75 Minuten portionenweise mit insgesamt 60 ml N,O-Bis-(trimethylsilyl)-acetamid versetzt. Die erhaltene klare Lösung wird noch 1 Stunde bei Raumtemperatur gerührt, auf 0° abgekühlt und nacheinander mit 6,10 ml Pyridin und einer Lösung von 34 g (2R)-2-BOC-Amino-2-diphenylmethoxycarbonyläthoxycarbonylchlorid in 200 ml Methylenchlorid versetzt. Nach 1 Stunde Rühren im Eisbad und 1,5 Stunden bei Raumtemperatur wird das Reaktionsgemisch in 4 Liter Aethylacetat gegossen, sukzessive mit 0,1 N Salzsäure, Wasser und Solelösung gewaschen, über Natriumsulfat getrocknet und das Lösungsmittel am Rotationsverdampfer bei 45° abgedampft. Beim Vermischen mit Petroläther wird die ($\alpha$R,S)-4-((2R)-2-BOC-Amino-2-diphenylmethoxycarbonyläthoxycarbonylamino)-$\alpha$-sulfo-phenylessigsäure fest. F. ab 160° (Zersetzung). DS: $Rf_{52A}$ = 0,42; $Rf_{67}$ = 0,31.

Eine Suspension von 26,5 g ($\alpha$R,S) - 4 - ((2R) - 2 - BOC - Amino - 2 - diphenylmethoxycarbonyläthoxycarbonylamino) - $\alpha$ - sulfo - phenylessigsäure und 40 g Toluol-4-sulfonsäure-monohydrat in 430 ml Acetonitril wird 5 Stunden bei Raumtemperatur gerührt, dann mit 250 ml Wasser versetzt und das Acetonitril am Rotationsverdampfer bei 50° abgedampft. Das angefallene Produkt wird abgenutscht und mit Wasser gewaschen. Die ($\alpha$R,S) - 4 - ((2R) - 2 - Amino - 2 - diphenylmethoxycarbonyläthoxycarbonylamino) - $\alpha$ - sulfo - phenylessigsäure wird durch Lösen in Wasser bei pH 6,8 (Zugabe von 2N Natronlauge), Filtrieren und Ansäuern der klaren Lösung mit 2N Salzsäure (pH 1,5) gereinigt. F. ab 190° (Zersetzung); $[\alpha]_D = +11 \pm 1°$ (c = 2,55 in Dimethylsulfoxid); DS: $Rf_{52A}$ = 0,23.

d) Die folgende Reaktion wird in einer Stickstoffatmosphäre ausgeführt. Eine Lösung von 5,1 g 7$\beta$ - Amino-3-[(1-methyl-1H-tetrazol-5-yl)-thiomethyl]-3-cephem-4-carbonsäure-diphenylmethylester und 5,0 g ($\alpha$R,S) - 4 - ((2R) - 2 - Amino - 2 - diphenylmethoxycarbonyläthoxycarbonylamino) - $\alpha$ - sulfo - phenylessigsäure in einem Gemisch von 270 ml Tetrahydrofuran und 30 ml Wasser wird

mit 2,6 g N,N'-Dicyclohexylcarbodiimid versetzt und das Reaktionsgemisch 8 Stunden bei Raumtemperatur gerührt. Darauf wird die Suspension abgenutscht, das Filtrat mit einem Gemisch von 1,4 Liter Petroläther und 0,7 Liter Diäthyläther verrrührt und das anfallende Produkt abgenuscht. Das Nutschgut wird in einem Gemisch von Aethanol (150 ml)-Diäthyläther (150 ml)-Petroläther (150 ml) aufgeschlämmt, abgenutscht und im Hochvakuum bei Raumtemperatur getrocknet. Das Rohprodukt wird mit 250 ml Methylenchlorid gerührt, der unlösliche Teil abgetrennt, die klare Lösung am Rotationsverdampfer bei 45° abgedampft und der zurückbleibende Schaum an der 25-fachen Menge Kieselgel chromatographiert. Die Fraktionen mit Methylenchlorid-Trifluoräthanol-Wasser-7:4:0,2) werden vereinigt. Man erhält daraus den 7β - {(2R,S) - 2 - [4 - ((2R) - 2 - Amino - 2 - diphenylmethoxycarbonylamino)phenyl] - 2 - sulfoacetylamino} - 3 - [(1 - methyl - 1H - tetrazol - 5 - yl) - thiomethyl] - 3 - cephem - 4 - carbonsäure - diphenylmethylester. F. 173—175° unter Zersetzung. DS (Fliessmittel: Methylacetat-Aethanol-(4:1)): Rf = 0,42.

Beispiel 21

a) Eine auf 0° gekühlte Lösung von 5,4 g (5,3 mMol) 7β - {(2R) - 2 - BOC - Amino - 2 - [3 - ((2R) - 2 - BOC - amino - 2 - diphenylmethoxycarbonyläthoxycarbonylamino)phenyl]acetylamino} - 3 - methoxy - 3 - cephem - 4 - carbonsäure - diphenylmethylester und 5,4 ml Anisol in 30 ml absolutem Methylenchlorid wird mit 25 ml Trifluoressigsäure versetzt und 30 Minuten unter Feuchtigkeitsausschluss gerührt. Nach Zugabe von 250 ml Petroläther-Diäthyläther 1:1 wird der gebildete Niederschlag abfiltriert, mit Diäthyläther gewaschen und getrocknet. Die wässrige Lösung (35 ml) des rohen Ditrifluoracetates wird mit Aethylacetat (3 x 20 ml) extrahiert, mit 2N-Natronlauge auf pH 5 gestellt und tropfenweise mit 130 ml Isopropanol versetzt. Die erhaltene 7β - {(2R) - 2 - Amino - 2 - [3 - (2R) - 2 - amino - 2 - carboxyäthoxycarbonylamino)phenyl]acetylamino} - 3 - methoxy - 3 - cephem - 4 - carbonsäure wird aus 140 ml Wasser-Isopropanol 1:3 umkristallisiert, zur Entfernung von isopropanol zweimal mit wenig Wasser abgezogen und am Hochvakuum getrocknet. F. 170° Zersetzung. DS ($Rf_{96} = 0,1$; $[\alpha]_D = 112 \pm 1°$ (c = 1,17 in 0,1 N HCl).

Das Ausgangsmaterial kann wie folgt hergestellt werden:

b) Eine auf 0° gekühlte Lösung von 4,45 g (12 mMol) (2R)-N-BOC-Serin-diphenylmethylester und 1,0 ml absolutem Pyridin in 100 ml absolutem Methylenchlorid wird unter Rühren und Feuchtigkeitsausschluss mit 6,2 ml 20%-igem Phosgen in Toluol versetzt und 3 Stunden bei Raumtemperatur gerührt (Lösung A).

3,2 g (12 mMol) (2R)-2-BOC-Amino-2-(3-amino)phenyl-essigsäure gelöst in 100 ml absolutem Methylenchlorid werden unter Stickstoff mit 3,2 ml Bis(trimethylsilyl)-acetamid versetzt und eine Stunde bei Raumtemperatur gerührt (Lösung B).

Bei 0° wird die Lösung B innert 5 Minuten zur Lösung A getropft, anschliessend mit 1,3 ml N-Methylmorpholin versetzt und 30 Minuten bei 0° und 3 Stunden bei 25° geruhrt. Die Lösung wird mit Eiswasser und gesättigter Natriumchloridlösung gewaschen, über Natriumsulfat getrocknet und am Rotationsverdampfer eingeengt. Das Rohprodukt wird an Kieselgel mit Diäthyläther als Eluiermittel gereinigt, woraus (2R) - 2 - BOC - Aminc - 2 - [3 - (2R) - 2 - BOC - amino - 2 - diphenylmethoxycarbonyläthoxycarbonylamino)phenyl - essigsäure als farbloses amorphes Pulver isoliert wird. DS: $Rf_{96} = 0,9$.

c) Eine auf —20° gekühlte Lösung von 3,4 g (5,1 mMol) (2R) - 2 - BOC - Amino - 2 - [3 - ((2R) - 2 - BOC - amino - 2 - diphenylmethoxycarbonyläthoxycarbonylamino)phenyl]essigsäure und 0,59 ml (5,1 mMol) N-Methylmorpholin in 150 ml absolutem Methylenchlorid wird unter Feuchtigkeitsausschluss und Rühren mit 0,67 ml (5,1 mMol) Chlorameisensäure-isobutylester versetzt. Zum so erhältlichen gemischten Anhydrid werden in einer Portion 2,21 g (5,1 mMol) 7β-Amino-3-methoxy-3-cephem-4-carbonsäure-diphenylmethylester-hydrochlorid, dann 0,57 ml N-Methylmorpholin bei —10° gegeben Nach 2 Stunden Reaktionszeit bei 0° wird die Lösung mit Aethylacetat (500 ml) verdünnt, mit Eiswasser und gesättigter Natriumchloridlösung gewaschen, über Natriumsulfat getrocknet und am Rotationsverdampfer eingeengt. Der Rückstand wird an Kieselgel mit Diäthyläther als Eluiermittel gereinigt, woraus der dünnschichtchromatographisch einheitliche 7β - {(2R) - 2 - BOC - Amino - 2 - [3 - ((2R) - 2 - BOC - amino - 2 - diphenylmethoxycarbonyläthoxycarbonylamino)phenyl]-acetylamino} - 3 - methoxy 3 - cephem - 4 - carbonsäure - diphenylmethylester als amorphes Produkt isoliert wird. DS (Fliessmittel: Aethylacetat): Rf = 0,60 Infrarotspektrum in Methylenchlorid: charakteristische Banden bei 3380, 1778, 1720, 1680, 1490 und 1160 $cm^{-1}$.

Beispiel 22

a) Eine auf 0°C gekühlte Lösung von 6,0 g (6,1 mMol) 7β - (2R,S) - 2 - Hydroxy - 2 - [3 - ((2R) - 2 - BOC - amino - 2 - diphenylmethoxycarbonyläthoxycarbonylamino)phenyl]-acetylamino} - 3 - carbamoyloxymethyl - 3 - cephem - 4 - carbonsäure - diphenylmethylester und 6 ml Anisol in 30 ml Methylenchlorid wird unter Ausschluss von Luftfeuchtigkeit und Rühren mit 30 ml Trifluoressigsäure versetzt. Nach 45 Minuten werden zur klaren Lösung bei 0° innert ca. 30 Minuten 300 ml Petroläther-Diäthyläther 1:1 gegeben. Das anfallende Trifluoracetat wird abfiltriert, mit Diäthyläther gewaschen, während 30 Minuten bei 10 Torr getrocknet, und in 20 ml Wasser teilweise

gelöst. Durch Zusatz von gesättigter wässriger Natriumbicarbonatlösung wird die Lösung auf pH 6 eingestellt und mit Aethylacetat (5 x 15 ml) extrahiert. Die leicht trübe wässrige Phase wird mit 2N Salzsäure auf pH 4 gebracht, über Celite klar filtriert und mit Isopropanol versetzt. Der Niederschlag wird abfiltriert, zur Entfernung von Isopropanol zweimal zusammen mit je 10 ml Wasser am Hochvakuum getrocknet und ergibt das 7$\beta$ - {(2R,S) - 2 - Hydroxy - 2 - [3 - ((2R) - 2 - amino - 2 - carboxyäthoxycarbonylamino)phenyl]acetylamino} - 3 - carbamoyloxymethyl - 3 - cephem - 4 - carbonsäure - natriumsalz - monohydrat als blassgelbes Pulver; F. ab 160° Zersetzung; $[\alpha]_D = 126 \pm 1°$ (c = 0,89, in $H_2O$); DS: $Rf_{96} = 0,14$.

Das Ausgangsmaterial kann wie folgt erhalten werden:

b) Eine Aufschlammung von 6,11 g (10,0 mMol) 7$\beta$ - 3 - carbamoyloxymethyl - 3 - cephem - 4 - carbonsäure - diphenylmethylester - p - toluolsulfosäuresalz in 100 ml absolutem Tetrahydrofuran wird unter Rühren und Feuchtigkeitsausschluss bei Raumtemperatur mit 1,1 ml (10 mMol) N-Methylmorpholin und nach ca. 5 Minuten bei 0° mit 2,8 g (12 mMol) 3-Nitro-(R,S)-mandelsäure-O-carboxyanhydrid gelöst in 20 ml absolutem Tetrahydrofuran versetzt. Nach 1 Stunde Reaktionszeit bei 0° und 16 Stunden bei Raumtemperatur wird das Lösungsmittel au Rotationsverdampfer entfernt. Der in Aethylacetat (500 ml) aufgenommene Rückstand wird mit 2 x 50 ml Eiswasser, gesättigter Natriumhydrogencarbonat- und Natriumchloridlösung gewaschen, über Natriumsulfat getrocknet und am Rotationsverdampfer eingeengt. Der rohe 7$\beta$ - [(2R,S) - 2 - Hydroxy - 2 - (3 - nitrophenyl)-acetylamino] - 3 - carbamoyloxymethyl - 3 - cephem - 4 - carbonsäurediphenylmethylester wird an Kieselgel mit Diäthyläther-Aethylacetat 9:1 als Eluiermittel gereinigt. DS (Fliessmittel: Diäthyläther-Aethylacetat 1:1) Rf = 0,63 und 0,74 (Epimerengemisch); Infrarotspektrum in Methylenchlorid: charakteristische Banden bei 3500, 3380, 1780, 1730, 1690, 1530, 1350 $cm^{-1}$.

c) Eine Lösung von 8g (13,3 mMol) 7$\beta$ - [(2R,S) - 2 - Hydroxy - 2 - (3 - nitrophenyl)-acetylamino] - 3 - carbamoyloxymethyl - 3 - cephem - 4 - carbonsäure - diphenylmethylester in 150 ml Aethylacetat wird in Gegenwart von 4g Palladium auf Aktivkohle (10%) hydriert. Nach Entfernung des Katalysators durch Filtration über Celit wird das Filtrat am Rotationsverdampfer eingeengt und das Rohprodukt an Kieselgel mit Methylenchlorid-Aethylacetat 1:1 als Eluiermittem gereinigt. Die dünnschichtchromatographisch einheitlichen Fraktionen enthaltend den 7$\beta$ - [(2R,S) - 2 - Hydroxy - 2 - (3 - aminophenyl)acetylamino) - 3 - carbamoyloxymethyl - 3 - cephem - 4 - carbonsäure - diphenylmethylester werden vereinigt und am Hochvakuum getrocknet. DS (Fliessmittel: Diäthyläther-Aethylacetat 9:1) Rf = 0,46 und 0,51 (Epimerengemisch).

d) Eine Lösung von 2,97 g (8 mMol) (2R)-N-BOC-Serin-diphenylmethylester und 0,68 ml Pyridin in 80 ml absolutem Methylenchlorid wird bei 0° unter Rühren und Feuchtigkeitsausschluss mit 4,2 ml 20%-igem Phosgen in Toluol versetzt und 1 Stunde bei 0° reagieren gelassen (Lösung A).

Eine Lösung von 4,7 g (8 mMol) 7$\beta$ - [(2R,S) - 2 - Hydroxy - 2 - (3 - aminophenyl)-acetylamino] - 3 - carbamoyloxymethyl - 3 - cephem - 4 - carbonsäurediphenylmethylester in 10 ml absolutem Methylenchlorid wird unter Feuchtigkeitsausschluss und Rühren bei Raumtemperatur mit 2,2 ml (8,8 mMol) Bis-(trimethylsilyl)-acetamid versetzt und eine Stunde gerührt (Lösung B).

Die auf 0° gekühlte Lösung B wird innert 5 Minuten zur Lösung A getropft und nach Zugabe von 0,95 ml (8,8 mMol) N-Methylmorpholin 30 Minuten bei 0° und 2 Stunden bei Raumtemperatur gerührt. Das mit 400 ml Aethylacetat verdünnte Reaktionsgemisch wird mit Eiswasser und gesättigter Natriumchloridlösung gewaschen, über Natriumsulfat getrocknet und am Rotationsverdampfer eingeengt. Der schaumige Rückstand wird an Kieselgel mit Diäthyläther als Eluiermittel gereinigt und ergibt den 7$\beta$ - {(2R,S) - 2 - Hydroxy - 2 - [3 - ((2R) - 2 - BOC - amino - 2 - diphenylmethoxycarbonyläthoxycarbonylamino)phenyl] - acetylamino} - 3 - carbamoyloxymethyl - 3 - cephem - 4 - carbonsäure - diphenylmethylester als amorphes Pulver.

DS (Fliessmittel: Diäthyläther): Rf = 0,29; Infrarotspektrum in Methylenchlorid; charakteristische Banden bei 3380, 1780, 1730, 1690, 1490, 1200, 1155 und 1070 $cm^{-1}$.

## Beispiel 23

a) Eine auf 0° C gekühlte Lösung von 4,9 g (5,2 mMol) 7$\beta$ - {(2R,S) - 2 - Hydroxy - 2 - [3 - ((2R) - 2 - BOC - amino - 2 - diphenylmethoxycarbonyläthoxycarbonylamino)phenyl] - acetylamino} - 3 - methoxy - 3 - cephem - 4 - carbonsäure - diphenylmethylester und 5 ml Anisol in 25 ml Methylenchlorid wird unter Ausschluss von Luftfeuchtigkeit und Rühren mit 25 ml Trifluoressigsäure versetzt. Nach 30 Minuten werden zur klaren Lösung 200 ml Petroläther-Diäthyläther 1:1 gegeben. Das ausfallende 7$\beta$ - {(2R,S) - 2 - Hydroxy - 2 - [3 - ((2R) - 2 - amino - 2 - carboxyäthoxycarbonylamino)phenyl]acetylamino} - 3 - methoxy - 3 - cephem - 4 - carbonsäure - trifluoracetat wird abfiltriert, mit Diäthyläther gewaschen, am Hochvakuum getrocknet, in 35 ml Wasser gelöst und mit Essigsäureäthylester (3 x 20 ml) extrahiert. Die wässrige Phase wird mit 1N Natronlauge auf pH 3 gestellt und zur Reinigung des Rohproduktes an einer Amberlite XAD-2 Säule mit Wasser-Isopropanol 9:1 als Eluiermittel chromatographiert. Man erhält nach Entfernung der Lösungsmittel am Rotationsverdampfer die 7$\beta$ - {(2R,S) - 2 - Hydroxy - 2 - [3 - ((2R) - 2 - amino - 2 - carboxyäthoxycarbonylamino)phenyl] - acetylamino} - 3 - methoxy - 3 - cephem - 4 - carbonsäure in Form des Monohydrates. F. ab 130° Zersetzung; $[\alpha]_D = 124 \pm 1°$ (c = 1,119, in 0,1 N HCl); DS: $Rf_{96} = 0,18$.

Das Ausgangsmaterial kann wie folgt erhalten werden:

b) Eine Lösung von 29,55 g (0,15 Mol) 3-Nitro-(R,S)-mandelsäure [Herstellung vgl. E. Adlarova, Collection Czech. Chem. Commun., *29*, 97 (1964)] in 300 ml absolutem Tetrahydrofuran wird mit 150 ml 20%-iger Phosgen-Lösung in Toluol versetzt und 3 Tage in einem verschlossenen Kolben bei 25° stehen gelassen. Nach der Entfernung des Ueberschusses Phosgen und der Lösungsmittel unter Feuchtigkeitsausschluss am Rotationsverdampfer wird das erhaltene 3-Nitro-(R,S)-mandelsäure-O-carboxyanhydrid am Hochvakuum getrocknet. Infrarotspektrum im Methylenchlorid: charakteristische Banden bei 1900, 1825, 1540, 1355, 1240, 1070 und 950 $cm^{-1}$.

c) Eine auf 0° gekühlte Aufschlämmung von 25,98 g (0,06 Mol) 7$\beta$ - Amino - 3 - methoxy - 3 - cephem - 4 - carbonsäurediphenylmethylester - hydrochlorid in 400 ml absolutem Tetrahydrofuran wird unter Stickstoff mit 6,66 ml (0,06 Mol) N-Methylmorpholin und nach 10 Minuten mit einer Lösung von 13,98 g (0,06 Mol) 3-Nitro-(RS)-mandelsäure-O-carboxyanhydrid in 150 ml Tetrahydrofuran versetzt. Nach einer Reaktionszeit von 3 Stunden bei Raumtemperatur wird das Lösungsmittel abdestilliert. Der Rückstand wird in Essigsäureäthylester aufgenommen mit Wasser (2 x 150 ml), verdünnter Natriumhydrogencarbonatlösung (2 x 150 ml) und gesättigter Natriumchloridlösung extrahiert. Die organische Phase wird über Natriumsulfat getrocknet und das Lösungsmittel am Rotationsverdampfer bei 30° abgedampft.

Der erhaltene rohe 7$\beta$ - [(2R,S) - 2 - Hydroxy - 2 - (3 - nitrophenyl) - acetylamino] - 3 - methoxy - 3 - cephem - 4 - carbonsäure - diphenylmethylester wird an Kieselgel mit Diäthyläther-Aethylacetat 1:1 als Eluiermittel gereinigt, DS (Fliessmittel: Diäthyläther-Aethylacetat 1:1): Rf = 0,54 und 0,65 (Epimerengemisch). Infrarotspektrum in Methylenchlorid: charakteristische Banden bei 3600, 1780, 1725, 1535, 1210 und 1050 $cm^{-1}$.

d) Eine Lösung von 26 g (0,045 Mol) 7$\beta$ - [(2R,S) - 2 - Hydroxy - 2 - (3 - nitrophenyl)-acetylamino] - 3 - methoxy - 3 - cephem - 4 - carbonsäure - diphenylmethylester in 500 ml Aethylacetat wird in Gegenwart von 13 g Palladium auf Aktivkohle (10%) hydriert. Nach Entfernung des Katalysators durch Filtration über Celite wird das Filtrat am Rotationsverdampfer eingeengt und das Rohprodukt an Kieselgel mit Diäthyläther-Aethylacetat als Eluiermittel gereinigt. Die dünnschichtchromatographisch einheitlichen Fraktionen an 7$\beta$ - [(2R,S) - 2 - Hydroxy - 2 - (3 - aminophenyl)-acetylamino] - 3 - methoxy - 3 - cephem - 4 - carbonsäure - diphenylmethylester werden vereinigt und am Hochvakuum getrocknet. DS (Fliessmittel: Essigester): Rf = 0,46; Infrarotspektrum in Methylenchlorid: charakteristische Banden bei 3380, 1780, 1720 und 1220 $cm^{-1}$.

e) Eine Lösung von 4,45 g (12 mMol) (2R)-N-BOC-Serin-diphenylmethylester und 1,0 ml Pyridin in 100 ml absolutem Methylenchlorid wird bei 0° unter Rühren mit 6,2 ml 20%-igem Phosgen in Toluol versetzt und ein Stunde bei 0° weiter gerührt (Lösung A).

Eine Lösung von 6,55 g (12 mMol) 7$\beta$ - [(2R,S) - 2 - Hydroxy - 2 - (3 - aminophenyl)-acetylamino] - 3 - methoxy - 3 - cephem - 4 - carbonsäure - diphenylmethylester in 100 ml absolutem Methylenchlorid wird unter Feuchtigkeitsausschluss und Rühren bei Raumtemperatur mit 3,3 ml Bis-(trimethylsilyl-)acetamid versetzt und eine Stunde gerührt (Lösung B).

Bei 0° wird die Lösung B innerhalb 5 Minuten zur Lösung A getropft, anschliessend mit 1,3 ml (12 mMol) N-Methylmorpholin versetzt und 30 Minuten bei 0° und 1 Stunde bei 25° gerührt.

Das mit Aethylacetat (600 ml) verdünnte Reaktionsgemisch wird mit je 3 x 150 ml Eiswasser, verdünnter Salzsäure und gesättigter Natriumchloridlösung extrahiert. Die organische Phase wird über Natriumsulfat getrocknet und am Rotationsverdampfer vom Lösungsmittel befreit. Das erhaltene Rohprodukt wird an Kieselgel mit Diäthyläther-Aethylacetat 9:1 als Eluiermittel gereinigt. Man erhält den dünnschichtchromatographisch einheitlichen 7$\beta$ - {(2R,S) - 2 - Hydroxy - 2 - [3 - ((2R) - 2 - BOC - amino - 2 - diphenylmethoxycarbonyläthoxycarbonylamino)phenyl]acetylamino} - 3 - methoxy - 3 - cephem - 4 - carbonsäure - diphenylmethylester. DS (Fliessmittel: Diäthyläther-Aethylacetat 1:1) Rf = 0,44; Infrarotspektrum in Methylenchlorid: charakteristische Banden bei 3400, 1780, 1735, 1720, 1600, 1495, 1205, 1160 $cm^{-1}$.

## Beispiel 24

a) Eine Lösung von 90 mg 7$\beta$ - [5 - ((2R) - 2 - BOC - Amino - 2 - diphenylmethoxycarbonyläthoxy - carbonylaminomethyl) - thien - 2 - ylacetamido] - 7$\alpha$ - methoxy - 3 - (1 - methyl - 1H - tetrazol - 5 - ylthiomethyl) - 3 - cephem - 4 - carbonsäure in 0,5 ml Methylenchlorid wird bei 0° mit 0,5 ml Trifluoressigsäure versetzt und die Lösung 45 Minuten bei 0° gerührt. Das Rohprodukt wird mit 5 ml Diäthyläther-Hexan 1:1 als Trifluoressigsäure-Salz ausgefällt und dieses mit Methanol-Pyridin 9:1 digeriert. Man erhält so die 7$\beta$ - [5 - ((2R) - 2 - Amino - 2 - carboxy - äthoxycarbonylaminomethyl) - thien - 2 - yl - acetamido] - 7$\alpha$ - methoxy - 3 - (1 - methyl - 1H - 1,2,3,4 - tetrazol - 5 - ylthiomethyl) - 3 - cephem - 4 - carbonsäure. DS: $Rf_{52A}$ = 0,24 (Laufstrecke unter Zwischentrocknen: 3 mal 10 cm).

Die Ausgangsverbindung kann wie folgt hergestellt werden:

b) Eine Lösung von 445 mg (R)-N-BOC-Serin-diphenylmethylester und 95 mg Pyridin in 3 ml Methylenchlorid wird bei 0° bis 5° zu 0,6 ml einer 20-prozentigen Lösung von Phosgen in Toluol getropft und anschliessend 1 Stunde bei dieser Temperatur gerührt. Das Gemisch wird im Vakuum eingedampft, der Rückstand in 3 ml Tetrahydrofuran gelöst und diese Lösung bei 0° bis 5° zu einer mittels

Titrator, (0,1-normale Natronlauge) auf pH 9 eingestellten Lösung von 510 mg 7β - (5 - Aminomethyl - thien - 2 - yl - acetamido) - 7α - methoxy - 3 - (1 - methyl - 1H - tetrazol - 5 - yl - thiomethyl) - 3 - cephem - 4 - carbonsäure in 4 ml Wasser und 2 ml Tetrahydrofuran getropft. Man rührt bei pH 9 (durch Titrator konstant gehalten) 45 Minuten bei 0° bis 5° sowie 30 Minuten bei 20°. Das Gemisch wird zu 50 ml 0,5-molarer Kaliumdihydrogenphosphat-Lösung gegeben, das pH auf einen Wart von 6,1 gestellt und im Vakuum die organischen Lösungsmittel abgedampft. Die verbliebene wässrige Phase wird 3 mal mit je 50 ml Essigsäureäthylester extrahiert. Der über Natriumsulfat getrocknete Auszug wird eingedampft und der Rückstand mit Diäthyläther digeriert. Man erhält ein Rohprodukt, das an 10 g Kieselgel chromatographier wird. Die mit Aceton-Methanol 1:1 eluierten Fraktionen enthalten die reine 7β - [5 - ((2R) - 2 - BOC - Amino - 2 - diphenylmethoxycarbonyläthoxycarbonylaminomethyl) - thien - 2 - ylacetamido] - 7α - methoxy - 3 - (1 - methyl - 1H - tetrazol - 5 - ylthiomethyl) - 3 - cephem - 4 - carbonsäure. DS: $Rf_{52A} = 0,45$.

Beispiel 25

Zu einer 70° warmen Lösung von 16,3 g Natriumjodid, 1,62 g (13,3 mMol) iso-Nicotinsäureamid und 0,59 ml (10,3 mMol) Eisessig in 7,7 ml Wasser gibt man 5,29 g (9:9 mMol) 7β - [4 - ((2R) - 2 - Amino - 2 - carboxy - äthoxycarbonylamino) - phenylacetamido] - cephalosporansäure-natriumsalz und rührt $1\frac{1}{2}$ Stunden bei 70° unter Stickstoffatmosphäre. Die warme Lösung wird zu 200 ml kaltem Aceton getropft. und die Ausfällung nach 30 Minuten bei —20° abfiltriert. Der Rückstand wird 2 mal mit je 70 ml Methanol digeriert und der nicht lösliche Teil abfiltriert. Eine Lösung dieses Rohprodukts in 17 ml Wasser wird mit 160 mg Aktiv-Kohle entfärbt, klarfiltriert und das Filtrat unter Rühren zu 870 ml Aethanol getropft. Nach 45 Minuten Rühren bei 0° wird das 7β - [4 - (2R) - 2 - Amino - 2 - carboxyäthoxycarbonylamino) - phenylacetamido] - 3 - (4 - carbamoyl - pyridiniomethyl) - 3 - cephem - 4 - carboxylat (Betain) abfiltriert. DS: $Rf_{101} = 0,18$.

Beispiel 26

a) Ein Gemisch von 8,0 g 7β - {2 - [4 - ((4R) - 4 - BOC - Amino - 4 - t.butoxycarbonylbutyrylamino)phenyl]acetylamino} - 3 - [(1 - methyl - 1H - tetrazol - 5 - yl) - thiomethyl] - 3 - cephem - 4 - carbonsäure - diphenylmethylester, 3,0 ml Anisol 8,0 ml Methylenchlorid und 80 ml Trifluoressigsäure wird 35 Minuten bei Raumtemperatur unter Ausschluss von Luftfeuchtigkeit gerührt. Die erhaltene Suspension wird auf ein eiskaltes Gemisch von Petroläther (1 Liter) und Diäthyläther (500 ml) gegossen, das anfallende Trifluoracetat abgenutscht, mit Diäthyläther gewaschen und bei Raumtemperatur im Hockvakuum getrocknet.

Das 7β - {2 - [4 - ((4R) - 4 - Amino - 4 - carboxybutylrylamino) - phenyl]acetylamino} - 3 - [(1 - methyl - 1H - tetrazol - 5 - yl) - thiomethyl] - 3 - cephem - 4 - carbonsäure-natriumsalz wird aus dem Trifluoracetat nach dam Verfahren von Beispiel 1 erhalten. F. ab 200° (Zersetzung). DS: $Rf_{52A} = 0,06$, $Rf_{101} = 0,28$, $Rf_{101A} = 0,28$.

Das Ausgangsmaterial kann wie folgt hergestellt werden:

b) Ein Gemisch von 10,5 g (2R) - Glutaminsäure - γ - benzylester (hergestellt nach dem Verfahren von R.L. Prestidge et al, J.O.C. *40*, 3287 (1975)), 90 ml Dioxan, 30 ml Wasser und 13 ml Di-tert.-butyl-dicarbonat wird bei Raumtemperatur gerührt und der pH-Wert des Reaktionsgemisches durch Zutropfen von 1N Natronlauge zwischen 7 und 7,6 gehalten. Nach 2 Stunden wird die klare Lösung am Rotationsverdampfer bei 50° auf ca. 50 ml eingeengt und mit Aethylacetat ausgezogen. Die wässrige Phase wird abgetrennt mit 20%-iger Zitronensäurelösung angesäuert (pH 3) und dreimal mit Aethylacetat ausgezogen. Die organischen Extrakte werden vereinigt, einmal mit Solelösung gewaschen, über Natriumsulfat getrocknet und das Lösungsmittel am Rotationsverdampfer bei 45° abgedampft. Beim Vermischen mit Petroläther und Kühlen wird der (2R)-N-BOC-Glutaminosäure-γ-benzylester fest. F. 50—58° (Zersetzung). $[\alpha]_D = -13 \pm 1°$ (c = 3,11 in Chloroform).

Der ölige (2R)-N-BOC-Glutaminsäure-(γ-benzylester)-α-tert.-butylester wird aus (2R)-N-BOC-Glutaminsäure-γ-benzylester durch Umsetzung mit O-tert.-Butylisoharnstoff in Methylenchlorid (E. Vowinkel, Chem. Ber. *100*, 16 (1967)) gewonnen. Das Rohprodukt wird durch Chromatographie an der 20-fachen Menge Kieselgel gereinigt. DS (Fliessmittel: Toluol-Aethylacetat-Chloroform-(3:1:1)): Rf = 0,58 (mit Ninhydrin entwickelt); $[\alpha]_D = 8 \pm 1°$ (c = 3,17 in Chloroform).

c) Eine Lösung von 28 g (2R)-N-BOC-Glutaminsäure-(γ-benzylester)-α-tert.-butylester in 250 ml Aethylacetat wird in Gegenwart von 4,0 g Palladium auf Aktivkohle (10%) hydriert. Nach ca. 4 Stunden ist die berechnete Wasserstoffmenge aufgenommen. Der Katalysator wird abfiltriert, mit Aethylacetat gewaschen, das Filtrat am Rotationsverdampfer bei 45° eingeengt und der (2R)-N-BOC-Glutaminsäure-α-tert.-butylester durch Zugabe von Petroläther kristallisiert. F. 109—112° (Zersetzung). $[\alpha]_D = -5 \pm 1°$ (c = 2,66 in Chloroform).

d) Eine Lösung von 9,0 g (2R)-N-BOC-Glutaminsäure-α-tert.-butylester und 7,50 g 4-Aminophenylessigsäurebenzylester in 80 ml Tetrahydrofuran wird mit einem Eisbad auf +5° abgekühlt. eine Lösung von 6,50 g N,N'-Di-cyclohexylcarbodiimid in 20 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird das Reaktionsgemisch mit 3,0 g festem N,N'-Dicyclohexylcarbodiimid versetzt. Nach insgesamt 7 Stunden Rühren wird die Suspension abgenutscht,

das Nutschgut mit Aethylacetat gewaschen, das Filtrat mit viel Aethylacetat versetzt und das Tetrahydrofuran durch Einengen am Rotationsverdampfer bei 45° entfernt. Die erhaltene Lösung wird mit Aethylacetat verdünnt und sukzessive mit Wasser, 1N Natriumbicarbonatlösung und Wasser gewaschen. Die organische Phase wird über Natriumsulfat getrocknet und am Rotationsverdampfer bei 45° eingedampft. Das Rohprodukt wird durch Filtrieren an der 10-fachen Menge Kieselgel gereinigt. Die Fraktionen mit Methylenchlorid-Methylacetat(10:0,5) als Eluiermittel werden vereinigt. Der erhaltene 4-((4R)-4-BOC-Amino-4-t.-butoxycarbonylbutyrylamino)phenylessigsäure-benzylester wird aus einem Gemisch von Aethylacetat-Petroläther umkristallisiert. F. 101—104° (Zersetzung).

e) Eine Lösung von 20,0 g 4-((4R)-4-BOC-Amino-4-t.butoxycarbonylbutyrylamino)phenylessigsäure-benzylester in einem Gemisch von Aethylacetat (150 ml) und Aethanol (150 ml) wird in Gegenwart von 5,0 g Palladium auf Aktivkohle (10%) bei Raumtemperatur hydriert. Nach ca. 5 Stunden ist die berechnete Wasserstoffmenge aufgenommen. Der Katalysator wird abfiltriert, mit einem Gemisch von Aethylacetat-Aethanol-(1:1) gewaschen und das Filtrat am Rotationsverdampfer bei 45° eingedampft. Die 4-((4R)-4-BOC-Amino-4-t.butoxycarbonylbutyrylamino)-phenylessigsäure wird durch Vermischen mit Petroläther gefällt. F. 129—136° (Zersetzung); $[\alpha]_D = +10\pm1°$ (c = 3,04 in Methanol).

f) Eine Lösung von 9,5 g 7β-Amino-3-[(1-methyl-1H-tetrazol-5-yl)-thiomethyl]-3-cephem-4-carbonsäurediphenylmethylester und 8,0 g 4-((4R)-4-BOC-Amino-4-t.butoxycarbonylbutyrylamino)-phenylessigsäure in einem Gemisch von 50 ml Tetrahydrofuran und 15 ml N,N-Dimethylformamid wird mit einer Lösung von 4,0 g N,N'-Dicyclohexylcarbodiimid in 20 ml Tetrahydrofuran bzw. 2,0 festem N,N'-Dicyclohexylcarbodiimid nach dem Verfahren von Beispiel 1 umgesetzt und das Reaktionsgemisch aufgearbeitet. Beim Einengen der Aethylacetatlösung fällt der kristalline 7β - {2 - [4 - ((4R) - 4 - BOC - Amino - 4 - t. - butoxycarbonylbutyrylamino)phenyl]acetamido} - 3 - [(1 - methyl - 1H - tetrazol - 5 - yl) - thiomethyl] - 3 - cephem - 4 - carbonsäure - diphenylmethylester aus. F. 172—178° (Zersetzung). DS (Fliessmittel: Toluol-Aethylacetat-Methylenchlorid-Aethanol-(16:16:16:1)). Rf = 0,32.

## Beispiel 27

Analog den vorstehenden Beispielen können ausgehend von den entsprechenden Verbindungen, worin die Carboxylgruppen, die Aminogruppen und gegebenenfalls die Hydroxylgruppen in geschützter Form vorliegen, die folgender Verbindungen hergestellt werden:

7β - {2 - [4 - ((4R) - 4 - Amino - 4 - carboxybutyrylamino)phenyl]acetylamino} - 3 - carbamoyloxymethyl - 3 - cephem - 4 - carbonsäure;

7β - {(R,S) - 2 - [4 - ((2R) - 2 - Amino - 2 - carboxyäthoxycarbonylamino)phenyl] - 2 - formyloxyacetylamino} - 3 - [(1 - methyl - 1H - tetrazol - 5 - yl) - thiomethyl] - 3 - cephem - 4 - carbonsäure;

7β - {(2R,S) - 2 - [4 - ((2R) - 2 - Amino - 2 - carboxyäthoxycarbonylamino)phenyl] - 2 - formyloxyacetylamino} - 3 - carbamoyloxymethyl - 3 - cephem - 4 - carbonsäure;

7β - {2 - [4 - ((2R) - 2 - Amino - 2 - carboxyäthoxycarbonylamino)phenyl] - 2 - syn - methoxyimino - acetylamino} - 3 - [(1 - methyl - 1H - tetrazol - 5 - yl) - thiomethyl] - 3 - cephem - 4 - carbonsäure;

7β - {2 - [4 - ((2R) - 2 - Amino - 2 - carboxyäthoxycarbonylamino)phenyl] - 2 - syn - methoxyimino - acetylamino} - 3 - carbamoyloxy - methyl - 3 - cephem - 4 - carbonsäure;

7β - {2 - [4 - ((2R) - 2 - Amino - 2 - carboxyäthoxycarbonylamino)phenyl] - acetylamino} - 3 - [1 - (2 - dimethylaminoäthyl) - 1H - tetrazol - 5 - yl] - thiomethyl] - 3 - cephem - 4 - carbonsäure;

7β - {2 - [4 - ((2R) - 2 - Amino - 2 - carboxyäthoxycarbonylamino)phenyl] - acetylamino} - 3 - [(1 - carboxymethyl - 1H - tetrazol - 5 - yl) - thiomethyl] - 3 - cephem - 4 - carbonsäure;

7β - {2 - [4 - ((2R) - 2 - Amino - 2 - carboxyäthoxycarbonylamino)phenyl]acetylamino} - 3 - [1 - sulfomethyl - 1H - tetrazol - 5 - yl) - thiomethyl] - 3 - cephem - 4 - carbonsäure;

7β - {(2R,S) - 2 - [4 - ((2R) - 2 - Amino - 2 - carboxyäthoxycarbonylamino)phenyl] - 2 - hydroxyacetylamino} - 3 - [(1 - carboxymethyl - 1H - tetrazol - 5 - yl) - thiomethyl] - 3 - cephem - 4 - carbonsäure;

7α - Methoxy - 7β - {2 - [4 - ((2R) - 2 - amino - 2 - carboxyäthoxycarbonylamino)phenyl]acetylamino} - 3 - acetoxymethyl - 3 - cephem - 4 - carbonsäure;

7α - Methoxy - 7β - {2 - [4 - ((2R) - 2 - amino - 2 - carboxyäthoxycarbonylamino)phenyl] - acetylamino} - 3 - carbamoyloxymethyl - 3 - cephem - 4 - carbonsäure;

7α - Methoxy - 7β - {(2R,S) - 2 - [4 - ((2R) - 2 - amino - 2 - carboxyäthoxycarbonylamino)phenyl] - 2 - hydroxy - acetylamino} - 3 - carbamoyloxymethyl - 3 - cephem - 4 - carbonsäure;

7α - Methoxy - 7β - {(2R,S) - 2 - [4 - ((2R) - 2 - amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure; sowie die entsprechenden (4S) 4 - Amino - 4 - carboxybutyrylamino-, (2S) - 2 - Amino - 2 - carboxyäthoxycarbonylamino, und (2R) - 2 - Hydroxyacetylamino-, (2S) - 2 - Hydroxyacetylamino-; (2R) - 2 - Formyloxyacetylamino- und (2S) - 2 - Formyloxyacetylamino - verbindungen, und entsprechende Salze, z.B. die Natriumsalze, davon.

Beispiel 28

Trockenampullen oder Vials, enthaltend 0,5 g 7α - {2 - [4 - ((2R) - 2 - Amino - 2 - carboxyäthoxycarbonylamino)phenyl]acetylamino} - 3 - [(1 - methyl - 1H - tetrazol - 5 - yl) - thiomethyl] - 3 - cephem - 4 - carbonsäure-natriumsalz werden wie folgt hergestellt: 7β - 2 - [4 - ((2R) - 2 - Amino - 2 - carboxyäthoxycarbonylamino)phenyl]acetylamino - 3 - [(1 - methyl - 1H - tetrazol - 5 - yl) - thiomethyl] - 3 - cephem - 4 - carbonsäure - natriumsalze 0,5 g Mannit 0,05 g

Eine sterile wässrige Lösung von 7β - {2 - [4 - ((2R) - 2 - Amino - 2 - carboxyäthoxycarbonylamino)phenyl]acetylamino - 3 - [(1 - methyl - 1H - tetrazol - 5 - yl) - thiomethyl] - 3 - cephem - 4 - carbonsäure - natriumsalze und des Mannits wird unter aseptischen Bedingungen in 5 ml.-Ampullen oder 5 ml.-Vials verschlossen und geprüft.

Beispiel 29

Trockenampullen oder Vials, enthaltend 0,5 g 7β - {(2R) - 2 - [4 - ((2R) - 2 - amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäurenatriumsalz werden wie folgt hergestellt: *Zusammensetzung* (für 1 Ampulle oder Vial) 7β - {(2R) - 2 - [4 - ((2R) - 2 - amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäurenatriumsalz 0,5 g Mannit 0,05 g

Eine sterile wässrige Lösung des 7β - {(2R) - 2 - [4 - ((2R) - 2 - amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäurenatriumsalzes und des Mannits wird unter aseptischen Bedingungen in 5 ml.-Ampullen oder 5 ml.-Vials verschlossen und geprüft.

## Patentansprüche

1. Acylamido-3-cephem-4-carbonsäure-Verbindungen der Formel

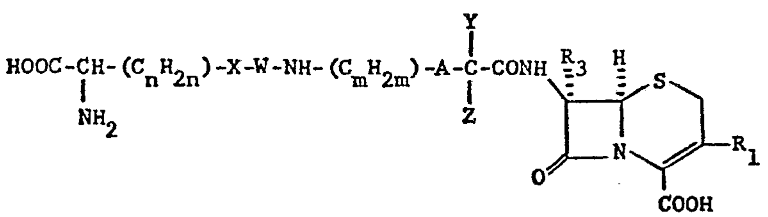

(I)

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2$NH—CO—, oder X—W zusammen eine Gruppe —CO— oder —CO—$NHSO_2$—, A gegebenenfalls durch Niederalkyl, Hydroxy, Niederalkoxy und/oder Halogen substituiertes Phenylen, gegebenenfalls durch Niederalkyl, Niederalkoxy und/oder Halogen substituiertes Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—$R^o$, worin $R^o$ Wasserstoff oder gegebenenfalls durch Niederalkoxy, Halogen, Hydroxy, acyliertes Hydroxy, Sulfo, Carboxy oder durch Niederalkyl verestertes Carboxy substituiertes Niederalkyl darstellt, $R_1$ Wasserstoff, $C_1$—$C_4$-Niederalkyl, $C_1$—$C_4$-Niederalkoxy, Halogen oder eine Gruppe der Formel —$CH_2$—$R_2$, worin $R_2$ eine Hydroxygruppe, eine durch eine niederaliphatische Carbonsäure, die Carbaminsäure oder eine am N-Atom durch Niederalkyl, Halogenniederalkyl oder Niederalkanoylniederalkyl substituierte Carbaminsäure veresterte Hydroxy- oder Mercaptogruppe, Niederalkoxy, Niederalkylthio oder eine durch einen über ein Ringkohlenstoffatom an die Mercaptogruppe gebundenen heterocyclischen Rest mit 1—4 Ringstickstoffatomen und gegebenenfalls einem weiteren Ringheteroatom der Gruppe Sauerstoff und Schwefel, der durch Niederalkyl, Hydroxyniederalkyl, Niederalkanoyloxyniederalkyl, Halogenniederalkyl, Carboxyniederalkyl, Niederalkoxycarbonylniederalkyl, Sulfoniederalkyl, amidiertes Sulfoniederalkyl, Aminoniederalkyl, Mono- oder Diniederalkylaminoniederalkyl, Acylaminoniederalkyl, Cycloalkyl, Aryl, Arylniederalkyl, Heterocyclyl, Halogen, Amino, Mono- oder Diniederalkylamino, Niederalkanoylamino, Halogenniederalkanoylamino, Carboxyniederalkanoylamino, Nitro, Hydroxy, Niederalkoxy, Carboxy, Niederalkoxycarbonyl, Carbamoyl, N-mono- oder N,N-diniederalkyliertes Carbamoyl, Cyan, Oxo oder Oxido ein- oder mehrfach substituiert sein kann, verätherte Mercaptogruppe oder eine quaternäre Ammoniumgruppe darstellt, wobei unter niederen Resten, sofern nicht ausdrücklich anders definiert, solche mit 1 bis 7 C-Atomen zu verstehen sind, und $R_3$ Wasserstoff oder Methoxy bedeuten, worin die Carboxylgruppen gegebenenfalls in physiologisch spaltbarer Form verestert sind, und ihre Salze.

2. Verbindungen der Formel (I) nach Patentanspruch 1, worin die Gruppe —$(C_nH_{2n})$— unverzweigt ist und die Indices n und m die angegebene Bedeutung haben, X Sauerstoff oder die Gruppe

—NH—, W eine Gruppe —CO— oder —CO—NHSO$_2$—, oder X—W zusammen eine Gruppe —CO— oder —CO—NHSO$_2$— bedeuten, A p- oder m-Phenylen, 2,5-Thienylen oder 2,5-Furylen darstellt, Y Wasserstoff, Hydroxyl, Amino oder Sulfo und Z Wasserstoff, oder Y und Z zusammen eine Gruppe =N—O—R° bedeuten, worin R° Wasserstoff oder Methyl ist, $R_1$ Niederalkyl, Niederalkoxy, oder eine Gruppe der Formel —$CH_2$—$R_2$ bedeutet, worin $R_2$ Niederalkanoyloxy, Carbamoyloxy, N-Niederalkylcarbamoyloxy, Triazolylthio, Tetrazolylthio, Thiazolylthio, Thiatriazolylthio, Thiadiazolylthio, Oxazolylthio, Oxadiazolylthio oder Pyridinio darstellt, worin die heterocyclischen Ringe gegebenenfalls durch Niederalkyl, N,N-Diniederalkylaminoniederalkyl, Carboxyniederalkyl, Sulfoniederalkyl, Amino, Carboxyniederalkanoylamino oder Carbamoyl substituiert sein können, und $R_3$ Wasserstoff oder Methoxy darstellen, und ihre Salze.

3. Verbindungen der Formel (I), nach Patentanspruch 1 oder 2, worin die Gruppe —($C_2H_{2n}$)— unverzweigt ist und die Indices n und m die angegebene Bedeutung haben, X Sauerstoff oder die Gruppe —NH— bedeutet, W eine Gruppe —CO— oder —CONHSO$_2$—, oder X—W zusammen eine Gruppe —CO— oder —CONHSO$_2$— bedeuten, A p- oder m-Phenylen, oder, wenn m 1 ist, auch 2,5-Thienylenoder 2,5-Furylen darstellt, Y Wasserstoff, Hydroxyl, Amino oder Sulfo und Z Wasserstoff, oder Y und Z zusammen eine Gruppe =N—O—R° bedeuten, worin R° Wasserstoff oder Methyl ist, $R_1$ Methyl, Methoxy, oder eine Gruppe der Formel —$CH_2$—$R_2$ bedeutet, worin $R_2$ Acetoxy, Carbamoyloxy, 1-Methyl-1H-tetrazol-5-ylthio, 1-Sulfomethyl-1H-tetrazol-5-ylthio, 1-Carboxymethyl-1H-tetrazolylthio, 1-(2-Dimethylaminoäthyl)-1H-tetrazol-5-ylthio, 2-Methyl-1,3,4-thiadiazol-5-ylthio, oder 4-Carbamoylpyridinio, darstellt, und $R_3$ Wasserstoff oder Methoxy bedeutet, und ihre Salze.

4. 7β - {2 - [4 - (2R) - 2 - Amino - 2 - carboxyäthoxycarbonylamino)phenyl]acetylamino} - 3 - [(1 - methyl - 1H - tetrazol - 5 - yl)thiomethyl] - 3 - cephem - 4 - carbonsäure,

7β - {2 - [4 - ((2S) - 2 - Amino - 2 - carboxyäthoxycarbonylamino)phenyl]acetylamino} - 3 - [(1 - methyl - 1H - tetrazol - 5 - yl)thiomethyl] - 3 - cephem - 4 - carbonsäure,

7β - {2 - [4 - ((2R) - 2 - Amino - 2 - carboxyäthoxycarbonylamino)phenyl]acetylamino} - 3 - methyl - 3 - cephem - 4 - carbonsäure,

7β - {2 - [4 - ((2R) - 2 - Amino - 2 - carboxyäthoxycarbonylamino)phenyl]acetylamino} - cephalosporansäure,

7β - {2 - [4 - ((5R,S) - 5 - Amino - 5 - carboxypentylaminocarbonylamino)phenyl]acetylamino} - cephalosporansäure,

7β - {2 - [4 - ((3R) - 3 - Amino - 3 - carboxypropionylamino)phenyl]acetylamino} - 3 - [(1 - methyl - 1H - tetrazol - 5 - yl) - thiomethyl] - 3 - cephem - 4 - carbonsäure,

7β - {2 - [4 - ((3R) - 3 - Amino - 3 - carboxypropionylamino)phenyl]acetylamino} - 3 - carbamoyloxymethyl - 3 - cephem - 4 - carbonsäure,

7β - {2 - [5 - ((2R) - 2 - Amino - 2 - carboxyäthoxycarbonylaminomethyl) - 2 - furyl] - 2 - syn - methoximinoacetylamino} - 3 - [(1 - methyl - 1H - tetrazol - 5 - yl) - thiomethyl] - 3 - cephem - 4 - carbonsäure,

7β - {2 - [5 - ((2R) - 2 - Amino - 2 - carboxyäthoxycarbonylaminomethyl) - 2 - furyl] - 2 - syn - methoximinoacetylamino} - 3 - carbamoyloxymethyl - 3 - cephem - 4 - carbonsäure,

7β - {2 - [5 - ((2R) - 2 - Amino - 2 - carboxyäthoxycarbonylaminomethyl) - 2 - thienyl] - 2 - syn - methoximinoacetylamino} - 3 - acetoxymethyl - 3 - cephem - 4 - carbonsäure,

7β - {2 - [4 - ((2R) - 2 - Amino - 2 - carboxyäthoxycarbonylaminosulfonylamino)phenyl]acetyl - amino} - 3 - carbamoyloxymethyl - 3 - cephem - 4 - carbonsäure,

7β - {(2R,S) - 2 - [4 - ((2R) - 2 - Amino - 2 - carboxyäthoxycarbonylamino)phenyl] - 2 - hydroxy - acetylamino} - 3 - carbamoyloxymethyl - 3 - cephem - 4 - carbonsäure,

7β - {(2R,S) - 2 - [4 - ((2R) - Amino - 2 - carboxyäthoxycarbonylamino)phenyl] - 2 - hydroxy - acetylamino} - 3 - [(1 - methyl - 1H - tetrazol - 5 - yl) - thiomethyl] - 3 - cephem - 4 - carbonsäure,

7β - [4 - (2R) - 2 - Amino - 2 - carboxyäthoxycarbonylamino)phenylacetamido] - 3 - (4 - carbamoyl - pyridiniomethyl) - 3 - cephem - 4 - carboxylat, und

7β - {2 - [4 - ((4R) - 4 - Amino - 4 - carboxybutyrylamino)phenyl]acetylamino} - 3 - [(1 - methyl - 1H - tetrazol - 5 - yl) - thiomethyl] - 3 - cephem - 4 - carbonsäure,

7α - Methoxy - 7β - {2 - [4 - ((2R) - 2 - amino - 2 - carboxyäthoxycarbonylamino)phenyl]acetyl - amino} - 3 - [(1 - methyl - 1H - tetrazol - 5 - yl) - thiomethyl] - 3 - cephem - 4 - carbonsäure,

7β - {(2R,S) - 2 - [4 - ((2R) - 2 - Amino - 2 - carboxyäthoxycarbonylamino)phenyl] - 2 - sulfo - acetylamino} - 3 - [(1 - methyl - 1H - tetrazol - 5 - yl) - thiomethyl] - 3 - cephem - 4 - carbonsäure,

7β - {(2R) - 2 - Amino - 2 - [3 - (2R) - 2 - amino - 2 - carboxyäthoxycarbonylamino)phenyl]acetyl - amino} - 3 - methoxy - 3 - cephem - 4 - carbonsäure,

7β - {(2R,S) - 2 - Hydroxy - 2 - [3 - ((2R) - 2 - amino - 2 - carboxyäthoxycarbonylamino)phenyl] - acetylamino} - 3 - carbamoyloxymethyl - 3 - cephem - 4 - carbonsäure,

7β - {(2R,S) - 2 - Hydroxy - 2 - [3 - ((2R) - 2 - amino - 2 - carboxyäthoxycarbonylamino)phenyl] - acetylamino} - 3 - methoxy - 3 - cephem - 4 - carbonsäure,

7β - [5 - ((2R) - 2 - Amino - 2 - carboxy - äthoxycarbonylaminomethyl) - thien - 2 - yl - acet - amido] - 7α - methoxy - 3 - (1 - methyl - 1H - tetrazol - 5 - ylthiomethyl) - 3 - cephem - 4 - carbon - säure, und pharmazeutisch annehmbare Salze davon, nach einem der Patentansprüche 1 bis 3.

5. 7β - {2 - [4 - ((2R) - 2 - Amino - 2 - carboxyäthoxycarbonylamino)phenyl]acetylamino} - 3 -

carbamoyloxymethyl - 3 - cephem - 4 - carbonsäure und pharmazeutisch annehmbare Salze davon, nach einem der Patentansprüche 1 bis 3.

6. 7β - {(2R) - 2 - [4 - ((2R) - 2 - Amino - 2 - carboxyäthoxycarbonylamino)phenyl] - 2 - hydroxy - acetylamino} - 3 - [(1 - methyl - 1H - tetrazol - 5 - yl) - thiomethyl] - 3 - cephem - 4 - carbonsäure und pharmazeutisch annehmbare Salze davon, nach einem der Patentansprüche 1 bis 3.

7. 7β - {(2R) - 2 - [4 - ((2R) - 2 - Amino - 2 - carboxyäthoxycarbonylamino)phenyl] - 2 - hydroxy - acetylamino} - 3 - carbamoyloxymethyl - 3 - cephem - 4 - carbonsäure und pharmazeutisch annehm - bare Salze davon, nach einem der Patentansprüche 1 bis 3.

8. Die Natriumsalze der in einem der Patentansprüche 1 bis 7 genannten Verbindungen.

9. Pharmazeutisch Präparate enthaltende eine der in einem der Patentansprüche 1 bis 8 beanspruchten Verbindungen.

10. Stoffe und Stoffgemische nach einem der Patentansprüche 1 bis 9 zur Verwendung in der Behandlung von durch gram-positive und gram-negative Bakterien oder Kokken verursachten Infektionen.

11. Acylamido-3-cephem-4-carbonsäure-Verbindungen der im Patentanspruch 1 definierten Formel I, worin mindestens eine der funktionellen Gruppen in geschützter Form vorliegt, sowie deren Salze.

12. Verfahren zur Herstellung von Acylamido-3-cephem-4-carbonsäure-Verbindungen der im Patentanspruch 1 definierten Formel I, worin die Carboxylgruppen gegebenenfalls in physiologisch spaltbarer Form verestert sind, und ihren Salzen, dadurch gekennzeichnet, dass man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung eine Gruppe $R_1$ in eine andere Gruppe $R_1$ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

13. Verfahren zur Herstellung von Acylamino-3-cephem-4-carbonsäure-Verbindungen der im Patentanspruch 1 definierten Formel I, worin mindestens eine der funktionellen Gruppen in geschützter Form vorliegt, dadurch gekennzeichnet, dass man

a) in einer Verbindung der Formel

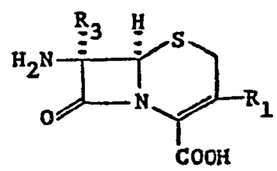

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einer Säure der Formel

$$HOOC-\underset{\displaystyle NH_2}{\underset{|}{CH}}-(C_nH_{2n})-X-W-NH-(C_mH_{2m})-A-\underset{\displaystyle Z}{\underset{|}{\overset{\displaystyle Y}{\overset{|}{C}}}}-\overset{\displaystyle O}{\overset{\|}{C}}-OH \qquad (III)$$

worin die Aminocarbonsäuregruppierung $HOOC-CH(NH_2)-$ und gegebenenfalls in der Gruppierung $-A-C(Y)(Z)-$ vorhandene weitere funktionelle Gruppen in geschützter Form vorliegen, oder mit einem reaktionsfähigen funktionellen Säurederivat oder einem Salz davon acyliert, oder

b) in einer Verbindung der Formel

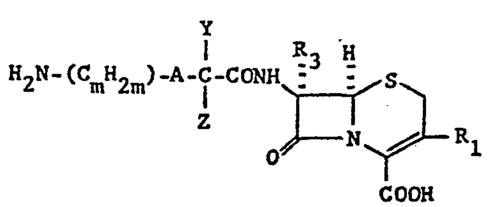

(IV)

worin die Aminogruppe gegebenenfalls durch eine, die Acylierung erlaubende Gruppe substituiert sein kann, und worin die 4-Carboxylgruppe und gegebenenfalls im Rest $R_1$ und in der Gruppierung $-A-C(Y)(Z)-$ vorhandene weitere funktionelle Gruppen in geschützter Form vorliegen können, die

Aminogruppe durch Behandeln mit einem reaktionsfähigen funktionellen Derivat einer Säure der Formel

$$HOOC-\underset{\substack{|\\ NH_2}}{CH}-(C_nH_{2N})-X-W-OH \qquad (V),$$

worin die Aminocarbonsäuregruppierung HOOC—CH($NH_2$)— in geschützter Form vorliegt, oder wenn X—W zusammen eine Gruppe —CO— bedeuten, auch mit einer entsprechenden freien Säure, oder mit einem Salz davon, acyliert, oder

c) in einer Verbindung der Formel

$$HOOC-\underset{\substack{|\\ NH_2}}{CH}-(C_nH_{2n})-X-H \qquad (VI)$$

worin X Sauerstoff, Schwefel oder die Gruppe —NH— bedeutet, und worin die Aminocarbonsäuregruppierung HOOC—CH($NH_2$)— in geschützter Form vorliegt, die Gruppe —X—H mit einem reaktionsfähigen funktionellen Derivat einer Verbindung der Formel

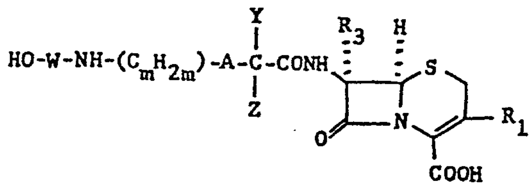

(VII),

worin die 4-Carboxylgruppe und gegebenenfalls im Rest $R_1$ und in der Gruppierung —A—C(Y)(Z)— vorhandene funktionelle Gruppen in geschützter Form vorliegen können, acyliert, und, wenn gewünscht, in einer erhaltenen Verbindung noch nicht geschützte funktionelle Gruppen schützt oder eine Schutzgruppe gegen eine andere austauscht, und/oder, wenn gewünscht, in einem Rest $R_1$ eine Gruppe $R_2$ gegen eine andere Gruppe $R_2$ austauscht, und/oder, wenn gewünscht, eine erhaltene Verbindung, worin $R_3$ Wasserstoff ist, in eine Verbindung überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in die einzelnen Isomeren auftrennt.

**Claims**

1. Acylamido-3-cephem-4-carboxylic acid compounds of the formula

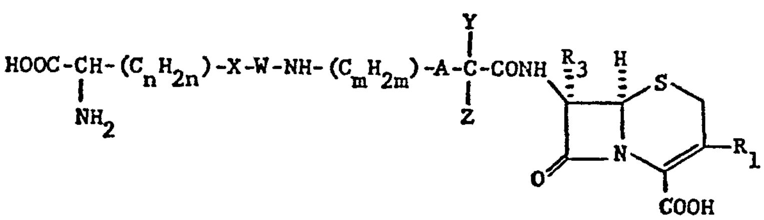

(I)

wherein

the index *n* is an integer from 1 to 4, the index *m* is 0 or 1, X is oxygen, sulfur or the —NH— group, W is a —CO—, —CO—$NHSO_2$— or —$SO_2$NH—CO— group, or X—W together are a —CO— or —CO—$NHSO_2$— group, A is phenylene which is unsubstituted or substituted by lower alkyl, hydroxyl, lower alkoxy and/or halogen, or it is thienylene or furylene, each unsubstituted or substituted by lower alkyl, lower alkoxy and/or halogen, Y is hydrogen, hydroxyl, formloxy, amino or sulfo optionally present in salt form, and Z is hydrogen, or Y and Z together are an oxo group or an =N—O—$R^o$ group in which $R^o$ is hydrogen, or lower alkyl which is unsubstituted or substituted by lower alkoxy, halogen, hydroxyl, acylated hydroxyl, sulfo, carboxyl or carboxyl esterified by lower alkyl, $R_1$ is hydrogen, $C_1$—$C_4$-lower alkyl, $C_1$—$C_4$-lower alkoxy, halogen or a group of the formula —$CH_2$—$R_2$, wherein $R_2$ is a hydroxyl group, a hydroxyl or mercapto group each esterified by a lower aliphatic carboxylic acid, carbamic acid or a carbamic acid substituted on the N atom by lower alkyl, halo-lower-alkyl or lower-alkanoyl-lower-alkyl, lower alkoxy, lower alkylthio, or a mercapto group etherified by a heterocyclic radical bound by way of a ring carbon atom to the mercapto group and having 1—4 ring nitrogen atoms and optionally a further ring hetero atom of the group oxygen and sulfur, which radical can be

mono- or polysubstituted by lower alkyl, hydroxy-lower-alkyl, lower-alkanoyloxy-lower-alkyl, halo-lower-alkyl, carboxy-lower-alkyl, lower-alkoxycarbonyl-lower-alkyl, sulfo-lower-alkyl, amidated sulfo-lower-alkyl, amino-lower-alkyl, mono- or di-lower-alkylamino-lower-alkyl, acylamino-lower-alkyl, cycloalkyl, aryl, aryl-lower-alkyl, heterocyclyl, halogen, amino, mono- or di-lower-alkylamino, lower-alkanoylamino, halo-lower-alkanoylamino, carboxy-lower-alkanoylamino, nitro, hydroxyl, lower alkoxy, carboxyl, lower-alkoxy-carbonyl, carbamoyl, N-mono- or N,N-di-lower-alkylated carbamoyl, cyano, oxo or oxido, or it is a quaternary ammonium group, the term 'lower radicals' denoting, where not otherwise specifically defined, those having 1 to 7 C atoms, and $R_3$ is hydrogen or methoxy, wherein the carboxyl groups are optionally esterified in a form that can be split under physiological conditions, and the salts thereof.

2. Compounds of the formula (I) according to Claim 1, wherein the group $—(C_nH_{2n})—$ is straight-chain, and the indices n and m have the given meanings, X is oxygen or the —NH— group, W is a —CO— or —CO—$NHSO_2$ group, or X—Y together are a —CO— or —CO—$NHSO_2$— group, A is p- or m-phenylene, 2,5-thienylene or 2,5-furylene, Y is hydrogen, hydroxyl, amino or sulfo, and Z is hydrogen, or Y and Z together are a group =N—O—$R^o$, wherein $R^o$ is hydrogen or methyl, $R_1$ is lower alkyl, lower alkoxy, or a group of the formula —$CH_2$—$R_2$, wherein $R_2$ is lower alkanoyloxy, carbamoyloxy, N-lower-alkylcarbamoyloxy, triazolylthio, tetrazolylthio, thiazolylthio, thiatriazolylthio, thiadiazolylthio, oxazolylthio, oxydiazolylthio or pyridino, wherein the heterocyclic rings are unsubstituted or substituted by lower alkyl, N,N-di-lower-alkylamino-lower-alkyl, carboxy-lower-alkyl, sulfo-lower-alkyl, amino, carboxy-lower-alkanoylamino or carbamoyl, and $R_3$ is hydrogen or methoxy, and the salts thereof.

3. Compounds of the formula (I) according to Claim 1 or 2, wherein the group $—(C_nH_{2n})—$ is straight-chain, and the indices n and m have the defined meanings, X is oxygen or the group —NH—, W is a group —CO— or —$CONHSO_2$—, or X—W together are a group —CO— or —$CONHSO_2$, A is p- or m-phenylene, or, if m is 1, also 2,5-thienylene or 2,5-furylene, Y is hydrogen, hydroxyl, amino or sulfo, and Z is hydrogen, or Y and Z together are a group =N—O—$R^o$, wherein $R^o$ is hydrogen, or methyl, $R_1$ is methyl, methoxy, or a group of the formula —$CH_2$—$R_2$, wherein $R_2$ is acetoxy, carbamoyloxy, 1-methyl-1H-tetrazol-5-ylthio, 1-sulfomethyl-1H-tetrazol-5-ylthio, 1-carboxymethyl-1H-tetrazolylthio, 1-(2-dimethylaminoethyl)-1H-tetrazol-5-ylthio, 2-methyl-1,3,4-thiadiazol-5-ylthio or 4-carbamoylpyridinio, and $R_3$ is hydrogen or methoxy, and the salts thereof.

4. 7β - {2 - [4 - ((2R) - 2 - Amino - 2 - carboxyethoxycarbonylamino)phenyl]acetylamino} - 3 - [(1 - methyl - 1H - tetrazol - 5 - yl)thiomethyl] - 3 - cephem - 4 - carboxylic acid,

7β - {2 - [4 - ((2S) - 2 - amino - 2 - carboxyethoxycarbonylamino)phenyl]acetylamino } - 3 - [(1 - methyl - 1H - tetrazol - 5 - yl) - thiomethyl] - 3 - cephem - 4 - carboxylic acid,

7β - {2 - [4 - ((2R) - 2 - amino - 2 - carboxyethoxycarbonylamino)phenyl]acetylamino} - 3 - methyl - 3 - cephem - 4 - carboxylic acid,

7β - {2 - [4 - ((2R) - 2 - amino - 2 - carboxyethoxycarbonylamino)phenyl]acetylamino} - cephalo - sporanic acid,

7β - {2 - [4 - ((5R,S) - 5 - amino - 5 - carboxypentylaminocarbonylamino)phenyl]acetylamino} - cephalosporanic acid,

7β - {2 - [4 - ((3R) - 3 - amino - 3 - carboxypropionylamino) - phenyl]acetylamnino} - 3 - [(1 - methyl - 1H - tetrazol - 5 - yl) - thiomethyl] - 3 - cephem - 4 - carboxylic acid,

7β - {2 - [4 - ((3R) - 3 - amino - 3 - carboxypropionylamino)phenyl]acetylamino} - 3 - carbamoyl - oxymethyl - 3 - cephem - 4 - carboxylic acid,

7β - {2 - [5 - ((2R) - 2 - amino - 2 - carboxyethoxycarbonylaminomethyl) - 2 - furyl] - 2 - syn-methoximinoacetylamino} - 3 - [(1 - methyl - 1H - tetrazol - 5 - yl) - thiomethyl] - 3 - cephem - 4 - carboxylic acid,

7β - {2 - [5 - ((2H) - 2 - amino - 2 - carboxyethoxycarbonylaminomethyl) - 2 - furyl] - 2 - syn - methoxyiminoacetylamino} - 3 - carbamoyloxymethyl - 3 - cephem - 4 - carboxylic acid,

7β - {2 - [5 - ((2R) - 2 - amino - 2 - carboxyethoxycarbonylaminomethyl) - 2 - thienyl] - 2 - syn - methoxyiminoacetylamino} - 3 - acetoxymethyl - 3 - cephem - 4 - carboxylic acid,

7β - {2 - [4 - ((2R) - 2 - amino - 2 - carboxyethoxycarbonylaminosulfonylamino)phenyl]acetyl - amino} - 3 - carbamoyloxymethyl - 3 - cephem - 4 - carboxylic acid,

7β - {(2R,S) - 2 - [4 - ((2R) - 2 - amino - 2 - carboxyethoxycarbonylamino)phenyl] - 2 - hydroxy - acetylamino} - 3 - carbamoyloxymethyl - 3 - cephem - 4 - carboxylic acid,

7β - {(2R,S) - 2 - [4 - ((2R) - amino - 2 - carboxyethoxycarbonylamino)phenyl] - 2 - hydroxy - acetylamino} - 3 - [(1 - methyl - 1H - tetrazol - 5 - yl) - thiomethyl] - 3 - cephem - 4 - carboxylic acid,

7β - [4 - (2R) - 2 - amino - 2 - carboxyethoxycarbonylamino)phenylacetamido] - 3 - (4 - carb - amoyl - pyridinomethyl) - 3 - cephem - 4 - carboxylate,

7β - {2 - [4 - ((4R) - 4 - amino - 4 - carboxybutyrylamino) - phenyl]acetylamino} - 3 - [(1 - methyl - 1H - tetrazol - 5 - yl) - thiomethyl] - 3 - cephem - 4 - carboxylic acid,

7α - methoxy - 7β - {2 - [4 - ((2R) - 2 - amino - 2 - carboxyethoxycarbonylamino)phenyl]acetyl - amino} - 3 - [(1 - methyl - 1H - tetrazol - 5 - yl) - thiomethyl] - 3 - cephem - 4 - carboxylic acid,

7β - {(2R,S) - 2 - [4 - ((2R) - 2 - amino - 2 - carboxyethoxycarbonylamino)phenyl] - 2 - sulfo - acetylamino} - 3 - [(1 - methyl - 1H - tetrazol - 5 - yl) - thiomethyl] - 3 - cephem - 4 - carboxylic acid,

7β - {(2R) - 2 - amino - 2 - [3 - (2R) - 2 - amino - 2 - carboxyethoxycarbonylamino)phenyl]acetyl -

amino} - 3 - methoxy - 3 - cephem - 4 - carboxylic acid,

7β - {(2R,S) - 2 - hydroxy - 2 - [3 - ((2R) - 2 - amino - 2 - carboxyethoxycarbonylamino)phenyl] - acetylamino} - 3 - carbamoyloxymethyl - 3 - cephem - 4 - carboxylic acid,

7β - {2R,S) - 2 - hydroxy - 2 - [3 - ((2R) - 2 - amino - 2 - carboxyethoxycarbonylamino)phenyl]-acetylamino} - 3 - methoxy - 3 - cephem - 4 - carboxylic acid, and

7β - [5 - ((2R) - 2 - amino - 2 - carboxy - ethoxycarbonylaminomethyl) - thien - 2 - yl - acet - amido] - 7α - methoxy - 3 - (1 - methyl - 1H - tetrazol - 5 - ylthiomethyl) - 3 - cephem - 4 - carboxylic acid, and pharmaceutically acceptable salts thereof, according to any one of Claims 1 to 3.

5. 7β - {2 - [4 - ((2R) - 2 - amino - 2 - carboxyethoxycarbonylamino)phenyl]acetylamino} - 3 - carbamoyloxymethyl - 3 - cephem - 4 - carboxylic acid and pharmaceutically acceptable salts thereof, according to any one of Claims 1 to 3.

6. 7β - {(2R) - 2 - [4 - ((2R) - 2 - amino - 2 - carboxyethoxycarbonylamino)phenyl] - 2 - hydroxy - acetylamino} - 3 - [(1 - methyl - 1H - tetrazol - 5 - yl) - thiomethyl] - 3 - cephem - 4 - carboxylic acid, and pharmaceutically acceptable salts thereof, according to any one of Claims 1 to 3.

7. 7β - {(2R) - 2 - [4 - ((2R) - 2 - amino - 2 - carboxyethoxycarbonylamino)phenyl] - 2 - hydroxy - acetylamino} - 3 - carbamoyloxymethyl - 3 - cephem - 4 - carboxylic acid, and physiologically acceptable salts thereof, according to any one of Claims 1 to 3.

8. The sodium salts of the compounds mentioned in any one of Claims 1 to 7.

9. Pharmaceutical preparations containing any one of the compounds claimed in Claims 1 to 8.

10. Substances and mixtures of substances according to any one of Claims 1 to 9 for use in the treatment of infections caused by gram-positive and gram-negative bacteria or cocci.

11. Acylamido-3-cephem-4-carboxylic acid compounds of the formula I defined in Claim 1, wherein at least one of the functional groups is in the protected form, and salts of these compounds.

12. A process for producing acylamido-3-cephem-4-carboxylic acid compounds of the formula I defined in Claim 1, wherein the carboxyl groups are optionally esterified in a form that can be split off under physiological conditions, and the salts thereof, characterised in that in a starting compound of the formula I, in which at least one of the functional groups present is protected, the functional group(s) is (are) liberated, if desired, in a resulting compound a group $R_1$ is converted into a different group $R_1$, and/or, if desired, a free carboxyl group is converted into an esterified carboxyl group that can be split off under physiological conditions, and/or, if desired, a mixture of isomers obtained is separated into the individual isomers, and/or, if desired, a compound obtained is converted into a salt, or a resulting salt is converted into a free compound or into another salt.

13. A process for producing acylamino-3-cephem-4-carboxylic acid compounds of the formula I defined in Claim 1, wherein at least one of the functional groups is present in the protected form, characterised in that

a) in a compound of the formula

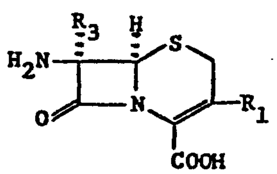

(II),

in which the amino group is optionally substituted by a group allowing acylation and in which the 4-carboxyl group and further functional groups optionally present in the radical $R_1$ may be present in protected form, the amino group is acylated by treatment with an acid of the formula

$$HOOC-\underset{NH_2}{\underset{|}{CH}}-(C_nH_{2n})-X-W-NH-(C_mH_{2m})-A-\overset{Y}{\overset{|}{\underset{Z}{\underset{|}{C}}}}-\overset{O}{\overset{\|}{C}}-OH \quad (III),$$

in which the aminocarboxylic acid grouping HOOC—CH($NH_2$)— and further functional groups optionally present in the grouping —A—C(Y)(Z)— are present in protected form, or with a reactive functional acid derivative or a salt thereof, or

b) in a compound of the formula

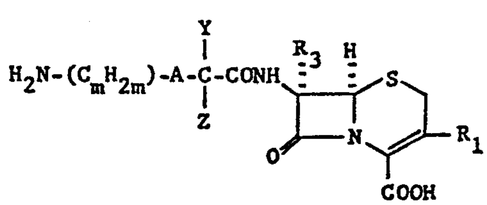

(IV),

in which the amino group may optionally be substituted by a group allowing acylation and in which the 4-carboxyl group and further functional groups optionally present in the radical $R_1$ and in the grouping —A—C(Y)(Z)— may be present in protected form, the amino group is acylated by treatment with a reactive functional derivative of an acid of the formula

$$\underset{\displaystyle NH_2}{HOOC—\underset{|}{CH}—(C_nH_{2n})—X—W—OH} \qquad (V),$$

in which the aminocarboxylic acid grouping HOOC—CH($NH_2$)— is present in protected form, or when X—W together are a group —CO— also with a corresponding free acid, or with a salt thereof, or c) in a compound of the formula

$$\underset{\displaystyle NH_2}{HOOC—\underset{|}{CH}—(C_nH_{2n})—X—H} \qquad (VI),$$

in which X is oxygen, sulfur or the group —NH— and in which the aminocarboxylic acid grouping HOOC—CH($NH_2$)— is present in protected form, the —X—H group is acylated with a reactive functional derivative of a compound of the formula

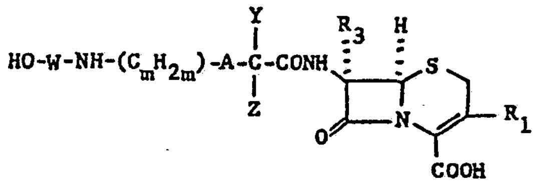

(VII),

in which the 4-carboxyl group and functional groups optionally present in the radical $R_1$ and in the grouping —A—C(Y)(Z)— may be present in protected form, and, if desired, in a resulting compound still unprotected functional groups are protected, or a protective group is exchanged for another, and/or, if desired, a group $R_2$ in a radical $R_1$ is exchanged for a different group $R_2$, and/or, if desired, a compound obtained in which $R_3$ is hydrogen is converted into a compound in which $R_3$ is methoxy, and/or, if desired, a 2-cephem compound obtained or a resulting mixture of a 2-cephem compound and 3-cephem compound is isomerised to form the 3-cephem compound, and/or, if desired, a mixture of isomers obtained is separated into the individual isomers.

**Revendications**

1. Composés d'acide acylamino-3-cephem-4-carboxylique de formule

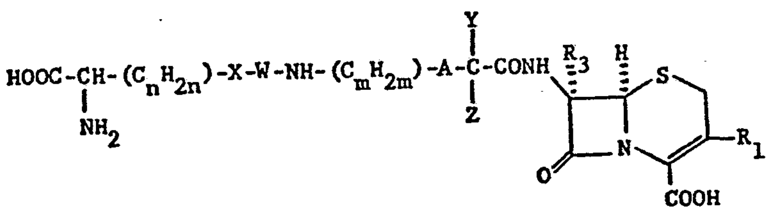

(I).

où l'indice n représente un nombre entier allant de 1 à 4, l'indice m 0 ou 1, X un oxygène, un soufre ou le groupe —NH—, W un groupe —CO—, —CO—$NHSO_2$— ou —$SO_2$NH—CO—, ou X—W ensemble un groupe —CO— ou —CO—$NHSO_2$—, A un phénylène éventuellement substitué par un alcoyle inférieur, un hydroxy, un alcoxy inférieur et/ou un halogène, un thiénylène ou un furylène éventuellement substitué par un alcoyle inférieur, un alcoxy inférieur et/ou un halogène, Y un hydrogène, un hydroxyle, un formyloxy, un amino ou un sulfo se présentant éventuellement sous forme de sel et Z un hydrogène, ou Y et Z ensemble un groupe oxo ou un groupe =N—O—$R^o$, où $R^o$ représente un hydrogène ou un alcoyle inférieur éventuellement substitué par un alcoxy inférieur, un halogène, un hydroxy, un hydroxy acylé, un sulfo, un carboxy ou un carboxy estérifié par un alcoyle inférieur, $R_1$ un hydrogène, un alcoyle inférieur en $C_1$ à $C_4$, un alcoxy inférieur en $C_1$ à $C_4$, un halogène ou un groupe de formule —$CH_2$—$R_2$, où $R_2$ représente un groupe hydroxy, un groupe hydroxy ou mercapto estérifié par un acide carboxylique aliphatique inférieur, par l'acide carbamique ou un acide carbamique susbtitué sur l'atome d'azote par un alcoyle inférieur, un alcoyle inférieur halogéné ou un alcanoyle

inférieur-alcoyle inférieur, un alcoxy inférieur, un alcoyle inférieur-thio ou un groupe mercapto éthérifié par un radical hétérocyclique, lié au groupe mercapto par l'intermédiaire d'un atome de carbone du noyau, ayant de 1 à 4 atomes d'azote dans son noyau, et éventuellement dans son noyau un autre hétéroatome du groupe de l'oxygène et du soufre, qui peut être mono- ou polysubstitué par un alcoyle inférieur, un hydroxyalcoyle, inférieur, un alcanoyloxy inférieur-alcoyle inférieur, un alcoyle inférieur halogéné, un carboxyalcoyle, inférieur, un alcoxycarbonyle inférieur-alcoyle inférieur, un sulfoalcoyle inférieur, un sulfoalcoyle inférieur amidé, un amino-alcoyle inférieur, un mono- ou di-alcoyle inférieur-aminoalcoyle inférieur, un acylaminoalcoyle inférieur, un cycloalcoyle, un aryle, un arylalcoyle inférieur, un hétérocyclyle, un halogène, un amino, un mono-ou di-alcoyle inférieur-amino, un alcanoyle inférieur-amino, un halogène-alcanoyle inférieur-amino, un carboxy-alcanoyle inférieur-amino, un nitro, un hydroxy, un alcoxy inférieur, un carboxy, un alcoxycarbonyle inférieur, un carbamoyle, un carbamoyle substitué par un N-mono-alcoyle inférieur ou un N,N-dialcoyle inférieur, un cyano, un oxo ou un oxydo, ou un groupe ammonium quaternaire, les radicaux décrits comme inférieurs comprenant, sauf définition expresse ontraite, ceux qui ont de 1 à 7 atomes de carbone, et $R_3$ représente un hydrogène ou un méthoxy, où les groupes carboxyle sont éventuellement estérifiés sous forme physiologiquement séparable, et leurs sels.

2. Composés de formule [I] selon la revendication 1, où le groupe $—[C_nH_{2n}]—$ est non ramifié et où les indices n et m ont la signification donnée, X représente un oxygène ou le groupe —NH—, W un groupe —CO— ou $—CO—NHSO_2—$, ou X—W ensemble un groupe —CO— ou $—CO—NHSO_2—$, A représente un p- ou un m-phénylène, un 2,5-thiénylène ou un 2,5-furylène, Y représente un hydrogène, un hydroxyle, un amino ou un sulfo et Z représente un hydrogène, ou Y et Z représentent ensemble un groupe $=N—O—R^o$, où $R^o$ est un hydrogène ou un méthyle, $R_1$ un alcoyle inférieur, un alcoxy inférieur, ou un groupe de formule $—CH_2—R_2$, où $R_2$ représente un alcanoyloxy inférieur, un carbamoyloxy, un N-alcoyle inférieur-carbamoyloxy, un triazolylthio, un tétrazolylthio, un thiazolylthio, un thiatriazolylthio, un thiadiazolylthio, un oxazolylthio, un oxadiazolylthio, ou un pyridinio, où les radicaux hétérocycliques peuvent être éventuellement substitués par un alcoyle inférieur, un N,N-dialcoyle inférieur-aminoalcoyle inférieur, un carboxyalcoyle inférieur, un sulfoalcoyle inférieur, un amino, un carboxyalcanoyle inférieur-amino ou un carbamoyle, et $R_3$ représente un hydrogène ou un méthoxy, et leurs sels.

3. Composés de formule I selon les revendications 1 ou 2, où le groupe $—[C_2H_{2n}]—$ est non ramifié et où les indices n et m ont la signification donnée, X représente un oxygène ou le groupe —NH—, W représente un groupe —CO— ou $—CONHSO_2—$, ou bien où X—W représentent ensemble un groupe —CO— ou $—CONHSO_2—$, A représente un p- ou un m-phénylène ou encore, lorsque m vaut 1, un 2,5-thiénylène ou un 2,5-furylène, Y représente un hydrogène, un hydroxyle, un amino ou un sulfo et Z représente un hydrogène, ou bien où Y et Z représentent ensemble un groupe $=N—O—R^o$, où $R^o$ est un hydrogène ou un méthyle, $R_1$ représente un méthyle, un méthoxy ou un groupe de formule $—CH_2—R_2$, où $R_2$ représente un acétoxy, un carbamoyloxy, un 1-méthyl-1H-tétrazol-5-yl-thio, un 1-sulfométhyl-1H-tétrazol-5-ylthio, un 1-carboxyméthyl-1H-tétrazolylthio, un 1-(2-diméthylamino-éthyl)-1H-tétrazol-5-ylthio, un 2-méthyl-1,3,4-thiadiazol-5-ylthio, un 4-carbamoylpyridinio, et $R_3$ un hydrogène ou un méthoxy, et leurs sels.

4. L'acide 7β - {2 - / 4 - [2R] - 2 - amino - 2 - carboxyéthoxycarbonylamino]phényl/acétyl - amino} - 3 - / [1 - méthyl - 1H - tétrazol - 5 - yl] - thiométhyl / - 3 - cephem - 4 - carboxylique,

l'acide 7β - {2 - / 4 - [[2S] - 2 - amino - 2 - carboxyéthoxycarbonylamino]phényl/acétylamino} - 3 - / [1 - méthyl - 1H - tétrazol - 5 - yl] - thiométhyl / - 3 - cephem - 4 - carboxylique,

l'acide 7β - {2 - / 4 - [[2R] - 2 - amino - 2 - carboxyéthoxycarbonylamino]phényl / acétylamino} - 3 - méthyl - 3 - cephem - 4 - carboxylique,

l'acide 7β - {2 - / 4 - [[2R] - 2 - amino - 2 - carboxyéthoxycarbonylamino]phényl/acétylamino} - céphalosporanique,

l'acide 7β - {2 - /4 - [[5R,S] - 5 - amino - 5 - carboxypentylaminocarbonylamino]phényl/acétyl - amino} - céphalosporanique,

l'acide 7β - {2 - /4 - [[3R] - 3 - amino - 3 - carboxypropionylamino] - phényl/acétylamino} - 3 -/ [1 - méthyl - 1H - tétrazol - 5 - yl]thiométhyl/ - 3 - cephem - 4 - carboxylique,

l'acide 7β - {2 - /4 - [[3R] - 3 - amino - 3 - carboxypropionylamino]phényl/acétylamino} - 3 - carbamoyloxyméthyl - 3 - cephem - 4 - carboxylique,

l'acide 7β - {2 - /5 - [[2R] - 2 - amino - 2 - carboxyéthoxycrbonylaminométhyl] - 2 - furyl/ - 2 - syn - méthoxyiminoacétylamino} - 3 - /[1 - méthyl - 1H - tétrazol - 5 - yl] - thiométhyl/ - 3 - cephem - 4 - carboxylique,

l'acide 7β - {2 - /5 - [[2R] - 2 - amino - 2 - carboxyéthoxycarbonylaminométhyl] - 2 - furyl/ - 2 - syn - méthoxyiminoacétylamino} - 3 - carbamoyloxyméthyl - 3 - cephem - 4 - carboxylique,

l'acide 7β - {2 - /5 - [[2R] - 2 - amino - 2 - carboxyéthoxycarbonylaminométhyl] - 2 - thiényl/ - 2 - syn - méthoxyiminoacétylamino} - 3 - acétoxyméthyl - 3 - cephem - 4 - carboxylique,

l'acide 7β - {2 - /4 - [[2R] - 2 - amino - 2 - carboxyéthoxycarbonylaminosulfonylamino]phényl/ - acétylamino} - 3 - carbamoyloxyméthyl - 3 - cephem - 4 - carboxylique,

l'acide 7β - {[2R,S] - 2 - /4 - [[2R] - 2 - amino - 2 - carboxyéthoxycarbonylamino]phényl/ - 2 - hydroxyacétylamino} - 3 - carbamoyloxyméthyl - 3 - cephem - 4 - carboxylique,

l'acide 7$\beta$ - {[2R,S] - 2 - /4 - [[2R] - amino - 2 - carboxyéthoxycarbonyl]phényl/ - 2 - hydroxy - acétylamino} - 3 - /[1 - méthyl - 1H - tétrazol - 5 - yl] - thiométhyl/ - 3 - cephem - 4 - carboxylique,

le 7$\beta$ - /4 - [2R] - 2 - amino - 2 - carboxyéthoxycarbonylamino] - phénylacétamido/ - 3 - [4 - carbamoyl - pyridiniométhyl] - 3 - cephem - 4 - carboxylate, et

l'acide 7$\beta$ - {2 - /4 - [[4R] - 4 - amino - 4 - carboxybutyrylamino] - phényl/acétylamino} - 3 -/ [1 - méthyl - 1H - tétrazol - 5 - yl]thiométhyl/ - 3 - cephem - 4 - carboxylique,

l'acide 7$\alpha$ - méthoxy - 7$\beta$ - {2 - /4 - [[2R] - 2 - amino - 2 - carboxyéthoxycarbonylamino]phényl/ - acétylamino} - 3 - /[1 - méthyl - 1H - tétrazol - 5 - yl] - thiométhyl/ - 3 - cephem - 4 - carboxylique,

l'acide 7$\beta$ - {[2R,S] - 2 - /4 - [[2R] - 2 - amino - 2 - carboxyéthoxycarbonylamino]phényl/ - 2 - sulfo acétylamino} - 3 - /[1 - méthyl - 1H - tétrazol - 5 - yl] - thiométhyl/ - 3 - cephem - 4 - carboxylique,

l'acide 7$\beta$ - {[2R] - 2 - amino - 2 - /3 - [2R] - 2 - amino - 2 - carboxy - éthoxycarbonylamino] - phényl/acétylamino} - 3 - méthoxy - 3 - cephem - 4 - carboxylique,

l'acide 7$\beta$ - {[2R,S] - 2 - hydroxy - 2 - /3 - [[2R] - 2 - amino - 2 - carboxyéthoxycarbonylamino] - phényl/acétylamino} - 3 - carbamoyloxyméthyl - 3 - cephem - 4 - carboxylique,

l'acide 7$\beta$ - {[2R,S] - 2 - hydroxy - 2 - /3 - [[2R] - 2 - amino - 2 - carboxyéthoxycarbonylamino] - phényl/acétylamino} - 3 - méthoxy - 3 - cephem - 4 - carboxylique,

l'acide 7$\beta$ - /5 - [[2R] - 2 - amino - 2 - carboxy - éthoxycarbonylaminométhyl] - thién - 2 - yl - acétamido/ - 7$\alpha$ - méthoxy - 3 - [1 - méthyl - 1H - tétrazol - 5 - ylthiométhyl] - 3 - cephem - 4 - carboxylique, et leurs sels pharmaceutiquement acceptables, selon l'une des revendications 1 à 3.

5. L'acide 7$\beta$ - {2 - /4 - [[2R] - 2 - amino - 2 - carboxyéthoxycarbonylamino] - phényl/acétyl - amino} - 3 - carbamoyloxyméthyl - 3 - cephem - 4 - carboxylique et ses sels pharmaceutiquement acceptables, selon l'une des revendications 1 à 3.

6. L'acide 7$\beta$ - {[2R] - 2 - /4 - [[2R] - 2 - amino - 2 - carboxyéthoxycarbonylamino]phényl/ - 2 - hydroxyacétylamino} - 3 - /[1 - méthyl - 1H - tétrazol - 5 - yl] - thiométhyl/ - 3 - cephem - 4 - carboxylique et ses sels pharmaceutiquement acceptables, selon l'une des revendications 1 à 3.

7. L'acide 7$\beta$ - {[2R] - 2 - /4 - [[2R] - 2 - amino - 2 - carboxyéthoxycarbonylamino]phényl/ - 2 - hydroxyacétylamino} - 3 - carbamoyloxyméthyl - 3 - cephem - 4 - carboxylique et ses sels pharmaceutiquement acceptables, selon l'une des revendications 1 à 3.

8. Les sels de sodium de l'un des composés mentionnés dans les revendications 1 à 7.

9. Préparations pharmaceutiques contenant l'un des composés revendiqués dans l'une des revendications 1 à 8.

10. Corps et mélanges de corps selon l'une des revendications 1 à 9 aux fins d'application au traitement d'infections provoquées par des bactéries ou des coques gram-positifs et gram-négatifs.

11. Composés d'acide acylamido-3-cephem-4-carboxylique de la formule I définie dans la revendication 1, où au moins l'un des groupes fonctionnels se présente sous forme protégée, ainsi que leurs sels.

12. Procédé de préparation de composés d'acide acylamido-3-cephem-4-carboxylique de la formule 1 définie dans la revendication 1, où les groupes carboxyle sont éventuellement estérifiés sous forme physiologiquement séparable, et de leurs sels, caractérisé en ce qu'on libère le[s] groupe[s] fonctionnel[s] dans un composé de départ correspondant à la formule I, où au moins l'un des groupes fonctionnels présents est protégé, si on le désire, en ce qu'on transforme un groupe carboxyle libre en un groupe carboxyle estérifié physiologiquement séparable, et/ou, si on le désire, en ce qu'on dédouble un mélange d'isomères obtenu pour donner les isomères isolés, et/ou, si on le désire, en ce qu'on transforme un composé obtenu en un sel ou un sel obtenu en un composé libre ou en un autre sel.

13. Procédé de préparation de composés d'acide acylamino-3-cephem-4-carboxylique de formule I défini dans la revendication 1, où au moins l'un des groupes fonctionnels se présente sous forme protégée, caractérisé en ce que

a] dans un composé de formule

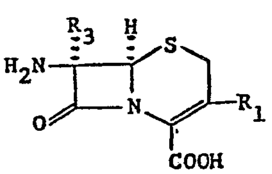

(II),

où le groupe amino est éventuellement substitué par un groupe permettant l'acylation, et où le groupe 4-carboxyle et éventuellement d'autres groupes fonctionnels présents dans le radical $R_1$ se présentent sous forme protégée, on acyle le groupe amino par traitement avec un acide de formule

$$HOOC-\underset{NH_2}{CH}-(C_nH_{2n})-X-W-NH-(C_mH_{2m})-A-\overset{Y}{\underset{Z}{C}}-\overset{O}{\overset{\|}{C}}-OH \qquad (III),$$

où le groupement acide carboxylique aminé HOOC—CH[$NH_2$]— et éventuellement d'autres groupes fonctionnels présents dans le groupement —A—C[Y][Z]— se présentent sous forme protégée, ou avec un dérivé d'acide fonctionnel réactif ou avec un de ses sels, ou

b] dans un composé de formule

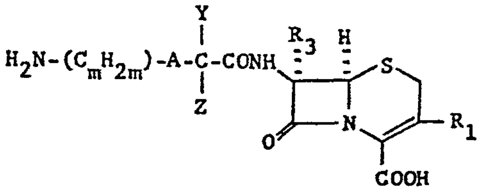

(IV),

où le groupe amino peut être éventuellement substitué par un groupe permettant l'acylation, et où le groupe 4-carboxyle et éventuellement d'autres groupes fonctionnels présents dans le radical $R_1$ et dans le groupement —A—C[Y][Z]— peuvent se présenter sous forme protégée, on acyl le groupe amino par traitement avec un dérivé fonctionnel réactif d'un acide de formule

$$HOOC—CH(NH_2)—(C_nH_{2n})—X—W—OH \quad (V),$$

où le groupement acide carboxylique aminé HOOC—CH[$NH_2$]— se présente sous forme protégée, ou bien lorsque X—W représentent ensemble un groupe —CO—, également avec un acide libre correspondant ou avec un de ses sels, ou

c] dans un composé de formule

$$HOOC—CH(NH_2)—(C_nH_{2n})—X—H \quad (VI),$$

où X représente un oxygène, un soufre ou le groupe —NH—, et où le groupement acide carboxylique aminé HOOC—CH[$NH_2$)— se présente sous forme protégée, on acyle le groupe —X—H— avec un dérivé fonctionnel réactif d'un composé de formule

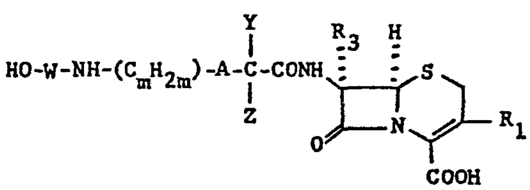

(VII),

où le groupe 4-carboxyle et éventuellement d'autres groupes fonctionnels présents dans le radical $R_1$ et dans le groupement —A—C[Y][Z]— peuvent se présenter sous forme protégée et, si on le désire, en ce qu'on protège dans un composé obtenu les groupes fonctionnels non encore protégés ou en ce qu'on échange un groupe protecteur contre un autre, et/ou, si on le désire, en ce qu'on échange dans un radical $R_1$ un groupe $R_2$ contre un autre groupe $R_2$, et/ou, si on le désire, en ce qu'on transforme un composé obtenu où $R_3$ est un hydrogène en un composé où $R_3$ est un méthoxy, et/ou, si nécessaire, en ce qu'on isomérise un composé 2-cephem obtenu ou un mélange obtenu d'un composé 2-cephem et d'un composé 3-cephem en un composé 3-cephem, et/ou, si on le désire, en ce qu'on dédouble un mélange d'isomères obtenu pour donner les isomères isolés.